# EUROPEAN PATENT APPLICATION

(11) **EP 4 640 838 A1**
(43) Date of publication of application: **29.10.2025**
(21) Application number: 23907213.5
(22) Date of filing: 22.12.2023
(51) Int. Cl.: C12P 1/00, A61K 35/12, A61K 35/28, A61K 35/51, A61K 38/18, A61K 38/19, A61K 38/20, A61P 19/00, A61P 43/00, C07K 5/062, C12N 1/00, C12N 5/0775

(54) **UTILIZATION OF MESENCHYMAL STEM CELLS OR CULTURE SUPERNATANT THEREOF**

(30) Priority: 23.12.2022 JP 2022207266
(71) Applicant: Cell Exosome Therapeutics Inc., Tokyo 150-0011 (JP)
(72) Inventor: YANAGITA, Yasutomo, Tokyo 150-0011 (JP); RINOIE, Chugo, Tokyo 150-0011 (JP); MINAMI, Itsunari, Tokyo 150-0011 (JP)
(74) Representative: Gulde & Partner
(86) International application number: PCT/JP2023/046277
(87) International publication number: WO 2024/135853

(57) **Abstract**

Provided is a culture supernatant of mesenchymal stem cells or mesenchymal stem cells useful for disease suppression or amelioration. Used is a method for producing a culture supernatant, comprising the step of recovering a culture supernatant from a medium containing mesenchymal stem cells and an imidazole dipeptide. It is also possible to use a method for producing cells, comprising the step of culturing mesenchymal stem cells in a medium containing an imidazole dipeptide to generate cultured cells.

## Description

### Technical Field

The technical field of the present invention relates to mesenchymal stem cells or a culture supernatant of mesenchymal stem cells, and the like.

### Background Art

Mesenchymal stem cells (MSCs) and MSC culture supernatants are used in the field of regenerative medicine. MSC culture supernatants contain components secreted from cells, such as cytokines. MSC culture supernatants have cost advantages because they can be administered to patients without purification of a single active ingredient.

Several studies on the MSCs or MSC culture supernatants have been reported. For example, Patent Literature 1 and Non-Patent Literature 1 describe the study on cytokine levels in MSC culture supernatants (see Examples in Patent Literature 1 and Abstract in Non-Patent Literature 1). In addition, Patent Literature 1 describes that administration of MSC cell suspension to a rat model of lower limb ischemia resulted in an increase in blood flow. Non-Patent Literature 1 describes that the MSC culture supernatant was found to promote repair of photodamage to skin and vascular regeneration.

### Citation List

### Patent Literature

Patent Literature 1: WO2021/200744

### Non Patent Literature

Non-patent document 1: "Cytoplasm or Supernatant: Where Is the Treasury of the Bioactive Antiaging Factor from Mesenchymal Stem Cells?" Luo et al., Stem Cells Dev. 2022 Sep; 31(17-18): 529-540.

### Summary of Invention

### Technical Problem

The composition of conventional MSC culture supernatants had room for improvement when considering their use for suppressing or ameliorating diseases.

Meanwhile, the present inventors have studied the method of culturing MSCs. Surprisingly, the addition of an imidazole dipeptide to a medium during MSC culture resulted in producing a culture supernatant useful for disease suppression or amelioration. In addition, the obtained cells were also useful for disease suppression or amelioration. Based on these findings, the present inventors completed the present invention.

### Solution to Problem

An aspect of the present invention provides a method for producing a culture supernatant, comprising the step of recovering a culture supernatant from a medium containing mesenchymal stem cells and an imidazole dipeptide. This method may be used to produce a culture supernatant useful for disease suppression or amelioration. Another aspect of the present invention provides a method for producing cells, the method comprising the step of culturing mesenchymal stem cells in a medium containing an imidazole dipeptide to generate cultured cells. This method may be used to produce cells useful for disease suppression or amelioration.

In addition, still another aspect of the present invention provides a method of culturing cells, comprising the step of culturing mesenchymal stem cells in a serum-free medium containing an imidazole dipeptide to generate cultured cells. Still another aspect of the present invention provide a medium for culturing mesenchymal stem cells, wherein the medium comprises an imidazole dipeptide and is serum-free. Still another aspect of the present invention provides a method of inhibiting generation of osteoblasts, the method comprising the step of culturing mesenchymal stem cells in a medium containing an imidazole dipeptide. Still another aspect of the present invention provides a method of inhibiting generation of osteoblasts, comprising the step of bringing mesenchymal stem cells into contact with a culture supernatant obtained by culturing mesenchymal stem cells in a medium containing an imidazole dipeptide. Still another aspect of the present invention provides a composition for inhibiting generation of osteoblasts, comprising an imidazole dipeptide. Still another aspect of the present invention provides a composition for inhibiting generation of osteoblasts, comprising a culture supernatant obtained by culturing mesenchymal stem cells in a medium containing an imidazole dipeptide. Still another aspect of the present invention provides a method of promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker, the method comprising the step of culturing mesenchymal stem cells in a medium containing an imidazole dipeptide. Still another aspect of the present invention provides a composition for promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker of mesenchymal stem cells, the composition comprising an imidazole dipeptide. Still another aspect of the present invention provides a method of promoting exosome secretion, the method comprising the step of culturing mesenchymal stem cells in a medium containing an imidazole dipeptide. Still another aspect of the present invention provides a composition for promoting exosome secretion, comprising an imidazole dipeptide. Still another aspect of the present invention provides a culture supernatant of mesenchymal stem cells, comprising at least 500 pg/ml of G-CSF. Still another aspect of the present invention provides a culture supernatant of mesenchymal stem cells, comprising at least 2700 pg/ml of IL-6. Still another aspect of the present invention provides an umbilical cord-derived mesenchymal stem cell that expresses or secretes a high level of G-CSF or IL-6. Still another aspect of the present invention provides an IL-34-positive mesenchymal stem cell. Still another aspect of the present invention provides an ADAM8-positive mesenchymal stem cell. Still another aspect of the present invention provides a pharmaceutical composition for inhibiting osteogenic differentiation, comprising cells obtained by culturing mesenchymal stem cells in a medium containing an imidazole dipeptide. Still another aspect of the present invention provides a method for producing a pharmaceutical composition for inhibiting osteogenic differentiation, the method comprising the step of culturing mesenchymal stem cells in a medium containing an imidazole dipeptide to generate cultured cells. Still another aspect of the present invention provides a medium comprising an imidazole dipeptide and a leukemia inhibitory factor (LIF) component. Still another aspect of the present invention provides a kit comprising an imidazole dipeptide and a LIF component.

### Brief Description of Drawings

[FIG. 1] FIG. 1 is a micrograph of MSCs after culturing in a carnosine-free medium.
[FIG. 2] FIG. 2 is a micrograph of MSCs after culturing in a medium containing 1 mM carnosine.
[FIG. 3] FIG. 3 is a micrograph of MSCs after culturing in a medium containing 10 mM carnosine.
[FIG. 4] FIG. 4 is a micrograph of MSCs after culturing in a medium containing 30 mM carnosine.
[FIG. 5] FIG. 5 is a table showing the results of counting the number of MSCs.
[FIG. 6] FIG. 6 is a graph showing the results of the level of exosome marker in each culture supernatant. The four bar graphs shown for first to fourth recovery times, respectively, refer to the results obtained under conditions of adding 0 mM, 1 mM, 10 mM, and 30 mM carnosine in the order from left. The same applies to FIGS 7 to 14.
[FIG. 7] FIG. 7 is a graph showing the results of the level of HGF in each culture supernatant.
[FIG. 8] FIG. 8 is a graph showing the results of the level of G-CSF in each culture supernatant.
[FIG. 9] FIG. 9 is a graph showing the results of the level of MCP-1 in each culture supernatant.
[FIG. 10] FIG. 10 is a graph showing the results of the level of VEGF-C in each culture supernatant.
[FIG. 11] FIG. 11 is a graph showing the results of the level of TGF-β1 in each culture supernatant.
[FIG. 12] FIG. 12 is a graph showing the results of the level of IL-6 in each culture supernatant. The "*>10,000" and "*>7,000" indicate that values greater than the respective values were over the limit of detection.
[FIG. 13] FIG. 13 is a graph showing the results of the level of IL-7 in each culture supernatant.
[FIG. 14] FIG. 14 is a graph showing the results of the level of IL-8 in each culture supernatant.
[FIG. 15] FIG. 15 is a graph showing the results of quantifying calcium deposition in bone marrow MSCs.
[FIG. 16] FIG. 16 is Alizarin Red S stained images of bone marrow MSCs.
[FIG. 17] FIG. 17 is a graph showing the results of quantifying calcium deposition when bone marrow MSCs and umbilical cord MSCs were co-cultured.
[FIG. 18] FIG. 18 is Alizarin Red S stained images when bone marrow MSCs and umbilical cord MSCs were co-cultured.
[FIG. 19] FIG. 19 is a table showing the culture conditions of umbilical cord MSCs. In the formula for calculating %Increase described in the Examples, all RPM values are increased by +1, and then the respective %Increase is calculated. For example, even if the RPM value of M0 is 0, %Increase is calculated while increased by +1. Accordingly, the %Increase value of M0 is (M0 + 1)/(M0 + 1) = (0 + 1)/(0 + 1), which value is 1.
[FIG. 20] FIG. 20 is graphs showing the change in %Increase values for 10 different genes. FIGS. 20(a) to 20(j) show the results of, in sequence, gremlin 1, DAN family BMP antagonist, KIT ligand, R-spondin 2, semaphorin 3B, fibroblast growth factor 11, TNF receptor superfamily member 11b, ADAM metallopeptidase domain 8, interleukin 34, insulin like growth factor binding protein 2, and dickkopf WNT signaling pathway inhibitor 1, respectively.
[FIG. 21] FIG. 21 is a graph showing the RPM values of interleukin 34 expression level in umbilical cord MSCs.
[FIG. 22] FIG. 22 is a graph showing the RPM values of ADAM metallopeptidase domain 8 expression level in umbilical cord MSCs.
[FIG. 23] FIG. 23 is a graph showing the results of ELISA analysis of Osteoprotegerin.
[FIG. 24] FIG. 24 is a graph showing the results of ELISA analysis of Osteoprotegerin.
[FIG. 25] FIG. 25 is a graph showing the results of ELISA analysis of Osteoprotegerin.
[FIG. 26] FIG. 26 is a graph showing the results of ELISA analysis of M-CSF.
[FIG. 27] FIG. 27 is a graph showing the results of ELISA analysis of M-CSF.
[FIG. 28] FIG. 28 is a graph showing the results of ELISA analysis of M-CSF.
[FIG. 29] FIG. 29 is a graph showing the results of qPCR analysis of Osteoprotegerin.
[FIG. 30] FIG. 30 is a graph showing the results of qPCR analysis of M-CSF.
[FIG. 31] FIG. 31 is graphs showing the results of ELISA analysis of Osteoprotegerin. FIG. 31(a) shows the results under carnosine-added conditions and FIG. 31(b) shows the results under anserine-added conditions.
[FIG. 32] FIG. 32 is graphs showing the results of qPCR analysis of Osteoprotegerin. FIG. 32(a) shows the results under UC_B_C conditions, FIG. 32(b) for BM_B_C conditions, and FIG. 32(c) for BM_C_C conditions.
[FIG. 33] FIG. 33 is graphs showing the results of qPCR analysis of Osteoprotegerin. FIG. 33(a) shows the results under UC_B_A conditions, FIG. 33(b) for BM_B_A conditions, and FIG. 33(c) for BM_C_A conditions.
[FIG. 34] FIG. 34 is graphs showing the results of ELISA analysis of M-CSF. FIG. 34(a) shows the results under carnosine-added conditions and FIG. 34(b) shows the results under anserine-added conditions.
[FIG. 35] FIG. 35 is graphs showing the results of qPCR analysis of M-CSF. FIG. 35(a) shows the results under UC_B_C conditions and FIG. 35(b) for BM_B_C conditions.
[FIG. 36] FIG. 36 is graphs showing the results of qPCR analysis of M-CSF. FIG. 36(a) shows the results under UC_B_A conditions and FIG. 36(b) for BM_B_A conditions.
[FIG. 37] FIG. 37 is graphs showing the results of counting cells in group 1 or group 2.
[FIG. 38] FIG. 38 is graphs showing the results of counting cells in group 3 or group 4.
[FIG. 39] FIG. 39 is graphs showing the results of measuring a phalloidin fluorescence area in groups 1 to 4.
[FIG. 40] FIG. 40 is graphs showing the results of measuring a CD44 fluorescence area in groups 1 to 4.
[FIG. 41] FIG. 41 is images showing the results of photographing fluorescent images (cell nucleus staining) of group 1 or 3.
[FIG. 42] FIG. 42 is images showing the results of photographing fluorescent images (phalloidin staining) of group 1 or 3.
[FIG. 43] FIG. 43 is images showing the results of photographing fluorescent images (CD44 staining) of group 1 or 3.
[FIG. 44] FIG. 44 is graphs showing the results of counting cells in group 5 or group 6.
[FIG. 45] FIG. 45 is graphs showing the results of counting cells in group 7 or group 8.
[FIG. 46] FIG. 46 is graphs showing the results of measuring a phalloidin fluorescence area in group 5 or 6.
[FIG. 47] FIG. 47 is graphs showing the results of measuring a phalloidin fluorescence area in group 7 or 8.
[FIG. 48] FIG. 48 is graphs showing the results of measuring a CD44 fluorescence area in group 5 or 6.
[FIG. 49] FIG. 49 is graphs showing the results of measuring a CD44 fluorescence area in group 7 or 8.
[FIG. 50] FIG. 50 is images showing the results of photographing fluorescent images (phalloidin staining) of group 5 or 7.
[FIG. 51] FIG. 51 is images showing the results of photographing fluorescent images (CD44 staining) of group 5 or 7.
[FIG. 52] FIG. 52 is a table showing the results of measuring the positive rate of each MSC marker after culturing umbilical cord MSCs in MSC medium D.
[FIG. 53] FIG. 53 is a graph showing the results of counting cells in group 9 or group 10.
[FIG. 54] FIG. 54 is a graph showing the results of measuring a phalloidin fluorescence area in group 9 or 10.
[FIG. 55] FIG. 55 is images showing the results of photographing fluorescent images (phalloidin staining) of group 9 or 10.

### Description of Embodiments

Hereinafter, embodiments of the present invention will be described in detail. Note that repeated descriptions of the same content are omitted, if appropriate, so as to avoid redundancy.

### (1) Method

An embodiment of the present invention provides a method comprising the step of bringing mesenchymal stem cells and an imidazole dipeptide into contact with each other. This method allows for the production of a culture supernatant useful for disease suppression or amelioration (e.g., a culture supernatant containing high concentrations of at least one or more cytokines useful for disease suppression or amelioration, or a culture supernatant useful for inhibiting osteogenic differentiation). This method may comprise, for example, a step of culturing MSCs in a medium containing an imidazole dipeptide to generate cultured MSCs, or a step of recovering a culture supernatant from a medium containing cultured MSCs. Examples of the method include: a method for producing cells or a method of culturing cells; a method for producing a culture supernatant; a method for producing a pharmaceutical composition; a method for producing a composition containing a cytokine(s); a method of promoting cytokine or exosome secretion; a method of inhibiting generation of osteoblasts; or a method of inhibiting osteogenic differentiation. Also, one embodiment of the present invention provides cells, a culture supernatant, a pharmaceutical composition, or a cytokine-containing composition, as obtained by using this method, or a container containing the same.

### (2) Method

An embodiment of the present invention provides a method for producing a culture supernatant, comprising the step of recovering a culture supernatant from a medium containing MSCs and an imidazole dipeptide. This method may be used to produce a culture supernatant useful for disease suppression or amelioration (e.g., a culture supernatant containing high concentrations of at least one or more cytokines useful for disease suppression or amelioration, or a culture supernatant useful for inhibiting osteogenic differentiation).

### (3) Method

An embodiment of the present invention provides a method of culturing cells or a method for producing cells, the method comprising the step of culturing MSCs in a medium containing an imidazole dipeptide to generate cultured cells. This method may be used to produce MSCs useful for disease suppression or amelioration (e.g., MSCs that secrete a high concentration of at least one or more cytokines useful for disease suppression or amelioration, MSCs useful for inhibiting osteogenic differentiation). In this method, MSCs may be cultured in a serum-free, serum albumin-free, insulin-free, IGF (Insulin-like growth factor)-free (e.g., IGF-1-free), FGF (Fibroblast growth factor)-free (e.g., FGF-1 or 2-free ), growth factor-free, cytokine-free, or protein-free medium to produce MSCs with excellent safety. If a culture supernatant is recovered from a medium containing cells obtained by this method, it is possible to produce a culture supernatant useful for disease suppression or amelioration (e.g., a culture supernatant containing high concentrations of at least one or more cytokines useful for disease suppression or amelioration, or a culture supernatant useful for inhibiting osteogenic differentiation).

### (4) Culture supernatant

An embodiment of the present invention provides a culture supernatant obtained by culturing MSCs in a medium containing an imidazole dipeptide. Use of this culture supernatant can provide, for example, effects characteristic of MSC culture supernatants or effects useful for disease suppression or amelioration (e.g., an effect exerted by high concentrations of at least one or more cytokines useful for disease suppression or amelioration, or an effect of inhibiting osteogenic differentiation).

### (5) Method

An embodiment of the present invention provides a method of suppressing a disease, the method comprising the step of administering to a subject a culture supernatant obtained by culturing MSCs in a medium containing an imidazole dipeptide. This method may be used to suppress or ameliorate osteogenic differentiation (e.g., suppress or ameliorate diseases associated with osteogenic differentiation). Another aspect provides use of a pharmaceutical composition containing a culture supernatant obtained by culturing MSCs in a medium containing an imidazole dipeptide in the manufacture of a pharmaceutical composition for disease suppression.

### (6) Composition

An embodiment of the present invention provides a pharmaceutical composition comprising a culture supernatant obtained by culturing MSCs in a medium containing an imidazole dipeptide. This composition may be used to suppress or ameliorate osteogenic differentiation (e.g., suppress or ameliorate diseases associated with osteogenic differentiation). Another aspect provides a pharmaceutical composition used for disease suppression, the pharmaceutical composition comprising a culture supernatant obtained by culturing MSCs in a medium containing an imidazole dipeptide.

### (7) Method

An embodiment of the present invention provides a method of suppressing a disease, the method comprising the step of administering to a subject cells obtained by culturing MSCs in a medium containing an imidazole dipeptide. This method may be used to suppress or ameliorate osteogenic differentiation (e.g., suppress or ameliorate diseases associated with osteogenic differentiation). Another aspect provides use of a pharmaceutical composition containing cells obtained by culturing MSCs in a medium containing an imidazole dipeptide in the manufacture of a pharmaceutical composition for disease suppression.

### (8) Composition

An embodiment of the present invention provides a pharmaceutical composition comprising cells obtained by culturing MSCs in a medium containing an imidazole dipeptide. This composition may be used to suppress or ameliorate osteogenic differentiation (e.g., suppress or ameliorate diseases associated with osteogenic differentiation). Another aspect provides a pharmaceutical composition used for disease suppression, the pharmaceutical composition comprising cells obtained by culturing MSCs in a medium containing an imidazole dipeptide.

### (9) Method

An embodiment of the present invention provides a method of inhibiting osteogenic differentiation of cells of a subject, the method comprising the step of administering to a subject a culture supernatant obtained by culturing MSCs in a medium containing an imidazole dipeptide. This method may be used to inhibit osteogenic differentiation of cells of a subject. Another aspect provides use of a pharmaceutical composition containing a culture supernatant obtained by culturing MSCs in a medium containing an imidazole dipeptide in the manufacture of a pharmaceutical composition for inhibiting osteogenic differentiation of cells of a subject. Another aspect provides a pharmaceutical composition used for inhibiting osteogenic differentiation of cells of a subject, the pharmaceutical composition comprising a culture supernatant obtained by culturing MSCs in a medium containing an imidazole dipeptide.

### (10) Method

An embodiment of the present invention provides a method of inhibiting osteogenic differentiation of cells of a subject, the method comprising the step of administering to a subject cells obtained by culturing MSCs in a medium containing an imidazole dipeptide. This method may be used to inhibit osteogenic differentiation of cells of a subject. Another aspect provides use of a pharmaceutical composition containing cells obtained by culturing MSCs in a medium containing an imidazole dipeptide in the manufacture of a pharmaceutical composition for inhibiting osteogenic differentiation of cells of a subject. Another aspect provides a pharmaceutical composition used for inhibiting osteogenic differentiation of cells of a subject, the pharmaceutical composition comprising cells obtained by culturing MSCs in a medium containing an imidazole dipeptide.

### (11) Medium

An embodiment of the present invention provides a medium for MSC culture, the medium comprising an imidazole dipeptide and being serum-free, serum albumin-free, insulin-free, IGF-free, FGF-free, growth factor-free, cytokine-free, or protein-free. MSCs may be cultured using this medium to produce cells useful for disease suppression or amelioration (e.g., cells secreting high concentrations of at least one or more cytokines useful for disease suppression or amelioration, or cells useful for inhibiting osteogenic differentiation). Alternatively, it is possible to produce a culture supernatant useful for disease suppression or amelioration (e.g., a culture supernatant containing high concentrations of at least one or more cytokines useful for disease suppression or amelioration, or a culture supernatant useful for inhibiting osteogenic differentiation). By culturing MSCs in this medium, MSCs or a culture supernatant with excellent safety characteristics can be obtained.

### (12) Method

An embodiment of the present invention provides a method of inhibiting generation of osteoblasts, the method comprising the step of bringing MSCs and an imidazole dipeptide into contact with each other. This method may be used to inhibit generation of osteoblasts. Another aspect provides use of an imidazole dipeptide-containing composition in the manufacture of a composition for inhibiting generation of osteoblasts. Still another aspect of the present invention provides a method of inhibiting generation of osteoblasts, the method comprising the step of administering an imidazole dipeptide to a subject.

### (13) Method

An embodiment of the present invention provides a method of inhibiting generation of osteoblasts, the method comprising the step of culturing MSCs in a medium containing an imidazole dipeptide. This method may be used to inhibit generation of osteoblasts.

### (14) Composition

An embodiment of the present invention provides a composition for inhibiting generation of osteoblasts, comprising an imidazole dipeptide. This composition may be brought into contact with MSCs to inhibit generation of osteoblasts. Another aspect provides a composition used for inhibiting generation of osteoblasts, the composition comprising an imidazole dipeptide.

### (15) Method

An embodiment of the present invention provides a method of inhibiting generation of osteoblasts, comprising the step of bringing MSCs into contact with a culture supernatant or MSC-secreted material obtained by culturing MSCs in a medium containing an imidazole dipeptide. This method may be used to inhibit generation of osteoblasts. Another aspect provides use of a composition containing a culture supernatant or MSC-secreted material obtained by culturing MSCs in a medium containing an imidazole dipeptide in the manufacture of a composition for inhibiting generation of osteoblasts. Another aspect provides a method of inhibiting generation of osteoblasts, comprising the step of administering to a subject a culture supernatant or MSC-secreted material obtained by culturing MSCs in a medium containing an imidazole dipeptide.

### (16) Composition

An embodiment of the present invention provides a composition for inhibiting generation of osteoblasts, comprising a culture supernatant or MSC-secreted material obtained by culturing MSCs in a medium containing an imidazole dipeptide. This composition may be brought into contact with MSCs to inhibit generation of osteoblasts. Another aspect provides a composition used for inhibiting generation of osteoblasts, the composition comprising a culture supernatant or MSC-secreted material obtained by culturing MSCs in a medium containing an imidazole dipeptide.

### (17) Method

An embodiment of the present invention provides a method of promoting expression of G-CSF (granulocytes colony stimulating factor), MCP-1 (monocyte chemoattractant protein), VEGF-C (vascular endothelial growth factor-C), TGF-β1 (transforming growth factor β1), IL-6 (interleukin 6), IL-7 (interleukin 7), IL-8 (interleukin 8), M-CSF, Osteoprotegerin, Gremlin 1, SCF, FGF11, R-spondin 2, DAM8, IL-34, IGFBP2, DKK1, semaphorin 3B, or an exosome marker, the method comprising the step of culturing MSCs in a medium containing an imidazole dipeptide. This method may be used to promote expression or secretion of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker from MSCs.

### (18) Method

An embodiment of the present invention provides a method of promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker, the method comprising the step of bringing MSCs and an imidazole dipeptide into contact with each other. This method may be used to promote expression or secretion of G-CSF, MCP-1, VEGF-C, TGF-β1, IL-6, IL-7, IL-8, or an exosome marker from MSCs. Another aspect provides use of an imidazole dipeptide-containing composition in the manufacture of a composition for promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker of MSCs. Still another aspect provides a method of promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker of MSCs, the method comprising the step of administering an imidazole dipeptide to a subject.

### (19) Composition

An embodiment of the present invention provides a composition for promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker of MSCs, the composition comprising an imidazole dipeptide. This composition may be brought into contact with MSCs to promote expression or secretion of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker from MSCs.

### (20) Method

An embodiment of the present invention provides a method of promoting exosome secretion, the method comprising the step of culturing MSCs in a medium containing an imidazole dipeptide. This method may be used to promote secretion of exosomes from MSCs.

### (21) Method

An embodiment of the present invention provides a method of promoting exosome secretion, the method comprising the step of bringing MSCs and an imidazole dipeptide into contact with each other. This method may be used to promote secretion of exosomes from MSCs. Another aspect provides use of an imidazole dipeptide-containing composition in the manufacture of a composition for promoting secretion of exosomes from MSCs. Still another aspect provides a method of promoting exosome secretion, the method comprising the step of administering an imidazole dipeptide to a subject.

### (22) Composition

An embodiment of the present invention provides a composition for promoting secretion of exosomes from MSCs, comprising an imidazole dipeptide. This composition may be brought into contact with MSCs to promote secretion of exosomes from MSCs.

### (23) Method

An embodiment of the present invention provides a method for producing a composition, comprising the step of recovering a culture supernatant from a medium containing MSCs and an imidazole dipeptide. This composition may be a culture supernatant or a cytokine-containing composition. This composition may be used to produce an effect of MSC culture supernatant or an effect useful for disease suppression or amelioration (e.g., an effect exerted by high concentrations of at least one or more cytokines useful for disease suppression or amelioration, or an effect of inhibiting osteogenic differentiation).

### (24) Method

An embodiment of the present invention provides a method for producing a composition containing an MSC secreted material, comprising the step of bringing MSCs and an imidazole dipeptide into contact with each other. This composition may be used to produce an effect of MSC secreted material or an effect useful for disease suppression or amelioration (e.g., an effect exerted by high concentrations of at least one or more cytokines useful for disease suppression or amelioration, or an effect of inhibiting osteogenic differentiation).

### (25) Culture supernatant

An embodiment of the present invention provides an MSC culture supernatant comprising protein. The culture supernatant may be, for example, an MSC culture supernatant containing at least 500 pg/ml of G-CSF. This culture supernatant may be used to produce an effect of suppressing or ameliorating a disease by highly concentrated G-CSF. The culture supernatant may be, for example, an MSC culture supernatant containing at least 2700 pg/ml of IL-6. This culture supernatant may be used to produce an effect of suppressing or ameliorating a disease by highly concentrated IL-6.

### (26) Cells

An embodiment of the present invention provides an MSC (preferably an umbilical cord-derived MSC) that expresses or secretes a high level of G-CSF or IL-6. This culture supernatant may be used to produce an effect of suppressing or ameliorating a disease by highly concentrated IL-6. This cell may be used to produce an effect of suppressing or ameliorating a disease by highly concentrated G-CSF or IL-6. Alternatively, if a culture supernatant is recovered from a medium containing the cells, the culture supernatant containing high concentrations of G-CSF or IL-6 can be obtained. Another aspect provides a composition comprising a cell population of MSCs, wherein 60% or more of the cells in the composition are MSCs (preferably umbilical cord-derived MSCs) that highly express or highly secrete G-CSF or IL-6. This composition may be used to produce an effect of suppressing or ameliorating a disease by highly concentrated G-CSF or IL-6. If a culture supernatant is recovered from this composition, a culture supernatant containing high concentrations of G-CSF or IL-6 can be obtained.

### (27) Cells

An embodiment of the present invention provides an MSC positive for G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, or semaphorin 3B. This MSC may be a purified or isolated MSC. Another aspect provides a composition comprising a purified or isolated form of MSCs. Another aspect provides an MSC having an increased level of expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, or semaphorin 3B. The increase in expression may be, for example, an increase when compared to that in MSCs cultured in the absence of an imidazole dipeptide. Another aspect provides a pharmaceutical composition containing the MSCs. Another aspect provides a method of treating a disease, comprising the step of administering the MSCs to a subject. Whether or not it is positive may be evaluated, for example, by immunological assay (e.g. ELISA) or PCR (e.g. quantitative PCR).

### (28) Cells

An embodiment of the present invention provides an IL-34-positive or ADAM8-positive MSC. This MSC is an MSC particularly suitable for use in inhibiting osteogenic differentiation. This MSC may be a purified or isolated MSC. Another aspect provides a composition comprising a purified or isolated form of MSCs. Another aspect provides a pharmaceutical composition containing the MSCs. Another aspect provides a method of treating a disease, comprising the step of administering the MSCs to a subject.

### (29) Method

An embodiment of the present invention provides a method of changing, during MSC culture, a property of a medium so as to promote more secretion of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker from MSCs, the method comprising the step of adding an imidazole dipeptide to the medium. This method may be used to prepare a medium suitable for producing a culture supernatant useful for disease suppression or amelioration, or for producing MSCs. Another aspect provides a method of preparing a medium, the method comprising the step of adding an imidazole dipeptide to a medium.

### (30) Medium

An embodiment of the present invention provides a medium comprising an imidazole dipeptide and a LIF component. This medium may be used to promote MSC cell proliferation.

### (31) Method

An embodiment of the present invention provides a method for producing cells or a culture supernatant, the method comprising the step of culturing MSCs in a medium containing an imidazole dipeptide and a LIF component to generate cultured cells. The obtained cells may be used to produce an effect of inhibiting osteogenic differentiation. The resulting culture supernatant may be used to produce an effect of MSC culture supernatant or an effect useful for disease suppression or amelioration (e.g., an effect exerted by high concentrations of at least one or more cytokines useful for disease suppression or amelioration, or an effect of inhibiting osteogenic differentiation).

### (32) Kit

An embodiment of the present invention provides a kit comprising an imidazole dipeptide and a LIF component. This kit may be, for example, a kit for preparing a culture medium (e.g., a medium for MSC culture). The components included in this kit may be mixed to prepare a medium. The resulting medium may be used to promote MSC cell proliferation. Another aspect provides a method for preparing a medium, the method comprising the step of mixing an imidazole dipeptide and a LIF component.

### (33) Medium

An embodiment of the present invention provides a medium comprising an imidazole dipeptide and a laminin fragment or a modified laminin fragment. This medium may be used to promote MSC cell proliferation.

### (34) Method

An embodiment of the present invention provides a method for producing cells or a culture supernatant, the method comprising the step of culturing MSCs in a medium containing an imidazole dipeptide and a laminin fragment or a modified laminin fragment to generate cultured cells. The obtained cells may be used to produce an effect of inhibiting osteogenic differentiation. The resulting culture supernatant may be used to produce an effect of MSC culture supernatant or an effect useful for disease suppression or amelioration (e.g., an effect exerted by high concentrations of at least one or more cytokines useful for disease suppression or amelioration, or an effect of inhibiting osteogenic differentiation).

### (35) Kit

An embodiment of the present invention provides a kit comprising an imidazole dipeptide and a laminin fragment or a modified laminin fragment. This kit may be, for example, a kit for preparing a culture medium (e.g., a medium for MSC culture). The components included in this kit may be mixed to prepare a medium. The resulting medium may be used to promote MSC cell proliferation. Another aspect provides a method for preparing a medium, the method comprising the step of mixing an imidazole dipeptide and LIF.

### (36) Method

An embodiment of the present invention provides a method for producing a cytokine-containing composition, the method comprising the step of treating a culture supernatant (including, for example, (4) or (25) above) or a composition (including, for example, (6), (8), (14), (16), (19), or (22) above). This composition obtained by this method may be used to produce an effect of MSC culture supernatant, an effect exerted by high concentrations of at least one or more cytokines useful for disease suppression or amelioration, or an effect of inhibiting osteogenic differentiation. The treatment may be, for example, formulation or filling into a container.

### (37) Composition

An embodiment of the present invention provides a cytokine-containing composition obtained by performing the method according to the embodiment of the present invention (including, for example, (1) to (3), (5), (7), (9), (10), (12), (13), (15), (17), (18), (20), (21), (23), (24), (29), (31), (34), or (36) above). This composition may be used to produce an effect of MSC culture supernatant, an effect exerted by high concentrations of at least one or more cytokines useful for disease suppression or amelioration, or an effect of inhibiting osteogenic differentiation.

### (38) Cells

An embodiment of the present invention provides cells obtained by performing the method according to the embodiment of the present invention (e.g. including (1) to (3), (31), or (34) above). These cells can secrete high concentrations of at least one or more cytokines useful for disease suppression or amelioration. These cells can be used to suppress or ameliorate a disease. If a culture supernatant is recovered from a medium containing the cells, it is possible to produce a culture supernatant containing high concentrations of at least one or more cytokines useful for disease suppression or amelioration, or a culture supernatant useful for inhibiting osteogenic differentiation.

### (39) Cells

An embodiment of the present invention provides a cell population containing cells according to the embodiment of the present invention (e.g., including (26) to (28) or (38) above). The cells may be used to produce an effect of suppressing or ameliorating a disease by highly concentrated G-CSF or IL-6 or an effect of inhibiting osteogenic differentiation. Alternatively, if a culture supernatant is recovered from a medium containing the cells, the culture supernatant containing high concentrations of G-CSF or IL-6 or the culture supernatant useful for disease suppression or amelioration can be obtained. Another aspect provides a composition comprising a cell population containing the cells of (25) or (28) above. This composition may be used to produce an effect of suppressing or ameliorating a disease by highly concentrated G-CSF or IL-6 or an effect of inhibiting osteogenic differentiation. Alternatively, if a culture supernatant is recovered from a medium containing this composition, the culture supernatant containing high concentrations of G-CSF or IL-6 or the culture supernatant useful for disease suppression or amelioration can be obtained.

### (40) Method

An embodiment of the present invention provides a method of treating a disease, the method comprising the step of administering to a subject a supernatant (including, for example, (4) or (25) above) or cells (including, for example, (26) to (28) or (38)) according to the embodiment of the present invention. The subject to be treated may be, for example, a patient in need of inhibiting osteogenic differentiation. Another aspect provides a pharmaceutical composition for use in treatment of a disease, comprising the supernatant or cells of an embodiment of the present invention. Another aspect provides use of a supernatant or cells of an embodiment of the present invention in the manufacture of a pharmaceutical composition for treating a disease.

### (41) Container

An embodiment of the present invention provides a container comprising a culture supernatant (including, for example, (4) or (25) above) or a composition (including, for example, (6), (8), (14), (16), (19), (22), or (37) above) according to the embodiment of the present invention. This container may be used to produce an effect of MSC culture supernatant, an effect exerted by high concentrations of at least one or more cytokines useful for disease suppression or amelioration, or an effect of inhibiting osteogenic differentiation.

The method according to an embodiment of the present invention (including, for example, (1) to (3), (5), (7), (9), (10), (12), (13), (15), (17), (18), (20), (21), (23), (24), (29), (31), (34), (36), or (40) above) may include one or more steps of the following (i) to (ii): step (i) of culturing MSCs in a medium containing an imidazole dipeptide to generate cultured MSCs; or step (ii) of recovering a culture supernatant from a medium containing cultured MSCs and an imidazole dipeptide. Also, the method according to an embodiment of the present invention (including, for example, (1) to (3), (5), (7), (9), (10), (12), (13), (15), (17), (18), (20), (21), (23), (24), (29), (31), (34), (36), or (40) above) may include one or more steps of the following (iii) through (xvi): step (iii) of suspending MSCs in a medium and seeding the resulting cell suspension onto cultureware; step (iv) of adding a medium containing an adhesion factor to the medium after seeding and culturing MSCs; step (v) of mixing an imidazole dipeptide and medium components; step (vi) of replacing the medium for MSC culture by a medium containing an imidazole dipeptide; step (vii) of culturing MSCs in the medium containing an imidazole dipeptide; step (viii) of growing cells by culturing the MSCs in adhesion culture; step (ix) of centrifuging or filtering the medium containing the MSCs and the imidazole dipeptide after culture; step (x) of recovering a culture supernatant or cultured MSCs; step (xi) of measuring the level of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker in the culture supernatant; step (xii) of sterilizing the recovered culture supernatant to generate a sterilized culture supernatant; step (xiii) of formulating the culture supernatant or cultured MSCs; step (xiv) of administering to a subject a therapeutically effective amount of the culture supernatant or cultured MSCs; step (xv) of transferring the culture supernatant or cultured MSCs to a medical container; or step (xvi) of transferring the culture supernatant or cultured MSCs from the medical container to a syringe, vial or medical bag. One or more of these steps may be adopted, which is useful for producing a culture supernatant containing high concentrations of at least one or more cytokines useful for disease suppression or amelioration, or a culture supernatant or MSCs useful for inhibiting osteogenic differentiation. When two or more of the above steps are adopted, any order is acceptable. The order can be determined according to the desired operation.

In an embodiment of the present invention (including, for example, any of (1) to (41) above), examples of the MSCs include umbilical cord-derived MSCs, adipose-derived MSCs, bone marrow-derived MSCs, placenta-derived MSCs, or cord blood-derived MSCs. The MSCs may be mammalian MSCs, preferably human MSCs. Examples of the mammals include humans, monkeys, rodents (e.g., mice, hamsters), rabbits, dogs, cats, horses, cattle, sheep, pigs, goats, or marmosets. The MSCs may exist as a cell population. As used herein, unless otherwise specified, references to MSCs include MSCs existing as a cell population. The cell population contains multiple cells resulting from cell division. The percentage of MSCs in the embodiment of the present invention (including, for example, (25) or (28) above) in the cell population may be, for example, 30, 40, 50, 60, 70, 80, 90, or 100% or more, and may range between any two of those values. The MSCs include MSCs before culturing by the method described above, and MSCs obtained by culturing by the method described above. The MSCs include MSCs cultured in a medium containing an imidazole dipeptide. The MSCs include MSCs positive for HLA-ABC, MSCs positive for CD105, MSCs negative for HLA-ABC, or MSCs negative for CD105. The MSCs include MSCs positive for CD44, CD73, CD90, or CD105, or negative for CD45, CD34, CD31, or HLA-DR. The MSCs may be purified or isolated MSCs. Examples of the Isolated or purified MSCs include a cell population free of cells other than MSCs in practice, or MSCs in a preparation from which impurities have been removed. The impurities may include, for example, culture medium components, and the removal may include, for example, partial removal.

In an embodiment of the present invention (including, for example, any of (1) to (41) above), MSCs that highly express or highly secrete G-CSF or IL-6 may be, for example, cells that secrete at least 5.56 × 10⁻³ pg/cell of G-CSF or cells that secrete at least 3 × 10⁻² pg/cell of IL-6 when cultured in a medium containing carnosine (e.g., at 10 mM) for 72 hours. The amount of G-CSF secreted may be, for example, at least 5.56 × 10⁻³, 6 × 10⁻³, 8 × 10⁻¹, 1 × 10⁻², 1.2 × 10⁻², 1.7 × 10⁻², 2 × 10⁻², 3 × 10⁻², 1 × 10⁻¹, 1, or 3 pg/cell, or may be between any two of those values. This value may be, for example, 5.56 × 10⁻³ to 3, 5.56 × 10⁻³ to 3 × 10⁻², 5.56 × 10⁻³ to 2 × 10⁻², 1.2 × 10⁻² to 3 × 10⁻², or 1.2 × 10⁻² to 1.7 × 10⁻² pg/cell. The amount of IL-6 secreted may be, for example, at least 3×10⁻², 4×10⁻², 5×10⁻², 6×10⁻², 7 × 10⁻², 8×10⁻², 1 × 10⁻¹, 1.5 × 10⁻¹, 2 × 10⁻¹, 1, 10, or 20 pg/cell, or may be between any two of those values. This value may be, for example, 3 × 10⁻² to 20, 3 × 10⁻² to 2 × 10⁻¹, 3 × 10⁻² to 8 × 10⁻², 4 × 10⁻² to 8 × 10⁻², or 4 × 10⁻² to 7 × 10⁻² pg/cell. The calculation of the secreted amount can be shown as follows: Concentration of material secreted in a medium after culturing cells (unit: pg/mL, for example) × Volume of medium used (unit: mL, for example) / Number of cells after culturing.

In an embodiment of the present invention (including, for example, any of (1) to (41) above), the imidazole dipeptide includes a compound having a structure in which an amino acid having an imidazole group is bonded to another amino acid. Examples of the imidazole dipeptide include a compound having the structure of the following formula (1) or (2):

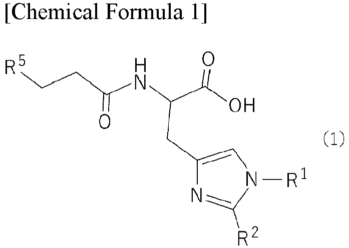

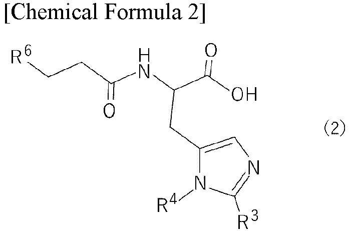

In the above formulas (1) and (2), the meanings of R¹ to R⁶ are as follows. R¹, R², R³, and R⁴ are each independently H or C₁₋₆ alkyl. R⁵ and R⁶ are each independently -NHR⁷ or -CH₂NHR⁷. Here, R⁷ is H or -COR⁸. Here, R⁸ is H, C₁₋₆ alkyl, optionally substituted phenyl, -OCH₂R⁹, or -CH=CHR⁹. Here, R⁹ is H, C₁₋₆ alkyl, or optionally substituted phenyl. The compound having a structure of formula (1) or (2) may have an effect of promoting secretion of cytokines from MSCs.

From the viewpoint of more efficiently promoting cytokine secretion by MSCs, it is preferable that one of R¹ or R² in formula (1) is H and the other is C₁₋₆ alkyl, and it is more preferable that both of them are H. Here, it is preferable that when one of R¹ or R² in formula (1) is C₁₋₆ alkyl, the C₁₋₆ alkyl is methyl. From the viewpoint of promoting cytokine secretion by MSCs, it is preferable that one of R³ or R⁴ in formula (2) is H and the other is C₁₋₆ alkyl, and it is more preferable that both of them are H. Here, it is preferable that when one of R¹ or R³ in formula (2) is C₁₋₆ alkyl, the C₁₋₆ alkyl is methyl. From the viewpoint of promoting cytokine secretion by MSCs, it is preferable that R⁸ in formula (1) or (2) is H, C₁₋₆ alkyl, or -OCH₃ and it is more preferable that R⁸ is methyl. From the viewpoint of promoting cytokine secretion by MSCs, it is preferable that R⁹ in formula (1) or (2) is H or C₁₋₆ alkyl. R⁷ in formula (1) or (2) may be formyl, acetyl, propionyl, benzoyl, or acryloyl. The optionally substituted phenyl of formula (1) or (2) may be, for example, unsubstituted phenyl, or phenyl substituted with -OH, C₁₋₆ alkyl, or -OCH₃. The substitution position may be at position 2, 3, 4, 5 or 6 of phenyl.

From the viewpoint of more efficiently promoting cytokine secretion by MSCs, in formula (1) and formula (2), R¹, R², R³, and R⁴ may be each independently H or C₁₋₆ alkyl, one of R¹ or R² may be H, one of R³ or R⁴ may be H, R⁵ and R⁶ may be each independently -NHR⁷ or -CH₂NHR⁷, and R⁷ may be H or -COCH₃.

The alkyl above included a linear or branched hydrocarbon chain. The C₁₋₆ above is a hydrocarbon having 1, 2, 3, 4, 5, or 6 carbon atoms. That is, the C₁₋₆ above is alkyl having 1, 2, 3, 4, 5, or 6 carbon atoms. Examples of the C₁₋₆ alkyl includes a methyl, ethyl, n-propyl, isopropyl, n-butyl, isobutyl, *sec*-butyl, *tert*-butyl, n-pentyl, or n-hexyl group.

For the method for producing a compound represented by formula (1) or (2), it is possible to use the methods and principles for the production of imidazole dipeptides described in JP-A-2020-022433, JP-A-2019-131532, JP-A-2010-31004, JP-A-2006-232686, or JP-A-2006-504701, etc.

In an embodiment of the present invention (including, for example, any of (1) to (41) above), the imidazole dipeptide includes carnosine, methylated carnosine (e.g., anserine or balenine), or homocarnosine. The imidazole dipeptide has a structure of formula (1) or (2), and includes a compound having an antioxidant action. For example, carnosine, anserine, balenine, and homocarnosine are known to have an antioxidant action (see, for example, Boldyrev et al., Physiol Rev. 2013 Oct; 93(4): 1803-45). The imidazole dipeptide may be an L- or D-form.

In an embodiment of the present invention (including, for example, any of (1) to (41) above), carnosine includes L-carnosine or D-carnosine, unless otherwise specified. L-carnosine may also be represented by the name beta-alanyl-L-histidine. L-carnosine may be denoted by CAS registration number: 305-84-0.

In an embodiment of the present invention (including, for example, any of (1) to (41) above), anserine includes L-anserine or D-anserine, unless otherwise specified. L-anserine may also be represented by the name beta-alanyl-3-methyl-L-histidine. L-anserine may be denoted by CAS registration number: 584-85-0.

In an embodiment of the present invention (including, for example, any of (1) to (41) above), balenine includes L-balenine or D-balenine, unless otherwise specified. L-balenine can also be represented by the name beta-alanyl-1-methyl-L-histidine. L-balenine may be denoted by CAS registration number: 331-38-4.

In an embodiment of the present invention (including, for example, any of (1) to (41) above), homocarnosine includes L-homocarnosine or D-homocarnosine, unless otherwise specified. L-homocarnosine may also be represented by the name gamma-aminobutyryl-L-histidine. L-homocarnosine may be denoted by CAS registration number: 3650-73-5.

In an embodiment of the present invention (including, for example, any of (1) to (41) above), examples of the effect elicited by a highly concentrated cytokine include an effect elicited by highly concentrated G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, or semaphorin 3B. Since G-CSF is a cytokine that inhibits bone formation, it is considered that high concentrations of G-CSF can inhibit bone formation. Since IL-6 is a cytokine that inhibits bone formation, highly concentrated IL-6 may inhibit bone formation. Since IL-6, VEGF, MCP-1, VEGF-C, TGF-β1, and IL-8 are angiogenic factors, it is considered that high concentrations of these cytokines can promote angiogenesis. Since G-CSF, MCP-1, TGF-β1, and IL-7 are immunomodulators, these cytokines may suppress autoimmune diseases. Since M-CSF, Osteoprotegerin, Gremlin 1, SCF, FGF11, R-spondin 2, ADAM8, IL-34, IGFBP2, DKK1, and semaphorin 3B are factors involved in bone formation inhibition, the combination of these cytokines may inhibit bone formation.

In an embodiment of the present invention (including, for example, any of (1) to (41) above), examples of the disease or symptom to be suppressed or ameliorated include bone differentiation or bone differentiation-associated diseases (e.g., ectopic ossification), ischemic diseases (e.g., lower extremity ischemia, myocardial infarction, cerebral infarction, spinal cord infarction, or chronic arterial occlusive disease), wounds (e.g., epithelial wounds or burns), aging-associated sarcopenia, arthritis (e.g., rheumatism, herniated disc, osteoarthritis), inflammatory diseases (e.g., nephritis, keratitis, cytokine storm), psychiatric disorders (e.g., autism or insomnia, which may be due in part to neuroinflammation), immunological diseases (e.g., GVHD (graft-versus-host disease), Sjogren's syndrome, atopic dermatitis, connective tissue disease, multiple sclerosis, or autoimmune disease), or cancer. The disease or symptom may be inhibited or ameliorated, for example, by administering to a subject the culture supernatant, cells, or composition in an embodiment of the present invention (including, for example, any of (1) to (41) above). The disease or symptom may be suppressed or ameliorated, for example, by the effects elicited by highly concentrated cytokines. Ectopic ossification above includes the phenomenon of abnormal bone formation at sites where bone formation does not normally occur. Examples of the ectopic ossification include ossification in soft tissues (e.g., tendons, membranes, ligaments, muscles, joint capsules). The ectopic ossification may be a condition with a trabecular structure. Examples of the ectopic ossification include posterior longitudinal ligament ossification, yellow ligament ossification, progressive ossifying fibrodysplasia, progressive ossifying myositis, traumatic ossifying myositis, or diffuse idiopathic skeletal hyperostosis.

In an embodiment of the present invention (including, for example, any of (1) to (41) above), the osteogenic differentiation inhibition is, for example, useful for inhibiting osteogenic differentiation-associated disease. Examples of the osteogenic differentiation-associated disease include ossification (e.g., ectopic ossification). Examples of the osteogenic differentiation inhibition include inhibition of abnormal bone differentiation. In addition, use of osteogenic differentiation inhibition also includes use of suppression or amelioration of osteogenic differentiation-associated disease. Examples of the pharmaceutical composition for inhibiting osteogenic differentiation include a pharmaceutical composition for osteogenic differentiation-associated disease such as ectopic ossification. Examples of the method of inhibiting osteogenic differentiation include a method of suppressing or ameliorating an osteogenic differentiation-associated disease such as ectopic ossification.

In an embodiment of the present invention (including, for example, any of (1) to (41) above), the culturing includes incubating cells under conditions suitable for growth or maintenance. The incubation may be performed at about 37°C and about 5% CO₂. The culture may be performed in a serum-free, no-serum-containing, serum albumin-free, insulin-free, IGF-free, FGF-free, growth factor-free, cytokine-free, or protein-free medium. The term "free" includes the state in which the target component is not added to the medium, the medium fails to completely contain the target component, or the medium does not contain the component at a concentration above the detection limit. The term "free" includes wording "substantially free" or "fails to substantially contain". The culture may be carried out by adhesion culture in the presence of cell adhesion factors (e.g., while mixed in culture medium or in pre-coated cultureware). If the culture involves cell growth, the culture may include cell production. The culturing time may be, for example, 0, 1, 2, 3, 4, 5, 10, 15, 20, 25, 30, 35, 40, or 50 days or longer, and may be between any two of those values. The culture may be conducted until the cells become confluent. The confluent state includes cells covering at least 80% of the bottom area of cultureware. More preferably, cells can be cultured until the cells cover 80 to 90% of the bottom area of cultureware. MSC culture may be performed by alternately using X and Y, where the medium X is an imidazole dipeptide-free medium (e.g., a protein-containing medium) and the medium Y is an imidazole dipeptide-containing medium (e.g., a protein-free medium). Alternate use includes starting a culture in X or Y, then replacing the medium X by the medium Y after culturing or the medium Y by medium X after culturing. For example, culture in X, replacement by and culture in Y, replacement by and culture in X, replacement by and culture in Y, replacement by and culture in X, replacement by and culture in Y, replacement by and culture in X, replacement by and culture in Y, replacement by and culture in X, and replacement by and culture in Y may be performed in this order. Under conditions in which X and Y are used alternately to culture MSCs in this manner, the culture in Y may be performed at least 1, 2, 3, 4, 5, 6, or 7 times. A culture supernatant may be recovered at any timing after culture in Y. Under conditions in which X and Y are used alternately to culture MSCs as described above, the culture supernatant may be recovered after 1st, 2nd, 3rd, 4th, 5th, 6th, or 7th culture. The supernatant may be recovered at any two or more timings above, or at all of these timings, and the solution obtained by mixing all the supernatants may be used as a target supernatant. The time from the start of culture to replacement may be the same as the time of incubation described above.

In an embodiment of the present invention (including, for example, any of (1) to (41) above), MSCs may be cultured in a medium containing, for example, 0.1 to 100 mM of imidazole dipeptide. This concentration may be, for example, 0.1, 0.2, 0.5, 1, 2, 5, 7, 10, 15, 20, 25, 30, 35, 40, 50, 60, 80, or 100 mM, and may be the above value or more or between any two of those values. From the viewpoint of more efficiently obtaining a culture supernatant containing high concentrations of at least one or more cytokines useful for suppressing or ameliorating a disease, this concentration is preferably 1 to 50 mM, more preferably 3 to 30 mM, still more preferably 5 to 25 mM , particularly preferably 5 to 20 mM, and most preferably 10 to 20 mM. The culture may be adhesion culture. Those skilled in the art can easily check whether the cells are adhering to cultureware, for example, by steps such as a step of tilting the cultureware to check that no cell migration occurs, or a step of checking that no cell migration occurs when the medium in the cultureware is changed. Adhesion culture may be implemented by a step in which cells are cultured in the presence of a cell adhesion factor (e.g., the step includes: a step of bringing cells and an adhesion factor into contact with each other in cultureware; a step of mixing a cell suspension containing cells and medium with an adhesion factor, placing the resulting mixture in cultureware, and culturing the cells; or a step of coating (e.g., precoating) cultureware with an adhesion factor and seeding cells in the cultureware). As used herein, the terms "cell adhesion factor" and "adhesion factor" refer to the same object. Examples of the adhesion factor include laminin, fibronectin, vitronectin, tenascin, cadherin, poly-L-lysine, poly-D-lysine, collagen, thrombospondin, galectin, or nidogen-1, or fragments thereof with cell adhesion properties. Cell adhesion may include the state of adhesion between cells and cultureware via an extracellular matrix (e.g., an adhesion factor). Adherent cells may include cells that can be subjected to adhesion culture or cells that can grow while adhering.

In the above adhesion culture, the concentration of adhesion factor in the medium may be, for example, 0.01, 0.1, 0.25, 0.5, 0.75, 1, 1.25, 1.5, 2, or 3 µg/ml and may be the above value or more or between any two of those values. This value may be, for example, 0.01 to 2, 0.1 to 1.5, or 0.5 to 1 µg/ml. The concentration of adhesion factor in the medium may be, for example, 0.005, 0.01, 0.1, 0.25, 0.5, 1, 1.5, or 2 µg per cm² of culture area in the cultureware, and may be the above value or more or between any two of those values. This value may be, for example, 0.01 to 1.5, 0.1 to 1, or 0.1 to 0.5 µg per cm² of culture area in the cultureware. The adhesion factor is a factor having adhesion activity and examples include laminin, fibronectin, vitronectin, tenascin, cadherin, poly-L-lysine, poly-D-lysine, collagen, thrombospondin, galectin, nidogen-1, or a fragment thereof, or a modified material thereof. The adhesion factor may be a laminin fragment or a modified laminin fragment. The laminin fragment may have integrin-binding activity and may be of human origin. The laminin fragment may be laminin E8 fragment. The laminin fragment may be laminin 511 E8 fragment, laminin 521 E8 fragment, laminin 411 E8 fragment, laminin 421 E8 fragment, laminin 332 E8 fragment, laminin 311 E8 fragment, laminin 321 E8 fragment, laminin 211 E8 fragment, laminin 221 E8 fragment, laminin 213 E8 fragment, laminin 111 E8 fragment, or laminin 121 E8 fragment. The laminin 511 E8 fragment may be prepared, for example, by the method described in WO2011/043405A1. Laminin 511 includes laminin composed of α5, β1 and γ1 subunit chains. The modified laminin fragment may be a known complex including a laminin fragment having integrin-binding activity and another functional molecule (e.g., a complex of a laminin fragment having integrin-binding activity and a cell adhesion molecule, or a complex of a laminin fragment having integrin-binding activity and a growth factor binding molecule (e.g., heparan sulfate)) (see, for example, WO2012/137970, WO2014/103534, and WO2016/010082). The modified laminin fragment may be produced as a recombinant protein by using known genetic recombination techniques. The above laminin fragment or modified laminin fragment may have integrin-binding activity, and examples include: (a) a trimer containing a polypeptide having the amino acid sequence set forth in SEQ ID NO: 1, a polypeptide having the amino acid sequence set forth in SEQ ID NO: 2, and a polypeptide having the amino acid sequence set forth in SEQ ID NO: 3; (b) a trimer containing a polypeptide with an amino acid sequence having 90% or more homology to the amino acid sequence set forth in SEQ ID NO: 1, a polypeptide with an amino acid sequence having 90% or more homology to the amino acid sequence set forth in SEQ ID NO: 2, and a polypeptide with an amino acid sequence having 90% or more homology to the amino acid sequence set forth in SEQ ID NO: 3; or (c) a trimer containing a polypeptide with an amino acid sequence having one to several amino acid deletions, substitutions, insertions or additions to the amino acid sequence set forth in SEQ ID NO: 1, a polypeptide with an amino acid sequence having one to several amino acid deletions, substitutions, insertions or additions to the amino acid sequence set forth in SEQ ID NO: 2, and a polypeptide with an amino acid sequence having one to several amino acid deletions, substitutions, insertions or additions to the amino acid sequence set forth in SEQ ID NO: 3. The above laminin fragments, modified laminin fragments, or subunit chains may optionally have a tag (see, e.g., Mishra, Curr Protein Pept Sci. 2020; 21(8): 821-830) at the N or C terminus. The tag may be, for example, a tag for purification. The tag for purification may be, for example, a peptide tag (e.g. His tag). Examples of the tag for purification include a biochemically inert tag. The cell seeding density may be, for example, 1 × 10e1, 1 × 10e2, 5 × 10e2, 1 × 10e3, 1.5 × 10e3, 1 × 10e4, 1 × 10e5, or 1 × 10e6 cells/mL, and may be the above value or more or between any two of those values. This value may be, for example, 1 × 10e1 to 1 × 10e5, 1 × 10e2 to 1 × 10e4, 5 × 10e2 to 5 × 10e4, or 5 × 10e2 to 1 × 10e4 cells/mL. The cell seeding density may be, for example, 0.002, 0.01, 0.1, 1, 50, 100, 300, 500, 1000, 1500, 2000 cells/cm², and may be the above value or more or between any two of those values. This value may be, for example, 0.01 to 2000, 0.1 to 1000, 1 to 1000, 1 to 500, or 1 to 100 cells/cm².

In an embodiment of the present invention (including, for example, any of (1) to (41) above), the culture supernatant includes a culture supernatant obtained by culturing cells. Examples of the culture supernatant include a cytokine-containing culture supernatant. The culture supernatant may contain, for example, cellular metabolites (e.g., amino acids, lipids, sugars), secretory proteins (e.g., hormones, peptides, cytokines, extracellular matrix), or exosomes. When MSCs are cultured in a medium containing an imidazole dipeptide, the culture supernatant may contain the imidazole dipeptide. The culture supernatant includes, in the scope, a supernatant separated from cell components after various treatments (e.g., centrifugation, filtration, freezing, lyophilization, preservation, sterilization). The culture supernatant may be a culture supernatant obtained using a medium containing only MSCs as cultured cells. From the viewpoint of production cost reduction or lot-to-lot uniformity, it is preferable that the cytokines contained in the culture supernatant are only those derived from cultured cells. From the viewpoint of production cost reduction, it is preferable that the culture supernatant be non-enriched. The culture supernatant may contain, for example, G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or exosomes.

In an embodiment of the present invention (including, for example, any of (1) to (41) above), the culture supernatant may contain at least 500 pg/mL of G-CSF or at least 2700 pg/mL of IL-6. This culture supernatant may be used to produce an effect of highly concentrated G-CSF or IL-6. The culture supernatant may contain, for example, 500 to 2000, 700 to 1700, 800 to 1600, or 1000 to 1600 pg/mL of G-CSF. This concentration may be, for example, at least 500, 600, 700, 800, 900, 1000, 1250, 1500, 1600, 1700, 1800, 1900, or 2000 pg/mL, and may be between any two of those values. The culture supernatant may contain, for example, 2700 to 10000, 3000 to 10000, 4000 to 10000, or 3000 to 4000 pg/mL of IL-6. This concentration may be, for example, at least 2700, 3000, 3500, 4000, 4500, 5000, 6000, 7000, 8000, 9000, 10000, 11000, or 12000 pg/mL, and may be between any two of those values. The culture supernatant may contain at least 6000 pg/mL of MCP-1, at least 150 pg/mL of VEGF-C, at least 670 pg/mL of TGF-β1, at least 3.5 pg/mL of IL-7, or at least 6500 pg/mL of IL-8 in addition to G-CSF and IL-6 at the above concentrations. This culture supernatant may be used to produce the effect of highly concentrated G-CSF, IL-6, MCP-1, VEGF-C, TGF-β1, IL-7, or IL-8. The culture supernatant may contain, for example, at least 6000, 7000, 8000, 9000, or 10,000 pg/mL of MCP-1 or MCP-1 at a concentration between any two of those values. The culture supernatant may contain, for example, at least 150, 200, 250, 300, 350, or 400 pg/mL of VEGF-C or VEGF-C at a concentration between any two of those values. The culture supernatant may contain, for example, at least 670, 700, 750, 800, 850, 900, 950, or 1000 pg/mL of TGF-β1 or TGF-β1 at a concentration between any two of those values. The culture supernatant may contain, for example, at least 3.5, 4, 5, 6, 7, 8, 9, or 10 pg/mL of IL-7 and the range may be between any two of those values. The culture supernatant may contain, for example, at least 6500, 7000, 8000, 9000, 10000, 11000, 12000, or 15000 pg/mL of IL-8 or IL-8 at a concentration between any two of those values. From the viewpoint of enhancing the effects of these cytokines, the culture supernatant preferably contains the above seven cytokines in the above concentration ranges. Further, the culture supernatant above may contain, for example, at least 1350, 1370, 1390, 1400, 1420, 1440, 1450, 1470, 1490, or 1500 pg/mL of M-CSF or M-CSF at a concentration between any two of those values. Furthermore, the culture supernatant above may contain, for example, at least 95, 100, 110, 120, 130, or 140 pg/mL of Osteoprotegerin or Osteoprotegerin at a concentration between any two of those values.

In an embodiment of the present invention (including, for example, any of (1) to (41) above), the culture supernatant may contain at least 100 pg/mL of G-CSF, at least 550 pg/mL of IL-6, at least 6000 pg/mL of MCP-1, at least 150 pg/mL of VEGF-C, at least 670 pg/mL of TGF-β1 670 pg/mL, at least 3.5 pg/mL of IL-7, at least 6500 pg/mL of IL-8, or at least 120 pg/mL of CD9/CD63 fusion protein. The culture supernatant may contain, for example, at least 100, 200, 300, 400, 500, 600, 700, 800, 900, 1000, 1250, 1500, 1600, 1700, 1800, 1900, or 2000 pg/mL of G-CSF or G-CSF at a concentration between any two of those values. The culture supernatant may contain, for example, at least 550, 800, 1000, 2000, 2500, 2700, 2700, 3000, 3500, 4000, 4500, 5000, 6000, 7000, 8000, 9000, 10000, 11000, or 12000 pg/mL of IL-6 or IL-6 at a concentration between any two of those values. The culture supernatant may contain, for example, at least 6000, 7000, 8000, 9000, or 10,000 pg/mL of MCP-1 or MCP-1 at a concentration between any two of those values. The culture supernatant may contain, for example, at least 150, 200, 250, 300, 350, or 400 pg/mL of VEGF-C or VEGF-C at a concentration between any two of those values. The culture supernatant may contain, for example, at least 670, 700, 750, 800, 850, 900, 950, or 1000 pg/mL of TGF-β1 or TGF-β1 at a concentration between any two of those values. The culture supernatant may contain, for example, at least 3.5, 4, 5, 6, 7, 8, 9, or 10 pg/mL of IL-7 and the range may be between any two of those values. The culture supernatant may contain, for example, at least 6500, 7000, 8000, 9000, 10000, 11000, 12000, or 15000 pg/mL of IL-8 or IL-8 at a concentration between any two of those values. The culture supernatant may contain, for example, at least 120, 140, 160,180, 200, 250, or 300 pg/mL of CD9/CD63 fusion protein or CD9/CD63 fusion protein at a concentration between any two of those values. Further, the culture supernatant above may contain, for example, at least 1350, 1370, 1390, 1400, 1420, 1440, 1450, 1470, 1490, or 1500 pg/mL of M-CSF or M-CSF at a concentration between any two of those values. Furthermore, the culture supernatant above may contain, for example, at least 95, 100, 110, 120, 130, or 140 pg/mL of Osteoprotegerin or Osteoprotegerin at a concentration between any two of those values.

In an embodiment of the present invention (including, for example, any of (1) to (41) above), the culture supernatant may contain at least 1350 pg/mL of M-CSF or at least 95 pg/mL of Osteoprotegerin. This culture supernatant may be used to produce an effect of highly concentrated M-CSF or Osteoprotegerin. The culture supernatant may contain, for example, 1350 to 1500, 1350 to 1450, 1350 to 1420, or 1350 to 1400 pg/mL of M-CSF. This concentration may be, for example, at least 1350, 1370, 1390, 1400, 1420, 1440, 1450, 1470, 1490, or 1500 pg/mL, and may be between any two of those values. The culture supernatant may contain, for example, 95 to 140, 95 to 130, 95 to 120, or 95 to 110 pg/mL of Osteoprotegerin. This concentration may be, for example, at least 95, 100, 110, 120, 130, or 140 pg/mL, and may be between any two of those values. In addition to the above concentrations of M-CSF and Osteoprotegerin, the culture supernatant may contain at least 500 pg/mL of G-CSF, at least 2700 pg/mL of IL-6, at least 6000 pg/mL of MCP-1, at least 150 pg/mL of VEGF-C, at least 670 pg/mL of TGF-β1, at least 3.5 pg/mL of IL-7, or at least 6500 pg/mL of IL-8. This culture supernatant may be used to produce the effect of highly concentrated M-CSF, Osteoprotegerin, G-CSF, IL-6, MCP-1, VEGF-C, TGF-β1, IL-7, or IL-8. Also, in another embodiment, the culture supernatant may contain, for example, at least 100, 200, 300, 400, 500, 600, 700, 800, 900, 1000, 1250, 1500, 1600, 1700, 1800, 1900, or 2000 pg/mL of G-CSF or G-CSF at a concentration between any two of those values. The culture supernatant may contain, for example, at least 550, 800, 1000, 2000, 2500, 2700, 2700, 3000, 3500, 4000, 4500, 5000, 6000, 7000, 8000, 9000, 10000, 11000, or 12000 pg/mL of IL-6 or IL-6 at a concentration between any two of those values. The culture supernatant may contain, for example, at least 6000, 7000, 8000, 9000, or 10,000 pg/mL of MCP-1 or MCP-1 at a concentration between any two of those values. The culture supernatant may contain, for example, at least 150, 200, 250, 300, 350, or 400 pg/mL of VEGF-C or VEGF-C at a concentration between any two of those values. The culture supernatant may contain, for example, 670, 700, 750, 800, 850, 900, 950, or 1000 pg/mL of TGF-β1 or TGF-β1 at a concentration between any two of those values. The culture supernatant may contain, for example, at least 3.5, 4, 5, 6, 7, 8, 9, or 10 pg/mL of IL-7 and the range may be between any two of those values. The culture supernatant may contain, for example, at least 6500, 7000, 8000, 9000, 10000, 11000, 12000, or 15000 pg/mL of IL-8 or IL-8 at a concentration between any two of those values.

In an embodiment of the present invention (including, for example, any of (1) to (41) above), examples of the medium include a medium used for culturing cells. The medium may be a liquid or solid medium. Examples of the medium include a culture medium. Any common cell culture medium may be used as the medium, and its composition is not limited. The medium may contain, for example, amino acids, inorganic salts, vitamins, minerals, or carbon sources (e.g., glucose). For example, serum medium (e.g., FBS), basic medium (e.g., MEM), composite medium, serum-free medium, etc. may be used for the medium. A commercially available medium for the growth of human MSCs may be used as the medium. Examples of the basic medium for cell culture available from manufacturers include MEM (e.g., Thermo Fisher Scientific Inc.), DMEM (Dulbecco's Modified Eagle Medium) (e.g., Sigma-Aldrich), IMDM (e.g., Sigma -Aldrich), Ham's F-12 (e.g., FUJIFILM Wako Pure Chemical Corporation), DMEM/F12 (e.g., Sigma-Aldrich), or RPMI1640 (e.g., NACALAI TESQUE, INC.). The medium used may be DMEM/F12 medium with amino acids. Examples of the commercially available medium addition-use amino acid solution that can be used include MEM essential amino acid solution (FUJIFILM Wako Pure Chemical Corporation) or MEM non-essential amino acid solution (FUJIFILM Wako Pure Chemical Corporation). This DMEM/F12-based medium is free of xenogeneic components, cytokines, insulin, protein, and human serum. Considering that cultured cells and culture supernatants are used for regenerative medicine and therapeutic applications, the medium should be a serum-free, serum albumin-free, insulin-free, IGF-free, FGF-free, growth factor-free, cytokine-free, protein-free, or xenofree medium. This case has the advantage of eliminating the risk of foreign active ingredients, other than those derived from cultured cells, entering the patient's body. The medium may contain, for example, LIF, adhesion molecules, FGF, insulin, albumin, or transferrin components. From the viewpoint of promoting cell proliferation of MSCs in particular, the medium should contain a LIF component. From the viewpoint of promoting cell proliferation of MSCs in particular, the medium should further contain an adhesion molecule.

In an embodiment of the present invention (including, for example, any of (1) to (41) above), LIF includes a component that is also described as, for example, leukemia inhibitory factor. Details of the amino acid sequence, etc., of LIF can be obtained from, for instance, NCBI or UniProt website. The primary accession number of LIF listed in UniProt is, for example, P15018. The amino acid sequence of LIF may be, for example, the amino acid sequence set forth in SEQ ID NO: 4 or 5. The LIF includes, for example, human-derived LIF. In an embodiment of the present invention (including, for example, any of (1) to (41) above), the LIF component includes a molecule that has LIF activity and is derived from wild-type human LIF. The LIF component may be, for example, (a) a protein having the amino acid sequence set forth in SEQ ID NO: 4 or 5 or a bioactive fragment thereof, (b) a protein having an amino acid sequence having 90% or more homology to the amino acid sequence set forth in SEQ ID NO: 4 or 5 or a bioactive fragment thereof, or (c) a protein having an amino acid sequence having one to several amino acid deletions, substitutions, insertions or additions to the amino acid sequence set forth in SEQ ID NO: 4 or 5 or a bioactive fragment thereof. The LIF activity includes, for example, activity to stimulate LIF signaling, activity to bind to a LIF receptor, or activity to inhibit leukemic cell proliferation. Examples of the bioactive fragment include a polypeptide that has LIF activity but lacks a portion of LIF not involved in LIF activity. The LIF component may optionally have a tag (see, e.g., Mishra, Curr Protein Pept Sci. 2020; 21(8): 821-830) at the N or C terminus. The tag may be, for example, a tag for purification. The tag for purification may be, for example, a peptide tag (e.g. His tag). Examples of the tag for purification include a biochemically inert tag. The concentration of LIF component in the medium may be, for example, 0.01 ng/mL or higher. This concentration may be, for example, 0.01, 0.05, 0.1, 0.5, 0.6, 0.7, 0.8, 0.9, 1, 1.1, 1.2, 1.3, 1.4, 1.5, 2, 3, 4, 5, 8, or 10 ng/mL, or may be the above value or more or between any two of those values. This concentration may be, for example, 0.1 to 4, 0.1 to 3, 0.5 to 2, or 0.8 to 1.2 ng/ml. From the viewpoint of MSC proliferation promotion, 0.1 ng/ml or more is preferred, 0.5 ng/ml or more is more preferred, and 0.8 ng/ml or more is still more preferred.

In an embodiment of the present invention (including, for example, any of (1) to (41) above), FGF includes a component that is also described as, for example, fibroblast growth factor. Examples of the FGF include bFGF. bFGF contains a component also called basic fibroblast growth factor. Details of the amino acid sequence, etc., of bFGF can be obtained from, for instance, NCBI or UniProt website. The primary accession number of bFGF listed in UniProt is, for example, P09038. The amino acid sequence of bFGF may be, for example, the amino acid sequence set forth in SEQ ID NO: 6, 7 or 8. Examples of the bFGF include human-derived bFGF. In an embodiment of the present invention (including, for example, any of (1) to (41) above), the bFGF component includes a molecule that has bFGF activity and is derived from wild-type human bFGF. The bFGF component may be, for example, (a) a protein having the amino acid sequence set forth in SEQ ID NO: 6, 7, or 8 or a bioactive fragment thereof, (b) a protein having an amino acid sequence having 90% or more homology to the amino acid sequence set forth in SEQ ID NO: 6, 7, or 8 or a bioactive fragment thereof, or (c) a protein having an amino acid sequence having one to several amino acid deletions, substitutions, insertions or additions to the amino acid sequence set forth in SEQ ID NO: 6, 7, or 8 or a bioactive fragment thereof. The bFGF activity includes, for example, activity to stimulate bFGF signaling, activity to bind to a bFGF receptor, or activity to inhibit fibroblast proliferation. Examples of the bioactive fragment include a polypeptide that has bFGF activity but lacks a portion of bFGF not involved in bFGF activity. The FGF component may optionally have a tag (see, e.g., Mishra, Curr Protein Pept Sci. 2020; 21(8): 821-830) at the N or C terminus. The tag may be, for example, a tag for purification. The tag for purification may be, for example, a peptide tag (e.g. His tag). Examples of the tag for purification include a biochemically inert tag. The concentration of bFGF component in the medium may be, for example, 0.1 ng/mL or higher. This concentration may be, for example, 0.1, 0.5, 1, 5, 6, 7, 8, 9, 10, 11, 12, 13, 14, 15, 20, 30, 40, 50, 80, or 100 ng/mL, and may be the above value or more or between any two of those values. This concentration may be, for example, 1 to 40, 1 to 30, 5 to 20, or 8 to 12 ng/ml. From the viewpoint of MSC proliferation promotion, 1 ng/ml or more is preferred, 5 ng/ml or more is more preferred, and 8 ng/ml or more is still more preferred.

In an embodiment of the present invention (including, for example, any of (1) to (41) above), insulin includes a kind of hormone. Details of the amino acid sequence, etc., of insulin can be obtained from, for instance, NCBI or UniProt website. The primary accession number for insulin listed in UniProt is, for example, P01308. The amino acid sequence of human insulin contains, for example, the amino acid sequence set forth in SEQ ID NO: 9. Examples of insulin include human-derived insulin. In an embodiment of the present invention (including, for example, any of (1) to (41) above), the insulin component includes a molecule that has insulin activity and is derived from wild-type human insulin. The insulin component may be, for example, (a) a protein having the amino acid sequence set forth in SEQ ID NO: 9 or a bioactive fragment thereof, (b) a protein having an amino acid sequence having 90% or more homology to the amino acid sequence set forth in SEQ ID NO: 9 or a bioactive fragment thereof, or (c) a protein having an amino acid sequence having one to several amino acid deletions, substitutions, insertions or additions to the amino acid sequence set forth in SEQ ID NO: 9 or a bioactive fragment thereof. The insulin activity includes, for example, activity to stimulate insulin signaling or activity to activate insulin receptor tyrosine kinase. Examples of the bioactive fragment include a polypeptide that has insulin activity but lacks a portion of insulin not involved in insulin activity. The insulin component may optionally have a tag (see, e.g., Mishra, Curr Protein Pept Sci. 2020; 21(8): 821-830) at the N or C terminus. The tag may be, for example, a tag for purification. The tag for purification may be, for example, a peptide tag (e.g. His tag). Examples of the tag for purification include a biochemically inert tag. The concentration of insulin component in the medium may be, for example, 0.1 µg/mL or higher. This concentration may be, for example, 0.1, 0.5, 1, 5, 6, 7, 8, 9, 10, 11, 12, 13, 14, 15, 20, 30, 40, 50, 80, or 100 µg/mL, and may be the above value or more or between any two of those values. This concentration may be, for example, 1 to 40, 1 to 30, 5 to 20, or 8 to 12 µg/mL. From the viewpoint of MSC proliferation promotion, 1 µg/mL or more is preferred, 5 µg/mL or more is more preferred, and 8 µg/mL or more is still more preferred.

In an embodiment of the present invention (including, for example, any of (1) to (41) above), albumin includes a kind of protein abundant in serum. Details of the amino acid sequence, etc., of albumin can be obtained from, for instance, NCBI or UniProt website. The primary accession number for albumin listed in UniProt is, for example, Q56G89. The amino acid sequence of human albumin contains, for example, the amino acid sequence set forth in SEQ ID NO: 10. Examples of albumin include human-derived albumin. In an embodiment of the present invention (including, for example, any of (1) to (41) above), the albumin component includes a molecule that has albumin activity and is derived from wild-type human albumin. The albumin component may be, for example, (a) a protein having the amino acid sequence set forth in SEQ ID NO: 10 or a bioactive fragment thereof, (b) a protein having an amino acid sequence having 90% or more homology to the amino acid sequence set forth in SEQ ID NO: 10 or a bioactive fragment thereof, or (c) a protein having an amino acid sequence having one to several amino acid deletions, substitutions, insertions or additions to the amino acid sequence set forth in SEQ ID NO: 10 or a bioactive fragment thereof. Examples of the albumin activity include activity to regulate osmotic pressure of blood or activity to bind to fatty acid or bilirubin. Examples of the bioactive fragment include a polypeptide that has albumin activity but lacks a portion of albumin not involved in albumin activity. The albumin component may optionally have a tag (see, e.g., Mishra, Curr Protein Pept Sci. 2020; 21(8): 821-830) at the N or C terminus. The tag may be, for example, a tag for purification. The tag for purification may be, for example, a peptide tag (e.g. His tag). Examples of the tag for purification include a biochemically inert tag. The concentration of albumin component in the medium may be, for example, 10 µg/mL or higher. This concentration may be, for example, 10, 50, 100, 500, 600, 700, 800, 900, 1000, 1100, 1200, 1300, 1400, 1500, 2000, 3000, 4000, 5000, 8000, or 10,000 µg/mL, and may be the above value or more or between any two of those values. This concentration may be, for example, 100 to 4000, 10 to 3000, 500 to 2000, or 800 to 1200 ng/ml. From the viewpoint of MSC proliferation promotion, 100 µg/mL or more is preferred, 500 µg/mL or more is more preferred, and 800 µg/mL or more is still more preferred.

In an embodiment of the present invention (including, for example, any of (1) to (41) above), transferrin includes a kind of protein present in serum. Details of the amino acid sequence, etc., of transferrin can be obtained from, for instance, NCBI or UniProt website. The primary accession number of transferrin listed in UniProt is, for example, P02787. The amino acid sequence of human transferrin contains, for example, the amino acid sequence set forth in SEQ ID NO: 11. Examples of transferrin include human-derived transferrin. In an embodiment of the present invention (including, for example, any of (1) to (41) above), the transferrin component includes a molecule that has transferrin activity and is derived from wild-type human transferrin. The transferrin component may be, for example, (a) a protein having the amino acid sequence set forth in SEQ ID NO: 11 or a bioactive fragment thereof, (b) a protein having an amino acid sequence having 90% or more homology to the amino acid sequence set forth in SEQ ID NO: 11 or a bioactive fragment thereof, or (c) a protein having an amino acid sequence having one to several amino acid deletions, substitutions, insertions or additions to the amino acid sequence set forth in SEQ ID NO: 11 or a bioactive fragment thereof. Examples of the transferrin activity include activity to bind to transferrin receptor or activity to bind to an iron ion. Examples of the bioactive fragment include a polypeptide that has transferrin activity but lacks a portion of transferrin not involved in transferrin activity. The transferrin component may optionally have a tag (see, e.g., Mishra, Curr Protein Pept Sci. 2020; 21(8): 821-830) at the N or C terminus. The tag may be, for example, a tag for purification. The tag for purification may be, for example, a peptide tag (e.g. His tag). Examples of the tag for purification include a biochemically inert tag. The concentration of transferrin component in the medium may be, for example, 0.1 µg/mL or higher. This concentration may be, for example, 0.1, 0.5, 1, 5, 6, 7, 8, 9, 10, 11, 12, 13, 14, 15, 20, 30, 40, 50, 80, or 100 µg/mL, and may be the above value or more or between any two of those values. This concentration may be, for example, 1 to 40, 1 to 30, 5 to 20, or 8 to 12 µg/mL. From the viewpoint of MSC proliferation promotion, 1 µg/mL or more is preferred, 5 µg/mL or more is more preferred, and 8 µg/mL or more is still more preferred.

In an embodiment of the present invention (including, for example, any of (1) to (41) above), examples of the cultureware include a flat-bottom cultureware. The flat-bottom cultureware may be, for example, a plate type, a dish type, or a flask type. The bottom or top of the cultureware may be square or round in shape.

In an embodiment of the present invention (including, for example, any of (1) to (41) above), the container housing a culture supernatant may be medical container. Examples of this container include a syringe, a vial, a bottle, or a bag. The medical container may be a sterile (e.g., a solution-housing portion is sterile), infusion, or cryopreservation container. Examples of the medical bag include a soft bag or a bag with tubing attached.

In an embodiment of the present invention (including, for example, any of (1) to (41) above), the recovery step may include, for example, a step of separating a cell fraction from a culture supernatant, or a step of transferring a culture supernatant to a container. The separation step may include, for example, a step of centrifuging or filtering a medium containing MSCs and an imidazole dipeptide to separate a cell fraction from a culture supernatant. The recovery step may include a step of centrifuging or filtering a supernatant to remove dead cells or impurities from the supernatant, a step of sterilizing a supernatant, or a step of transferring a supernatant to a medical container. The recovery step may include a step of removing a medium component from a composition containing cells and the medium. The recovery step may be performed under aseptic conditions.

The method in an embodiment of the present invention (including, for example, any of (1) to (41) above) may include a step of sterilizing the recovered culture supernatant to produce a sterilized culture supernatant. The sterilization may include, for example, a step of passing the culture supernatant through a sterile filter.

In an embodiment of the present invention (including, for example, any of (1) to (41) above), the contact step may be performed such that MSCs are brought into contact by culturing them in a medium containing an imidazole dipeptide. The contact step may be performed *in vitro* or *in vivo.*

In an embodiment of the present invention (including, for example, any of (1) to (41) above), the disease suppression includes treatment of a disease. The suppression includes, for example, the ability to exert a suppressive, relapse-inhibiting, symptom-alleviating, or prophylactic effect on a disease of a patient or one or more symptoms associated with the disease. In addition, the inhibition also includes, for example, inhibition of patient ossification, inhibition of osteoblast generation, or inhibition of cellular osteogenic differentiation (e.g., inhibition of differentiation from MSCs into osteoblasts or chondrocytes).

In an embodiment of the present invention (including, for example, any of (1) to (41) above), the pharmaceutical composition may include a culture supernatant or cultured MSCs. If the pharmaceutical composition contains a culture supernatant, the pharmaceutical composition includes a composition consisting of a culture supernatant. The pharmaceutical composition may contain, for example, cytokines or exosomes. The concentrations of cytokines and exosome marker may be in the concentration ranges described in the culture supernatant embodiment above. The pharmaceutical composition may contain, for example, an imidazole dipeptide. The concentration of imidazole dipeptide may be in the concentration range described in the above medium embodiment. Examples of the pharmaceutical composition include a composition used for the above disease suppression or amelioration or a composition used for the prevention of the onset of the above diseases. The pharmaceutical composition may be produce by any procedure known in the art of formulation, which procedure includes mixing, for instance, an active ingredient and at least one pharmaceutically acceptable carrier. In addition, a dosage form of the pharmaceutical composition is not limited as long as it can be used for treatment, and may be an active ingredient alone or may be a mixture of an active ingredient and any component(s). Also, the form of the above carrier is not particularly limited, and may be, for instance, a solid or liquid (e.g., a buffer). The content of the above carrier may be, for instance, a pharmaceutically effective amount. The effective amount may be, for instance, an amount sufficient to pharmaceutically stabilize or deliver the active ingredient. For instance, the buffer is effective in stabilization of the active ingredient in a container. The pharmaceutical composition may also contain a stabilizer, a buffer, or a pH adjuster. The dose, dosing interval, administration method, and/or administration route are not particularly limited, and may be selected, if appropriate, in view of the age, body weight, symptom, and/or affected organ of a patient. It is also preferable to contain a therapeutically effective amount or effective amount of the active ingredient so as to elicit a desired action. In one embodiment of the present invention, the therapeutically effective amount includes the amount necessary for clinically observed amelioration or suppression of a symptom in a patient (e.g., an amount sufficient to inhibit osteogenic differentiation (e.g., to inhibit ectopic ossification) in a subject). The term "pharmaceutically acceptable" in one embodiment of the present invention includes a condition that fits for use within appropriate medical discretion and that is commensurate with a reasonable benefit/risk ratio. An additional ingredient(s) other than the culture supernatant or MSCs in the pharmaceutical composition is not particularly limited as long as the effects of the present invention are not impaired, and may be selected, if appropriate, depending on the purpose.

In an embodiment of the present invention (including, for example, any of (1) to (41) above), examples of the subject (including a patient) include a human or a non-human mammal (e.g., at least one species of a mouse, a guinea pig, a hamster, a rat, a mouse, a rabbit, a pig, sheep, a goat, a cow, a horse, a cat, a dog, a marmoset, a monkey, or a chimpanzee). Meanwhile, the patient may also be a patient in need of inhibition of osteogenic differentiation, a patient diagnosed as having ossification, or a patient in need of ossification treatment, or a patient in need of increased expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker.

In an embodiment of the present invention (including, for example, any of (1) to (41) above), the route of administration of the culture supernatant, MSCs, or pharmaceutical composition to a subject should be effective in the treatment. For example, the route may be intravenous, intra-arterial, subcutaneous, intra-lymph node, intramuscular, intraperitoneal, or oral. The dosage form should be effective for the treatment, and examples include an injection (e.g., an intravenous injection), a liquid, or a solid preparation.

In an embodiment of the present invention (including, for example, any of (1) to (41) above), the dose of the culture supernatant, MSCs, or pharmaceutical composition to a subject, the dosing interval, and the administration method may be selected, if appropriate, depending on the age and weight, symptoms, target organs, etc., of the patient. The dose may be, for example, 0.01 to 1000 mL in the case of supernatant and the single dose may be 1 × 10³ to 1 × 10¹¹ cells in the case of cells. The dosing interval may be, for example, 1 or 2 doses every 1 to 28 days or 1 to 4 weeks.

In an embodiment of the present invention (including, for example, any of (1) to (41) above), inhibition of osteoblast generation includes, for example, inhibition caused by inhibition of MSC differentiation into osteoblasts, or inhibition caused by inhibition of osteoblast proliferation. For example, MSCs and an imidazole dipeptide may be brought into contact to cause inhibition of MSC differentiation into osteoblasts.

In an embodiment of the present invention (including, for example, any of (1) to (41) above), the method of inhibiting generation of osteoblasts may include, for example, a step of culturing MSCs in a medium containing an imidazole dipeptide, a step of recovering a material secreted from MSCs, and a step of bringing the MSC-secreted material into contact with other MSCs. In this case, MSCs in culture and MSCs in contact are different MSCs.

In an embodiment of the present invention (including, for example, any of (1) to (41) above), examples of the MSC-secreted material include G-CSF, MCP-1, VEGF-C, TGF-β1, IL-6, IL-7, IL-8, M-CSF, Osteoprotegerin, Gremlin 1, SCF, FGF11, R-spondin 2, ADAM8, IL-34 , IGFBP2, DKK1, semaphorin 3B, or exosomes. These may be of human origin.

In an embodiment of the present invention (including, for example, any of (1) to (41) above), the percentage of cells of (26) to (28) or (38) above contained in the composition or cell population may be, for example, 30, 40, 50, 60, 70, 80, 90, 95, 98, 99, or 100%, and may be the above value or more or between any two of those values. This percentage may be, for example, 30 to 100%, 60 to 100%, 80 to 100%, 60 to 90%, 80 to 90%, or 90 to 100%.

In one embodiment of the present invention, the above homology (%) may be calculated, for example, as the percentage of homologous amino acids among multiple amino acid sequences, according to methods known in the art. Prior to the percentage calculation, the groups of sequences to be compared are aligned and gaps are introduced in some of the sequences, if necessary, to maximize the percentage of homologous/identical amino acids. The alignment procedure, the percentage calculation method, and related computer programs have been well-known in the art. Homology is calculated using, for example, global or local alignment. The former may be obtained by the Needleman-Wunsch algorithm (Needleman et al., J Mol Biol. 1970 Mar; 48(3): 443-53) or may be calculated using EMBOSS Needle (Rice et al., EMBOSS User's Guide: Practical Bioinformatics, 25 July 2011). The default settings for EMBOSS Needle may include, for example, MATRIX: BLOSUM62, Gap Open Penalty: 10, Gap Extend Penalty: 0.5, Output formats: pair, End Gap Penalty: false, End Gap Open Penalty: 10, and End Gap Extend Penalty: 0.5. The latter may be obtained by the BLAST algorithm (Altschul et al., J Mol Biol. 1990 Oct 5; 215(3): 403-10.) or may be calculated using blastp (The BLAST Sequence Analysis Tool. The NCBI Handbook, 2nd edition. March 15, 2013). The default settings for blastp may include, for example, MATRIX: BLOSUM62, Gap Open Penalty: 11, Extension: 1, and Compositional adjustments: Conditional compositional score matrix adjustment. Homology may be expressed as (Number of homologous amino acids/Number of amino acids in the amino acid sequence of the source compared) × 100. Homology is preferably calculated using global alignment. Unless otherwise specified, the fact that homology of the test object is a specific value or more includes that at least one of the above algorithms is used to obtain a value equal to or greater than a specific value by calculation. Any of the above algorithms may be used under default settings.

In one embodiment of the present invention, the above 90% or more described as the homology value may be, for example, 90, 91, 92, 93, 94, 95, 96, 97, 98, 99, or 100%, and may be the above value or more or between any two of those values. From the viewpoint of maintaining functional equivalence more, this percentage is preferably 95% or higher, more preferably 97% or higher, still more preferably 98% or higher, and particularly preferably 99% or higher. In one embodiment of the invention, the above number or less may be, for example, 15, 10, 8, 6, 5, 4, 3, 2, 1, or 0, and may be less than or equal to any of those values, or between any two of those values. From the viewpoint of maintaining functional equivalence more, the number is preferably 15 or less, more preferably 10 or less, and particularly preferably 5 or less.

In an embodiment of the present invention, when an substitution occurs in the original sequence, a conservative amino acid substitution (see, for example, French et al., J Mol Evol (1983) 19, 171-175) is preferred. In an embodiment of the present invention, a conservative amino acid substitution involves replacing one amino acid residue with an amino acid residue having a side chain with similar characteristics. Amino acid residues are classified by their side chains into several families such as basic side chains (e.g., lysine, arginine, histidine), acidic side chains (e.g., aspartic acid, glutamic acid), uncharged polar side chains (e.g., asparagine, glutamine, serine, threonine, tyrosine, cysteine), nonpolar side chains (e.g., glycine, alanine, valine, leucine, isoleucine, proline, phenylalanine, methionine, tryptophan), β-branched side chains (e.g., threonine, valine, isoleucine), and aromatic side chains (e.g., tyrosine, phenylalanine, tryptophan). The conservative amino acid substitution is preferably substitution between amino acid residues within the same family.

In one embodiment of the present invention, the above "amino acid" is a general term for any organic compound having an amino group and a carboxyl group. When a protein according to an embodiment of the present invention contains a "specific amino acid sequence", any of amino acids in the amino acid sequence may be chemically modified. In addition, any of amino acids in the amino acid sequence may form a salt or a solvate. In addition, any of amino acids in the amino acid sequence may be in an L-form or D-form. In such cases, a protein according to an embodiment of the present invention can be said to contain the above "specific amino acid sequence". Examples of the *in vivo* chemical modification of amino acids included in a protein include N-terminal modification (e.g., acetylation, myristoylation), C-terminal modification (e.g., amidation, glycosylphosphatidylinositol addition), or side-chain modification (e.g., phosphorylation, glycosylation).

As used herein, the term "significant(ly) " may mean that a statistically significant difference may be evaluated using a Student's t-test (one- or two-tailed) as significant when p < 0.05 or p < 0.01. Alternatively, when a substantial difference occurs, the difference may be evaluated as significant.

All the literatures cited herein are incorporated by reference in their entirety. As used herein, the term "or" is used when "at least one" matter listed in the text is acceptable. The same applies to "or". When the wording "between any two of those values" is indicated herein, this range encompasses the two values inclusive. The wording "from A to B" herein means A or more and B or less. As used herein, the wording "any of (1) to (41) above" means (1), (2), (3), (4), (5), (6), (7), (8), (9), (10), (11), (12), (13), (14), (15), (16), (17), (18), (19), (20), (21), (22), (23), ( (24), (25), (26), (27), (28), (29), (30), (31), (32), (33), (34), (35), (36), (37), (38), (39), (40), or (41), including reference to one or more of the above.

Examples of an embodiment of the present invention may include the following embodiments.
1. A method for producing a culture supernatant, comprising the step of recovering a culture supernatant from a medium containing mesenchymal stem cells and an imidazole dipeptide.
2. The production method according to item 1, wherein the mesenchymal stem cells are mesenchymal stem cells cultured in a medium containing an imidazole dipeptide.
3. The production method according to item 1 or 2, comprising a step of culturing the mesenchymal stem cells in a medium containing an imidazole dipeptide to generate cultured mesenchymal stem cells.
4. The production method according to any one of items 1 to 3, wherein the recovering step comprises a step of centrifuging or filtering the medium containing mesenchymal stem cells and an imidazole dipeptide.
5. The production method according to any one of items 1 to 4, comprising a step of sterilizing the recovered culture supernatant to prepare a sterilized culture supernatant.
6. The production method according to any one of items 1 to 5, wherein the culture supernatant is a culture supernatant obtained by adhesion culture.
7. The production method according to any one of items 1 to 6, wherein the culture supernatant contains at least 500 pg/mL of G-CSF or at least 2700 pg/mL of IL-6.
8. The production method according to any one of items 1 to 7, wherein the culture supernatant contains G-CSF, MCP-1, VEGF-C, TGF-β1, IL-6, IL-7, IL-8, and exosomes.
9. The production method according to any one of items 1 to 8, wherein the imidazole dipeptide is a compound having a structure of the above-mentioned formula (1) or (2).
10. The production method according to any one of items 1 to 9, wherein the imidazole dipeptide is carnosine, anserine, balenine, or homocarnosine.
11. The production method according to any one of items 1 to 10, wherein the imidazole dipeptide is carnosine.
12. The production method according to any one of items 1 to 10, wherein the imidazole dipeptide is anserine.
13. A culture supernatant obtained by the production method according to any one of items 1 to 12.
14. A pharmaceutical composition comprising a culture supernatant obtained by the production method according to any one of items 1 to 12.
15. The culture supernatant according to item 13 or the pharmaceutical composition according to item 14, for inhibiting osteogenic differentiation.
16. A syringe, vial, or medical bag comprising the culture supernatant according to item 13 or the pharmaceutical composition according to item 14 or 15.
17. A method of culturing cells, comprising the step of culturing mesenchymal stem cells in a serum-free medium containing an imidazole dipeptide to generate cultured cells.
18. The culture method according to item 17, comprising a step of recovering the cultured cells from the medium.
19. The culture method according to item 17 or 18, wherein the culturing comprises a step of culturing in the presence of an adhesion factor.
20. The culture method according to any one of items 17 to 19, wherein the medium is an insulin-free, IGF-free, or FGF-free medium.
21. The culture method according to any one of items 17 to 19, wherein the medium is a growth factor-free medium.
22. The culture method according to any one of items 17 to 19, wherein the medium is a protein-free medium.
23. A method for producing cells, comprising the step of performing the culture method according to any one of items 17 to 22.
24. A cell obtained by the production method according to item 23.
25. A composition comprising a cell population of the cells according to item 24.
26. A pharmaceutical composition comprising cells obtained by the production method according to item 23.
27. The pharmaceutical composition according to item 26, for inhibiting osteogenic differentiation.
28. A medium for culturing mesenchymal stem cells, wherein the medium comprises an imidazole dipeptide and is serum-free.
29. The medium according to item 28, wherein the medium is insulin-free, IGF-free, or FGF-free.
30. The medium according to item 28 or 29, wherein the medium is growth factor-free.
31. The medium according to any one of items 28 to 30, wherein the medium is protein-free.
32. The medium according to any one of items 28 to 31, comprising mesenchymal stem cells.
33. A method of inhibiting generation of osteoblasts, comprising the step of culturing mesenchymal stem cells in a medium containing an imidazole dipeptide.
34. A method of inhibiting generation of osteoblasts, comprising the step of bringing mesenchymal stem cells into contact with a culture supernatant obtained by culturing mesenchymal stem cells in a medium containing an imidazole dipeptide.
35. A composition for inhibiting generation of osteoblasts, comprising an imidazole dipeptide.
36. A composition for inhibiting generation of osteoblasts, comprising a culture supernatant obtained by culturing mesenchymal stem cells in a medium containing an imidazole dipeptide.
37. A method of promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker, the method comprising the step of culturing mesenchymal stem cells in a medium containing an imidazole dipeptide.
38. A composition for promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker of mesenchymal stem cells, the composition comprising an imidazole dipeptide.
39. A method of promoting exosome secretion, comprising the step of culturing mesenchymal stem cells in a medium containing an imidazole dipeptide.
40. A composition for promoting secretion of exosomes from mesenchymal stem cells, comprising an imidazole dipeptide.
41. A culture supernatant of mesenchymal stem cells, comprising at least 500 pg/ml of G-CSF.
42. A culture supernatant of mesenchymal stem cells, comprising at least 2700 pg/ml of IL-6.
43. An umbilical cord-derived mesenchymal stem cell that expresses or secretes a high level of G-CSF or IL-6.
44. A composition comprising a cell population of the cells according to item 43.
45. An IL-34-positive mesenchymal stem cell.
46. The mesenchymal stem cell according to item 45, wherein the mesenchymal stem cell is purified.
47. An ADAM8-positive mesenchymal stem cell.
48. The mesenchymal stem cell according to item 47, wherein the mesenchymal stem cell is purified.
49. The mesenchymal stem cell according to any one of items 45 to 48, .wherein the mesenchymal stem cell is positive for Osteoprotegerin, Gremlin 1, SCF, FGF11, R-spondin 2, ADAM8, IL-34, IGFBP2, DKK1, and semaphorin 3B.
50. The mesenchymal stem cell according to item 49, wherein the mesenchymal stem cell is purified.
51. A pharmaceutical composition comprising the mesenchymal stem cells according to any one of items 45 to 50.
52. The pharmaceutical composition according to item 51, for inhibiting osteogenic differentiation.
53. A pharmaceutical composition for inhibiting osteogenic differentiation, comprising cells obtained by culturing mesenchymal stem cells in a medium containing an imidazole dipeptide.
54. The pharmaceutical composition according to item 53, further comprising a pharmaceutically acceptable carrier.
55. The pharmaceutical composition according to item 53 or 54, wherein the cells are positive for Osteoprotegerin, Gremlin 1, SCF, FGF11, R-spondin 2, ADAM8, IL-34, IGFBP2, DKK1, and semaphorin 3B.
56. The pharmaceutical composition according to any one of items 53 to 55, wherein the cells are cells obtained by culturing in a medium containing an imidazole dipeptide and LIF.
57. The pharmaceutical composition according to any one of items 53 to 56, wherein the culturing is adhesion culture.
58. A method for producing a pharmaceutical composition for inhibiting osteogenic differentiation, the method comprising the step of culturing mesenchymal stem cells in a medium containing an imidazole dipeptide to generate cultured cells.
59. The production method according to item 58, comprising a step of recovering the cultured cells from the medium.
60. The production method according to item 58 or 59, comprising a step of mixing the cultured cells with a pharmaceutically acceptable carrier.
61. A medium comprising an imidazole dipeptide and a leukemia inhibitory factor (LIF) component.
62. The medium according to item 61, further comprising an adhesion factor.
63. The medium according to item 61 or 62, for culturing mesenchymal stem cells.
64. The medium according to any one of items 61 to 63, comprising mesenchymal stem cells.
65. A method for producing cells, comprising the step of culturing mesenchymal stem cells in the medium according to any one of items 61 to 64 to generate cultured cells.
66. The production method according to item 65, wherein the culturing is adhesion culture.
67. A cell obtained by the production method according to item 65 or 66.
68. A composition comprising a cell population of the cells according to item 67.
69. A pharmaceutical composition comprising cells obtained by the production method according to item 65 or 66.
70. The pharmaceutical composition according to item 69, for inhibiting osteogenic differentiation.
71. A kit comprising an imidazole dipeptide and a LIF component.
72. The kit according to item 71, for use in preparation of a medium for culturing mesenchymal stem cells.

Hereinabove, embodiments of the invention have been described. These are examples of forms that can be included in the present invention. The present invention is not limited to these, and various configurations other than the above can be adopted. **In** addition, in the present invention, the configurations or features described in the above embodiments may be combined and adopted independently.

### Examples

Hereinbelow, the present invention will be described in more detail with reference to Examples. However, the present invention is not limited to them.

### Example 1: To analyze culture supernatant

### 1.1 Experimental procedure: To culture MSCs using medium containing imidazole dipeptide and to recover culture supernatant

Carnosine was used as the imidazole dipeptide in this experiment. The experimental procedure is as follows. Human umbilical cord-derived mesenchymal stem cells (0.3×10e5 cells) were suspended in 20 mL of MSC Expansion XSFM B (FUJIFILM Wako Pure Chemical Corporation) (hereinafter also referred to as MSC medium B). To 20 ml of cell suspension, 10 µl of iMatrix-511 laminin fragment (0.5 µg/µl) (Nippi, Inc.) was added, and the resulting cell suspension was seeded onto a T150 flask (Sumitomo Bakelite Co., Ltd.).

Seven days after seeding, 10 ml of MSC medium B with 5 µl of iMatrix-511 laminin fragment (0.5 µg/µl) (Nippi Inc.) was added. Fourteen days after seeding (day 14), the medium was changed twice with PBS to wash out the remaining medium. Then, 30 ml of protein-free medium based on DMEM/F12 medium with additional amino acids (hereinafter, also referred to as MSC medium A) was added (hereinafter, 30 ml was used when replacing with MSC medium A). The amino acids added here are MEM essential amino acid solution (FUJIFILM Wako Pure Chemical Corporation) and MEM non-essential amino acid solution (FUJIFILM Wako Pure Chemical Corporation). At this time, L-carnosine (FUJIFILM Wako Pure Chemical Corporation), an imidazole dipeptide, was added to MSC medium A to a final concentration of 0 mM (no addition), 1 mM, 10 mM, or 30 mM. MSC medium A supplemented with each carnosine was added to the cells, and the mixture was cultured for 3 days (production culture). This culture is denoted as the "first production culture".

After the first production culture, the culture supernatant of MSC medium A was recovered. The levels of cytokines contained in the culture supernatant were analyzed using an ELISA analysis kit (R&D Systems) or Milliplex kit (Merck Millipore). The cytokines analyzed included HGF (Hepatocyte growth factor), G-CSF (Granulocyte colony stimulating factor), MCP-1 (Monocyte chemotactic protein 1), VEGF-C (Vascular endothelial growth factor-C), TGF-β1 (Transforming growth factor-β1), IL-6 (Interleukin-6), IL-7 (Interleukin-7), and IL-8 (Interleukin-8). In addition, the amount of exosomes contained in the culture supernatant was analyzed using the CD9/CD63 ELISA kit (COSMO BIO) for the exosome marker protein (CD9/CD63 fusion protein) as an indicator. In this Example, the levels of cytokines and the level of exosome marker protein refer to the values measured by ELISA using each specific antibody.

After the first production culture, the cells were returned to MSC medium B and cultured for 2 days (recovery culture). After 2 days of recovery culture, the medium was again replaced by MSC medium A supplemented with carnosine as described above and cultured for 3 days (carnosine concentration was the same as that of the first production culture). This culture is denoted as the "second production culture".

After the second production culture, the culture supernatant of MSC medium A supplemented with carnosine was recovered. Cytokines contained in the culture supernatant were analyzed using an ELISA analysis kit (R&D Systems).

After the second production culture, the cells were returned to MSC medium B and cultured for 2 days (recovery culture). After 2 days of recovery culture, the medium was again replaced by MSC medium A supplemented with carnosine as described above and cultured for 3 days (carnosine concentration was the same as that of the first production culture). This culture is denoted as the "third production culture".

After the third production culture, the culture supernatant of MSC medium A supplemented with carnosine was recovered. Cytokines contained in the culture supernatant were analyzed using an ELISA analysis kit (R&D Systems).

After the third production culture, the cells were returned to MSC medium B and cultured for 2 days (recovery culture). After 2 days of recovery culture, the medium was again replaced by MSC medium A supplemented with carnosine as described above and cultured for 3 days (carnosine concentration was the same as that of the first production culture). This culture is denoted as the "fourth production culture".

After the fourth production culture, the culture supernatant of MSC medium A supplemented with carnosine was recovered. Cytokines contained in the culture supernatant were analyzed using an ELISA analysis kit (R&D Systems).

As described above, a total of four production cultures were performed, and a total of four culture supernatant recoveries and cytokine analyses were performed for MSC medium A supplemented with carnosine at each concentration. In addition, the morphology of the cells was also observed after the fourth culture supernatant recovery. After the fourth culture supernatant recovery, the cells were further enzyme-treated with TrypLE TM Select (Thermo Fisher Scientific) for 20 minutes to disperse the cells, and the cells were then counted. The results of these experiments are described below.

### 1.2 To observe cell morphology

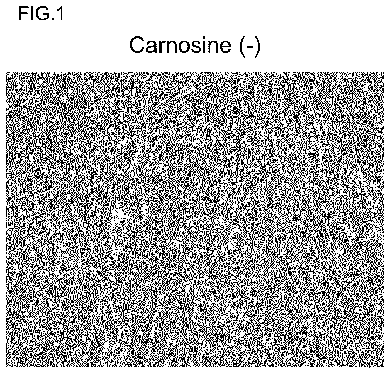

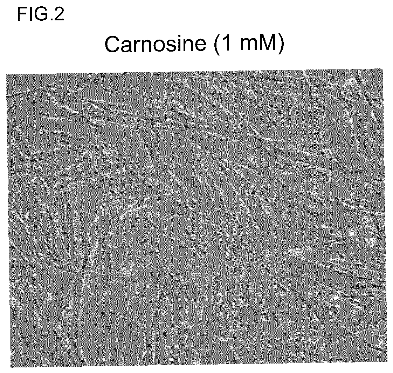

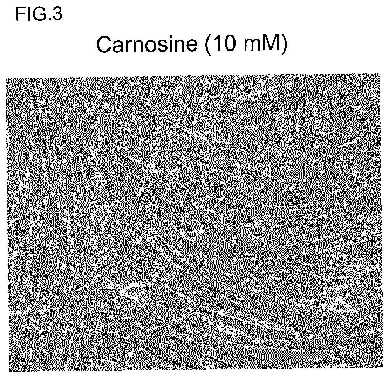

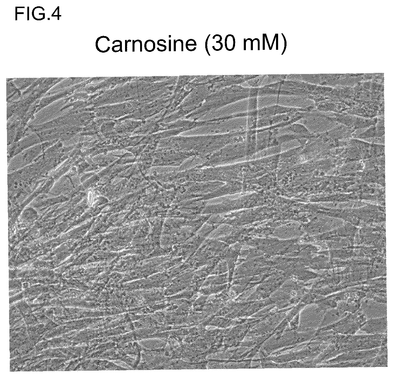

FIGS. 1 to 4 are micrographs of MSCs after culturing in a carnosine-free medium or each carnosine-containing medium. Cell death was not observed in large numbers, and there were no obvious differences in the cell morphology at each carnosine concentration.

### 1.3 To count cells

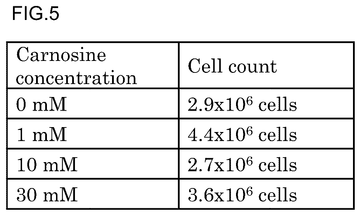

FIG. 5 shows the results of counting MSCs after culturing in a carnosine-free medium or each carnosine-containing medium. The addition of carnosine to the medium did not significantly change the number of cells.

### 1.4 Exosome marker level

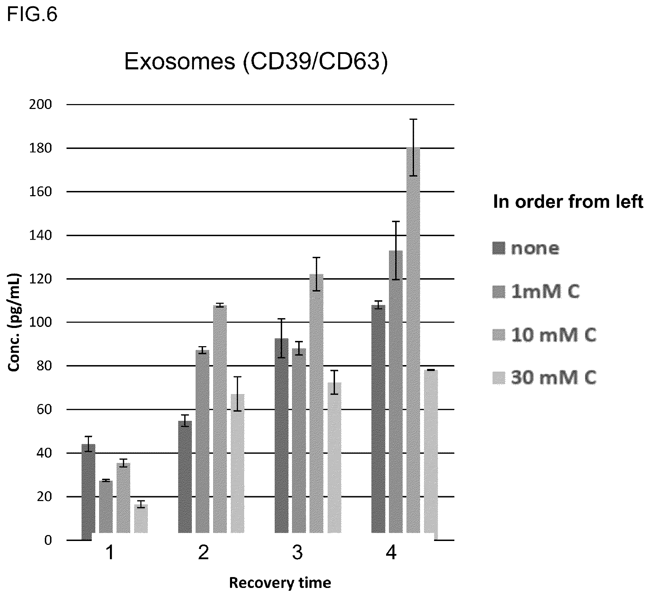

FIG. 6 shows the results of the level of exosome marker in each culture supernatant. The addition of carnosine to the medium showed a trend of increase in the exosome marker.

### 1.5 Cytokine levels

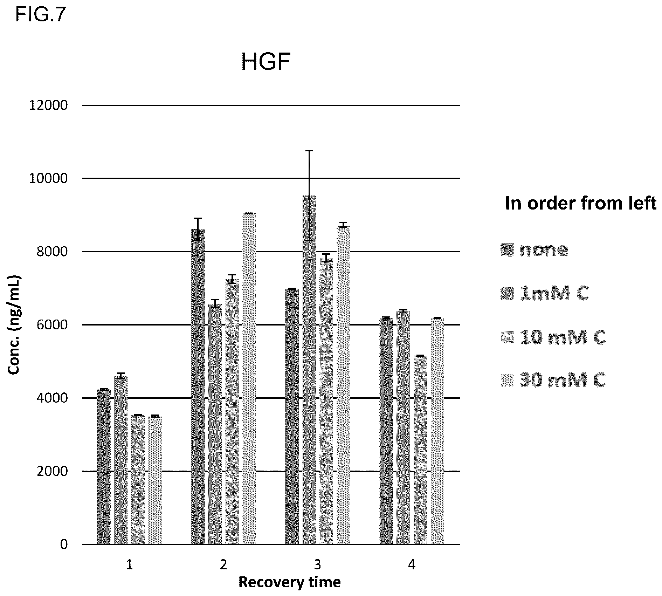

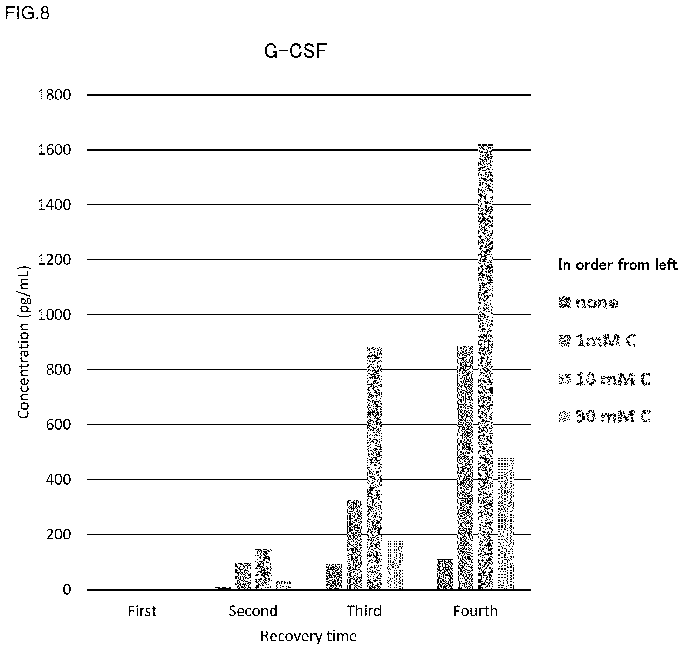

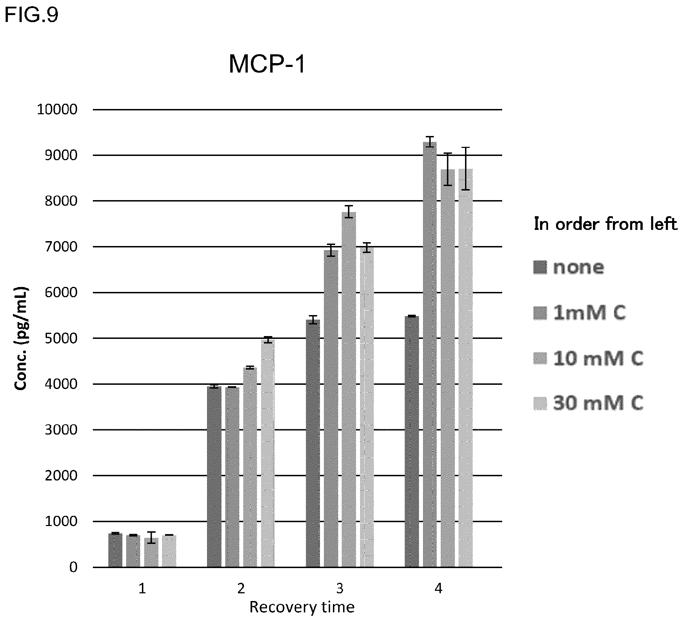

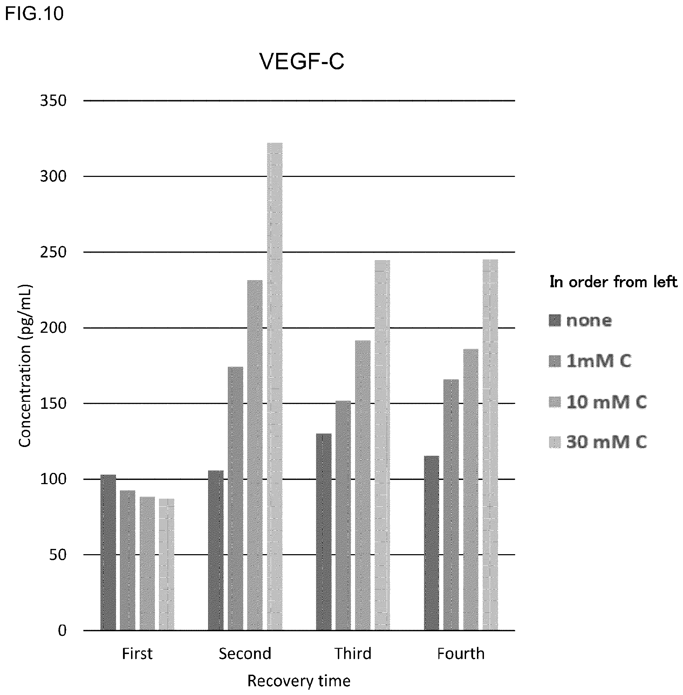

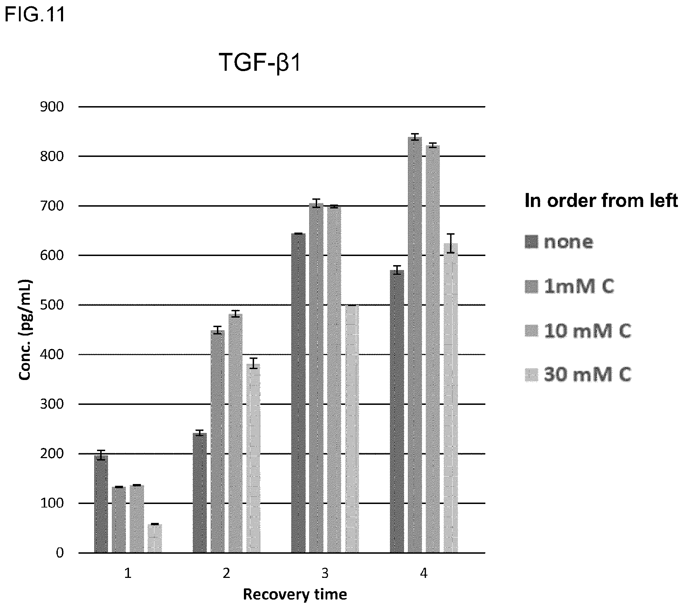

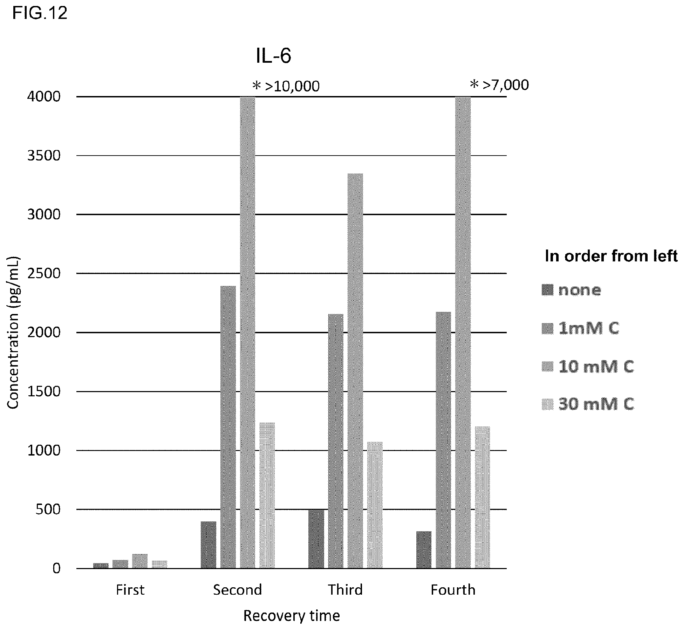

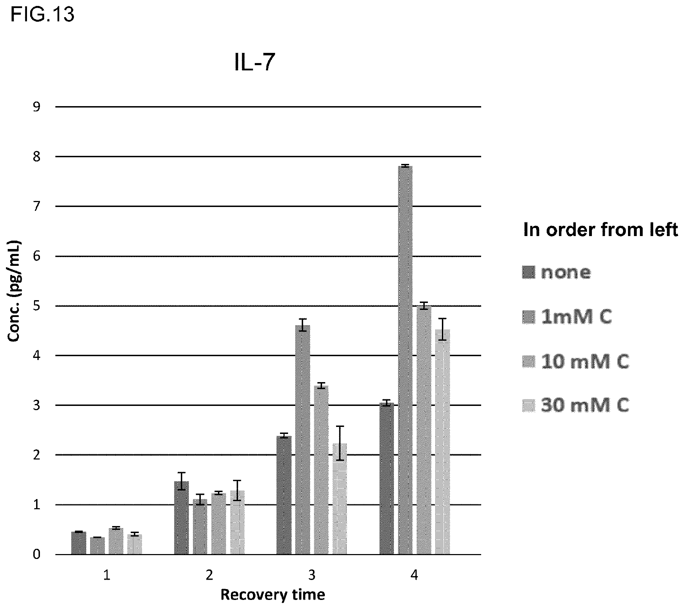

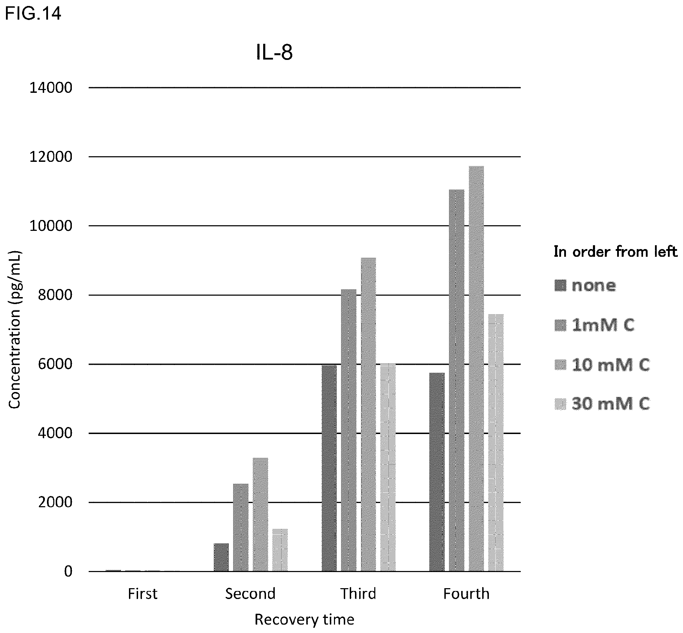

FIGS. 7 to 14 show the results of analyzing the levels of HGF (HGF, 3082), G-CSF (CSF3, 1440), MCP-1 (CCL2, 6347), VEGF-C (VEGFC, 7424), TGF-β1 (TGFB1, 7040), IL-6(IL6, 3569), IL-7(IL7, 3574), and IL-8(CXCL8, 3576) in the culture supernatants. The addition of carnosine to the medium did not significantly change the level of HGF. By contrast, G-CSF, MCP-1, VEGF-C, TGF-β1, IL-6, IL-7, and IL-8 showed an increasing trend (each parenthesis means Official Symbol and Gene ID of NCBI). In particular, the increase rates of G-CSF and IL-6 were marked. When compared with the fourth culture supernatant recovery, G-CSF concentration increased 14.7-fold (approximately 1621 pg/mL) and IL-6 concentration increased 22-fold (over 7000 pg/mL) when 10 mM of carnosine was added.

In the above experimental procedure, umbilical cord-derived MSCs were also cultured in place of adipose-derived MSCs. The levels of G-CSF and MCP-1 in the culture supernatant were then measured. The results showed that the addition of carnosine to the medium increased the levels of both.

### Example 2: Effect of culture supernatant on inhibition of osteogenic differentiation

### 2.1 Experimental procedure

### 2.1.1 To culture MSCs using medium containing imidazole dipeptide and to recover culture supernatant

Human umbilical cord-derived mesenchymal stem cells (CET03 strain) established by the present inventors were used to recover the culture supernatant according to the experimental procedure up to the third production culture in Example 1 described above. In this case, the MSC medium A used in Example 1 was replaced by DMEM (Sigma), DMEM/F12 (Sigma), or IMDM (Sigma). In addition, the concentration of carnosine added was changed to 0 or 10 mM. This resulted in the first, second, and third production culture supernatants. Further, equal volumes of the first to the third production culture supernatants were mixed to obtain the umbilical cord MSC supernatant. The 6 obtained umbilical cord MSC supernatants are denoted as DMEM_ supernatant, DMEM_C_ supernatant, DMEM/F12_ supernatant, DMEM/F12_C_supernatant, IMDM_ supernatant, and IMDM_C_supernatant (the names of these supernatants are indicated such that the name of the medium used is on the left side; the supernatant obtained under the carnosine-added conditions is marked with "C" on the right of the medium name.).

### 2.1.2 To induce MSC osteogenic differentiation and to analyze calcium deposition

Protocol 1. Human bone marrow MSCs (primary cultured human bone marrow MSCs) were cultured in αMEM medium containing 10% FBS (Hyclone), treated with trypsin EDTA solution, and made into single cells. The resulting single cells were seeded onto a fibronectin-coated 24-well plate at 8 × 10e4 cells/well in basic medium (DMEM (Sigma) supplemented with 0.6 mM CaCl₂) containing 10% FBS. The day after seeding, the medium was changed to basic medium (DMEM (Sigma) supplemented with 0.6 mM CaCl₂) (hereinafter also referred to as osteogenic differentiation-inducing medium) supplemented with osteogenic differentiation-inducing components (10% FBS, 0.1 µM dexamethasone, 50 µM ascorbic acid, and 10 mM β-glycerophosphate), and a final concentration of 0 or 10 mM carnosine. Using the same medium used for this medium change, the cells were cultured for 10 to 14 days while the medium was changed every 2 to 3 days, until calcium deposition was observed in the cells. The intracellular calcium deposition was quantified by calcium assay on the cultured cells. The cells obtained are denoted as DMEM_medium_cells, and DMEM_C_medium_cells (the names of these cells are indicated such that the name of the medium used is on the left side; the cells obtained under carnosine-added conditions are marked with C on the right of the medium name).

Protocol 2. The basic medium in protocol 1 was changed to DMEM/F12 (Sigma) with 0.6 mM CaCl₂ and the protocol was conducted under these conditions. The cells obtained are denoted as DMEM/F12_medium_cells, and DMEM/F12_C_medium_cells (the names of these cells are indicated such that the name of the medium used is on the left side; the cells obtained under carnosine-added conditions are marked with C on the right of the medium name).

Protocol 3. The basic medium in protocol 1 was changed to IMDM (Sigma) with 2 mM L-glutamine and the protocol was conducted under these conditions. The cells obtained are denoted as IMDM_medium_cells, and IMDM_C_medium_cells (the names of these cells are indicated such that the name of the medium used is on the left side; the cells obtained under carnosine-added conditions are marked with C on the right of the medium name). In addition, the same procedure as in protocol 3 was separately performed, and calcium deposition images of the cells after osteogenic differentiation-inducing culture were obtained using an Alizarin Red S staining kit (CosmoBio) and then photographed.

Protocol 4. The osteogenic differentiation-inducing medium of protocol 1 was changed to the umbilical cord MSC culture supernatant obtained in 2.1.1 above (DMEM_supernatant, or DMEM_C_supernatant) supplemented with the components for inducing osteogenic differentiation (10% FBS, 0.1µM dexamethasone, 50µM ascorbic acid, and 10mM β-glycerophosphate), and the protocol was conducted under these conditions. The cells obtained are denoted as DMEM_supernatant_cells, and DMEM_C_supernatant_cells (the names of these cells are indicated such that the name of the medium used is on the left side; the cells obtained under carnosine-added conditions are marked with C on the right of the medium name).

Protocol 5. The osteogenic differentiation-inducing medium of protocol 2 was changed to the umbilical cord MSC culture supernatant obtained in 2.1.1 above (DMEM/F12_supernatant, or DMEM/F12_C_ supernatant) supplemented with the components for inducing osteogenic differentiation (10% FBS, 0.1µM dexamethasone, 50µM ascorbic acid, and 10mM β-glycerophosphate), and the protocol was conducted under these conditions. The cells obtained are denoted as DMEM/F12_ supernatant_cells, and DMEM/F12_C_supernatant_cells (the names of these cells are indicated such that the name of the medium used is on the left side; the cells obtained under carnosine-added conditions are marked with C on the right of the medium name).

Protocol 6. The osteogenic differentiation-inducing medium of protocol 3 was changed to the umbilical cord MSC culture supernatant obtained in 2.1.1 above (IMDM_ supernatant, or IMDM_C_supernatant) supplemented with the components for inducing osteogenic differentiation (10% FBS, 0.1µM dexamethasone, 50µM ascorbic acid, and 10mM β-glycerophosphate), and the protocol was conducted under these conditions. The cells obtained are denoted as IMDM_supernatant_cells, and IMDM_C_supernatant_cells (the names of these cells are indicated such that the name of the medium used is on the left side; the cells obtained under carnosine-added conditions are marked with C on the right of the medium name). In addition, the same procedure as in protocol 6 was separately performed, and calcium deposition images of the cells after osteogenic differentiation-inducing culture were obtained using an Alizarin Red S staining kit (CosmoBio) and then photographed.

### 2.2 Results

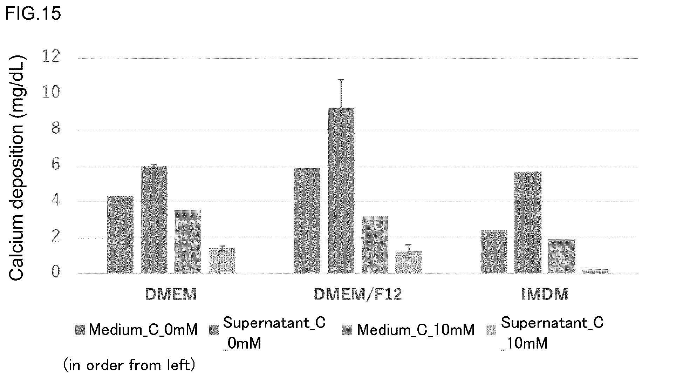

FIG. 15 shows the results of quantifying calcium deposition in bone marrow MSCs. The name of the medium in the figure is the name of the basic medium used. Osteogenic differentiation of bone marrow MSCs was quantified by calcium content. As a result, when compared with cells obtained using a carnosine-free medium (medium_C_0mM in the figure; corresponding samples are DMEM_medium_cells, DMEM/F12_medium_cells, and IMDM_medium_cells) and cells obtained using a supernatant of MSCs cultured in a carnosine-free medium (supernatant_C_0mM in the figure; corresponding samples are DMEM_supernatant_cells, DMEM/F12_supernatant_cells, and IMDM_supernatant_cells), cells obtained using a supernatant of MSCs cultured in a carnosine-containing medium (supernatant_C_10mM in the figure; corresponding samples are DMEM_C_ supernatant_cells, DMEM/F12_C_supernatant_cells, and IMDM_C_supernatant_cells) had markedly lower calcium content. In addition, when compared with cells obtained using a carnosine-containing medium (medium_C_10mM in the figure; corresponding samples are DMEM_C_medium_cells, DMEM/F12_C_medium_cells, and IMDM_C_medium_cells), the above cells obtained using the supernatant of MSCs had markedly lower calcium content. The supernatant of MSCs cultured in a carnosine-containing medium showed a marked inhibitory effect on osteogenic differentiation.

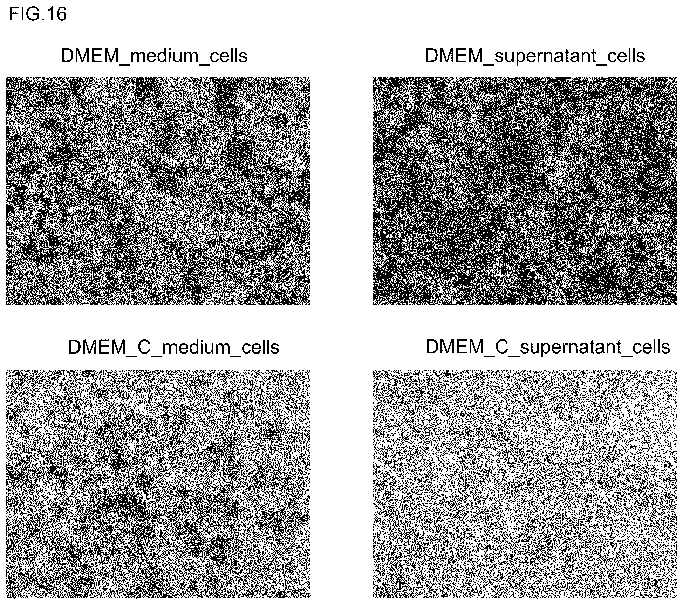

FIG. 16 is Alizarin Red S stained images of bone marrow MSCs. Bone marrow MSCs were stained with Alizarin Red S after induction of osteogenic differentiation. The results showed that cells obtained using a carnosine-free medium (DMEM_medium_cells), cells obtained using a supernatant of MSCs cultured in a carnosine-free medium (DMEM_supernatant_cells), and cells obtained using a carnosine-containing medium (DMEM_C_medium_cells) had staining with Alizarin Red. By contrast, cells obtained using a supernatant of MSCs cultured in a carnosine-containing medium (DMEM_C_supernatant_cells) showed no staining by Alizarin Red. The supernatant of MSCs cultured in a carnosine-containing medium showed a marked inhibitory effect on osteogenic differentiation.

### Example 3: Effect of MSCs on inhibition of osteogenic differentiation

### 3.1 Experimental procedure

### 3.1.1 To culture umbilical cord MSCs using medium containing imidazole dipeptide

Human umbilical cord MSCs established by the present inventors were cultured in DMEM/F12 medium (hereinafter, also referred to as MSC medium C) containing 10 ng/ml bFGF (PeproTech, AF-100-18C), 10 µg/ml insulin (NACALAI TESQUE, 12878-44), 1000 µg/ml albumin (Sigma, A9511), 10 ug/ml transferrin (NACALAI TESQUE, 12879-34), and 1 ng/ml LIF (PeproTech, AF-300-05), treated with trypsin EDTA solution, and then made into single cells. The same medium was admixed with carnosine at a final concentration of 0 or 5 mM, and iMatrix-511 laminin fragment (Nippi, Inc) was added at a final concentration of 0.25 µg/ml, and the cells were seeded onto a T25 flask (Corning). After 2 weeks of culture, the cells were washed with PBS to remove carnosine, treated with trypsin EDTA solution, made into single cells, and then used for co-culture with bone marrow MSCs as described below.

### 3.1.2 To co-culture bone marrow MSCs and umbilical cord MSCs

Bone marrow MSCs (primary cultured human bone marrow MSCs) were cultured in αMEM medium containing 10% FBS (Hyclone), treated with trypsin EDTA solution, and made into single cells. The bone marrow MSCs were mixed with the umbilical cord MSCs cultured in a medium containing 0 or 5 mM carnosine as described in 3.1.1 above at the ratio of 1:0, 0:1, 1:1, and 3:1, respectively. Further, the respective MSCs were seeded onto a 24-well plate in αMEM medium with 10% FBS so that the total cell count of respective MSCs combined was 8.0 × 10e4/well. On the day after seeding, the wells in each co-culture condition were incubated for 9 days in (1) 10% FBS-containing αMEM medium supplemented with osteogenic differentiation-inducing components (0.1 µM dexamethasone, 50 µM ascorbic acid, and 10 mM β-glycerophosphate), or (2) 10% FBS-containing αMEM medium without osteogenic differentiation-inducing components. The cells were cultured for 9 days, with the medium being changed with fresh medium of the same composition every 2 to 3 days. The osteogenic differentiation was evaluated by calcium assay and Alizarin Red S staining.

### 3.2 Results

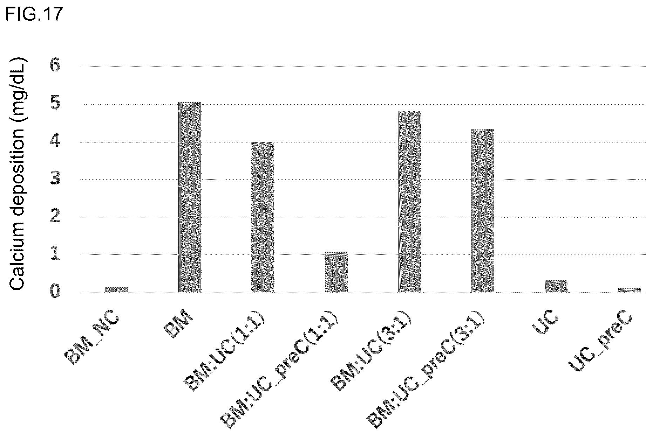

FIG. 17 shows the results of quantifying calcium deposition when bone marrow MSCs and umbilical cord MSCs were co-cultured. When bone marrow MSCs and umbilical cord MSCs were co-cultured at a ratio of 1:1 under osteogenic differentiation conditions (BM:UC(1: 1)), there were abundant calcium deposition. However, when bone marrow MSCs and umbilical cord MSCs pre-cultured with carnosine were co-cultured at a ratio of 1:1 under osteogenic differentiation conditions (BM:UC_preC(1:1)), calcium deposition was markedly low. The umbilical cord MSCs pre-cultured with carnosine were shown to inhibit MSC osteogenic differentiation. Carnosine was removed by washing the umbilical cord MSCs in PBS after pre-culture, and no carnosine was added to the culture medium during induction of osteogenic differentiation. From the results, the effect of purely cells (umbilical cord MSCs pre-cultured with carnosine) alone has been observed. Each term in the figure is described as follows.

BM: Non-co-culture conditions using bone marrow MSCs at 8 × 10^4 cells/well; αMEM medium conditions with osteogenic differentiation-inducing components.

BM_NC: non-co-culture conditions using bone marrow MSCs at 8 × 10^4 cells/well; αMEM medium conditions without osteogenic differentiation-inducing components.

UC: non-co-culture conditions using umbilical cord MSCs, at 8 × 10^4 cells/well, pre-cultured in carnosine-free MSC medium C for 2 weeks; αMEM medium conditions with osteogenic differentiation-inducing components.

UC_preC: non-co-culture conditions using umbilical cord MSCs, at 8 × 10^4 cells/well, pre-cultured in 5 mM carnosine-containing MSC medium C for 2 weeks; αMEM medium conditions with osteogenic differentiation-inducing components.

BM:UC(1:1): co-culture conditions in which bone marrow MSCs, at 4 × 10^4 cells/well, and umbilical cord MSCs, at 4 × 10^4 cells/well, pre-cultured in carnosine-free MSC medium C for 2 weeks were mixed and seeded; αMEM medium conditions with osteogenic differentiation-inducing components.

BM:UC_preC(1:1): co-culture conditions in which bone marrow MSCs, at 4 × 10^4 cells/well, and umbilical cord MSCs, at 4 × 10^4 cells/well, pre-cultured in 5 mM carnosine-containing CET original medium for 2 weeks were mixed and seeded; αMEM medium conditions with osteogenic differentiation-inducing components.

BM:UC(3:1): co-culture conditions in which bone marrow MSCs, at 6 × 10^4 cells/well, and umbilical cord MSCs, at 2 × 10^4 cells/well, pre-cultured in carnosine-free MSC medium C for 2 weeks were mixed and seeded; αMEM medium conditions with osteogenic differentiation-inducing components.

BM:UC_preC(3:1): co-culture conditions in which bone marrow MSCs, at 6 × 10^4 cells/well, and umbilical cord MSCs, at 2 × 10^4 cells/well, pre-cultured in 5 mM carnosine-containing CET original medium for 2 weeks were mixed and seeded; αMEM medium conditions with osteogenic differentiation-inducing components.

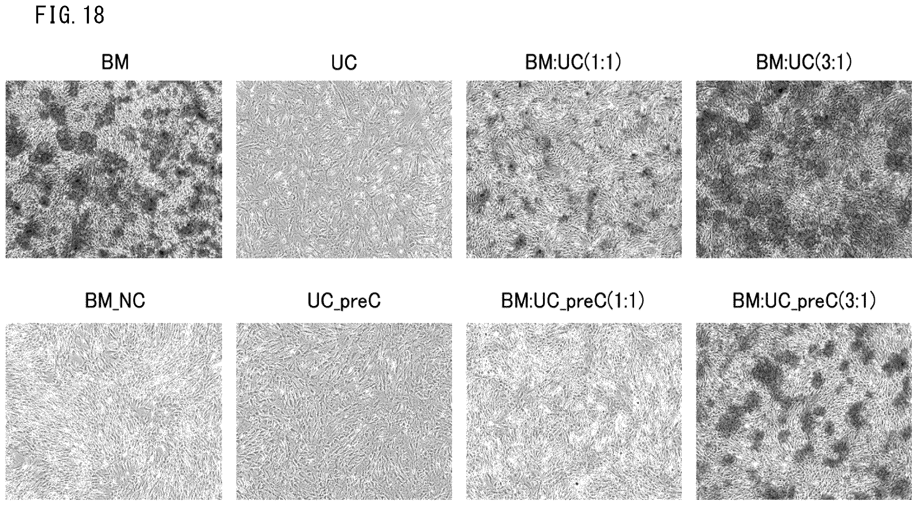

FIG. 18 is Alizarin Red S stained images when bone marrow MSCs and umbilical cord MSCs were co-cultured. When bone marrow MSCs and umbilical cord MSCs were co-cultured under osteogenic differentiation conditions (BM:UC (1:1)), there were abundant calcium deposition. However, when bone marrow MSCs and umbilical cord MSCs pre-cultured with carnosine were co-cultured under osteogenic differentiation conditions (BM:UC_preC (1:1)), calcium deposition was markedly low. The umbilical cord MSCs pre-cultured with carnosine were shown to inhibit MSC osteogenic differentiation.

### Example 4: Analysis of MSCs

### 4.1 Experimental procedure

### 4.1.1 To collect RNA from MSCs cultured using medium containing imidazole dipeptide

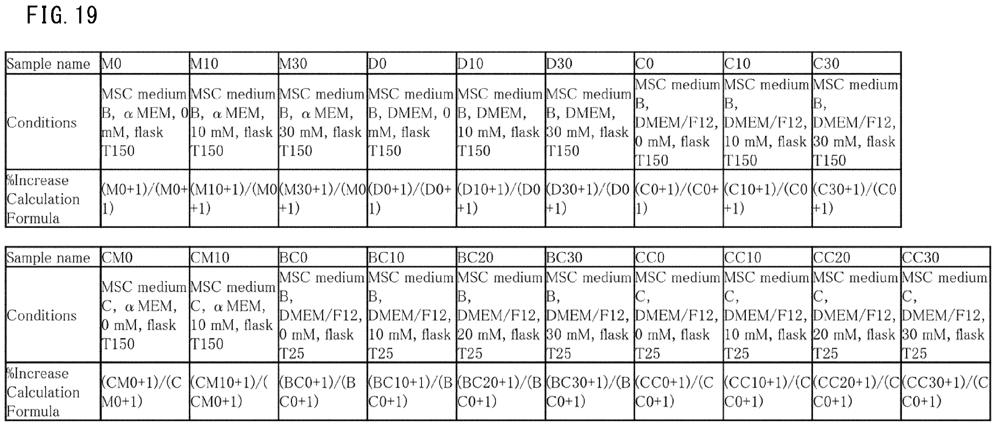

Human umbilical cord-derived mesenchymal stem cells (CET03 strain) established by the present inventors were used to recover the culture supernatant according to the experimental procedure up to the third production culture in Example 1 described above. Meanwhile, the growth medium (MSC medium B), supernatant recovery medium (MSC medium A), carnosine concentration, and cultureware used in Example 1 were changed as shown in FIG. 19. The supernatant recovery medium used was αMEM (Sigma), DMEM (Sigma), or DMEM/F12 (Sigma). The growth medium used was MSC medium B or MSC medium C (see Example 3 for composition). The concentration of carnosine added was 0 mM, 10, 20 or 30 mM. The resulting first, second, and third production culture supernatants were used in ELISA tests. RNA was then extracted from the cells and used for RNAseq analysis as described below.

### 4.1.2 RNAseq analysis

Cells were collected on day 27 after supernatant recovery, and RNA was collected using an miRNeasy Mini Kit (QIAGEN) and used for RNAseq analysis. NextSeq500 (Illumina, Inc.) was used as the sequence analyzer. Here, 75 bp per single read was used as the sequence read length and reads equivalent to 1 million reads were used in the analysis. The analysis program used was StrandNGS ver4.0 (Strand Life Sciences Pvt Ltd), and fastq reads after removal of unreliable nucleotides were used for mapping to the human reference genome hg38, and the RPM expression level was corrected. The RPM values corrected for each gene were calculated for a total of 19 samples.

### 4.1.3 Listing of genes with increased expression by RNAseq analysis

The %Increase of gene expression (under carnosine 0 mM condition vs. under carnosine 10 to 30 mM condition) was averaged for a total of 13 cell samples obtained under carnosine-added conditions. Genes with the high %Increase average in cells obtained under carnosine-added conditions were ranked from the top. The top 21 genes encoding secretory proteins were selected as follows. From the top: gremlin 1, DAN family BMP antagonist, inhibin subunit beta E, thymic stromal lymphopoietin, hyaluronan and proteoglycan link protein 3, KIT ligand, R-spondin 2, semaphorin 3B, ADAM metallopeptidase with thrombospondin type 1 motif 13, fibroblast growth factor 11, TNF receptor superfamily member 11b, angiopoietin like 7, ADAM metallopeptidase domain 8, fibroblast growth factor binding protein 3, hyaluronan and proteoglycan link protein 1, transforming growth factor beta regulator 4, interleukin 34, neurotrophin 3, insulin like growth factor binding protein 2, growth differentiation factor 6, amphiregulin, and dickkopf WNT signaling pathway inhibitor 1.

### 4.2 Results

RNAseq analysis picked up the top 21 secretory protein-coding genes, the expression level of which was increased by carnosine. The following 10 factors among them have been previously reported to be involved in the inhibition of bone formation: gremlin 1, DAN family BMP antagonist(GREM1, 26585, Gremlin 1), KIT ligand(KITLG, 4254, SCF), R-spondin 2(RSPO2, 340419), semaphorin 3B(SEMA3B, 7869), fibroblast growth factor 11(FGF11, 2256), TNF receptor superfamily member 11b(TNFRSF11B, 4982, Osteoprotegerin), ADAM metallopeptidase domain 8(ADAM8, 101), interleukin 34(IL34, 146433, IL-34), insulin like growth factor binding protein 2(IGFBP2, 3485), and dickkopf WNT signaling pathway inhibitor 1(DKK1, 22943). Each parenthesis means Official Symbol and Gene ID of NCBI, and optionally an alternative name. Among the secretory proteins upregulated by carnosine, the proportion of secretory proteins involved in osteogenesis inhibition was large, and carnosine was demonstrated to upregulate the expression of many kinds of secretory proteins involved in osteogenesis inhibition. These results have suggested that the inhibitory effect on osteogenic differentiation as produced by umbilical cord MSCs cultured under carnosine-added conditions is a paracrine effect caused by proteins secreted from the MSCs.

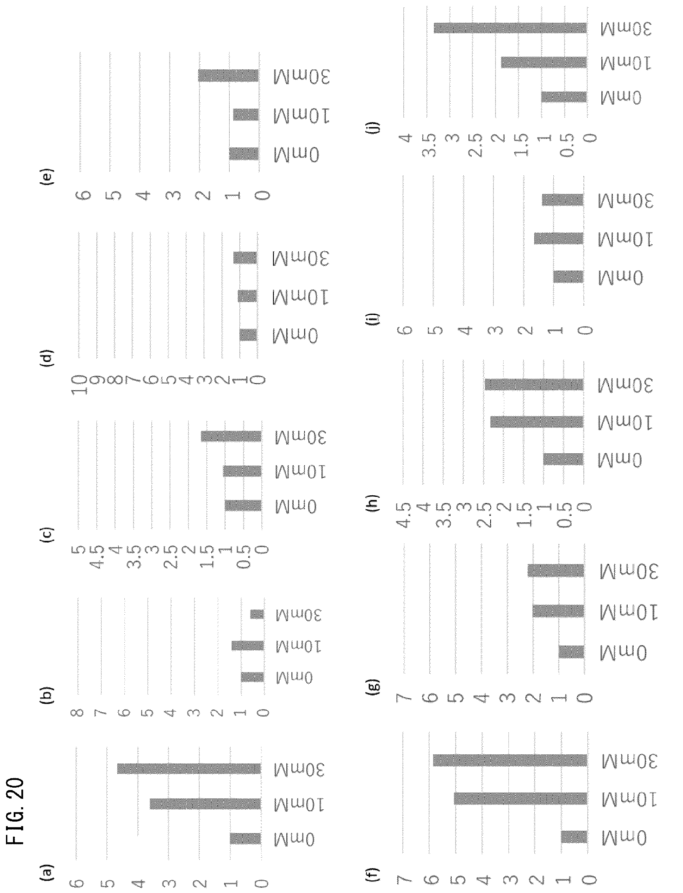

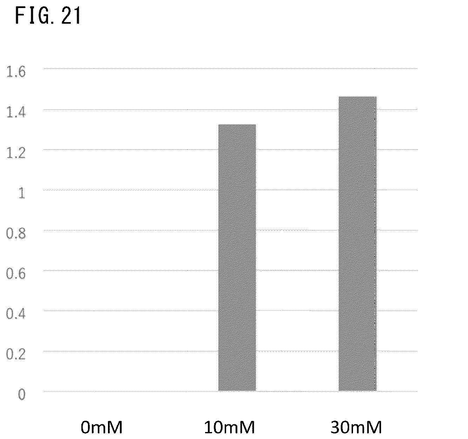

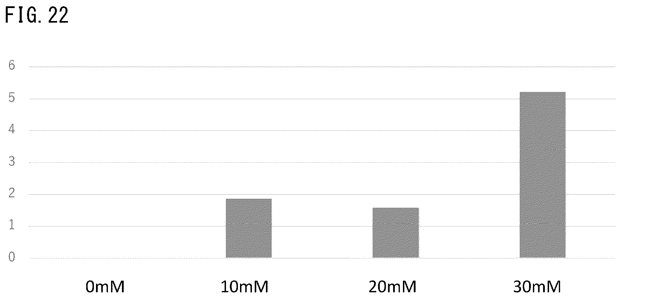

FIGS. 20(a) to 20(j) show the changes in %Increase values for the above 10 different genes. FIGS. 20(a) to 20(j) show the results of, in sequence, gremlin 1, DAN family BMP antagonist, KIT ligand, R-spondin 2, semaphorin 3B, fibroblast growth factor 11, TNF receptor superfamily member 11b, ADAM metallopeptidase domain 8, interleukin 34, insulin like growth factor binding protein 2, and dickkopf WNT signaling pathway inhibitor 1. FIGS. 20 to 22 show the results when DMEM/F12 (Sigma) was used as the supernatant recovery medium and MSC medium B was used as the growth medium. The concentrations in the figures are each the concentration of carnosine added. FIG. 21 is a graph showing the RPM values of interleukin 34 expression level in umbilical cord MSCs. Expression of interleukin 34 was observed when carnosine was added at 10 mM or 30 mM, but not when no carnosine was added (0 mM). FIG. 22 is a graph showing the RPM values of ADAM metallopeptidase domain 8 expression level in umbilical cord MSCs. Expression of ADAM metallopeptidase domain 8 was observed when carnosine was added at 10 mM, 20 mM, or 30 mM, but not when no carnosine was added (0 mM).

### Example 5: To analyze culture supernatant and MSCs

### 5.1 To culture MSCs using medium containing imidazole dipeptide and to recover culture supernatant

Human umbilical cord-derived mesenchymal stem cells (CET03 strain) established by the present inventors were used to recover the culture supernatant according to the experimental procedure up to the third production culture in Example 1 described above. In this case, the MSC medium A used in Example 1 was replaced by αMEM (Sigma), DMEM (Sigma), or DMEM/F12 (Sigma). In addition, the concentration of carnosine added was changed to 0 or 30 mM (500 mM carnosine solution in distilled water was used for the addition; in the case of 0 mM, the same volume of distilled water was used). The resulting first, second, and third production culture supernatants were obtained and used in ELISA tests. RNA was then extracted from the cells and used for RNAseq analysis as described below.

### 5.2 ELISA analysis

The levels of cytokines contained in each culture supernatant obtained in the above Section 5.1 were analyzed using an ELISA analysis kit (R&D Systems). Osteoprotegerin and M-CSF (CSF1, 1435) were analyzed as the cytokines. These two cytokines are factors previously reported to be involved in osteogenesis inhibition. In this experiment, the levels of cytokines are indicated by the value measured by ELISA using each specific antibody.

### 5.3 Quantitative PCR (qPCR) analysis

Cells were collected on day 27 after supernatant recovery, and RNA was collected using an miRNeasy Mini Kit (QIAGEN). Quantitative PCR (PowerUp SYBR Green Master Mix, Thermo Fisher) was used to analyze the expression levels of TNFRSF11B, the gene for Osteoprotegerin, and CSF1, the gene for M-CSF, while using the Glyceraldehyde-3-phosphate dehydrogenase (GAPDH) gene as an internal standard.

### 5.4 Results

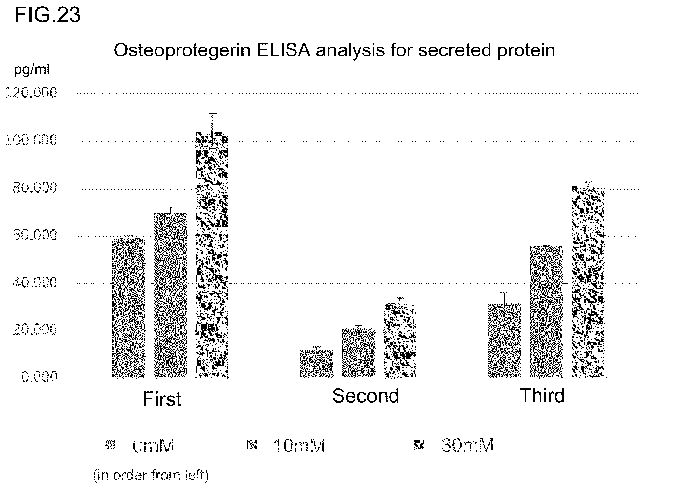

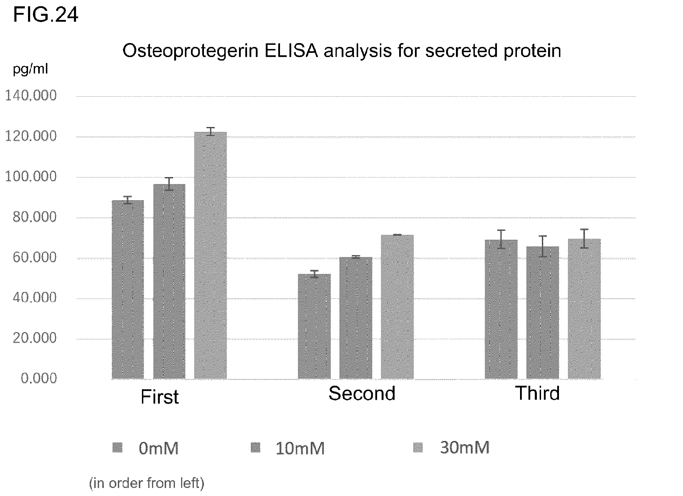

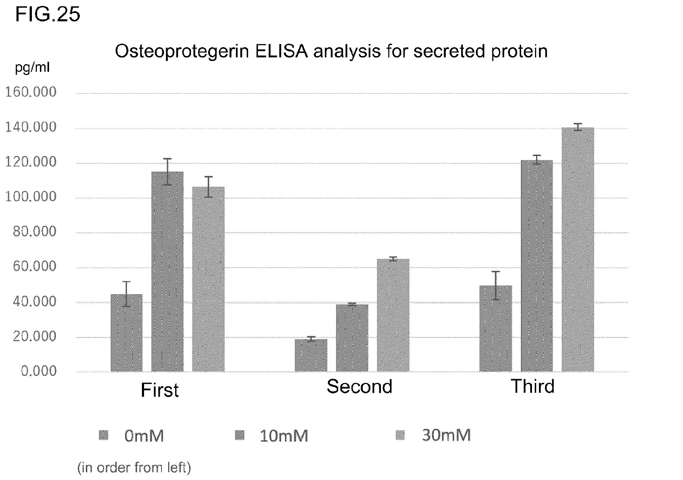

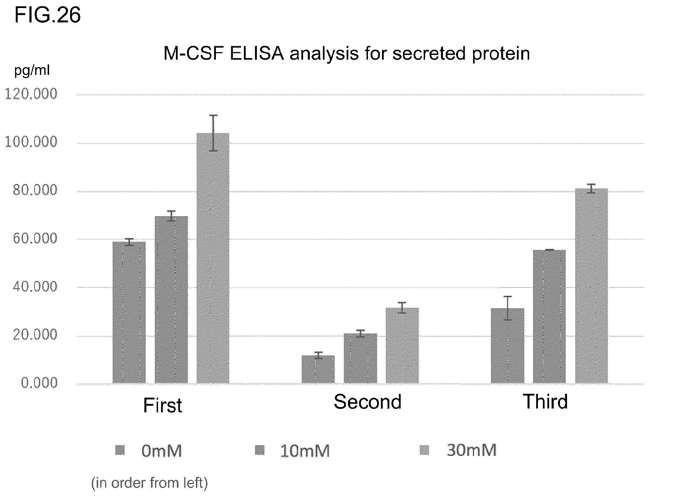

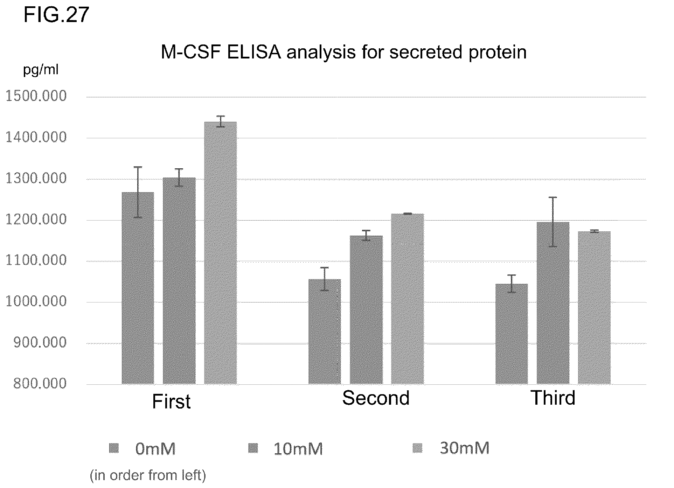

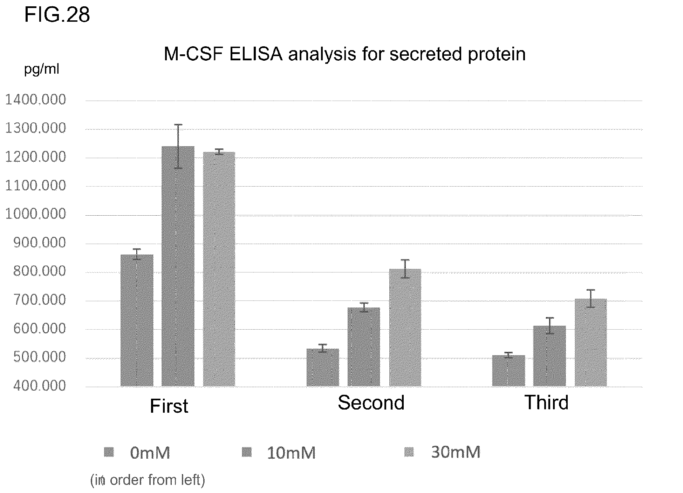

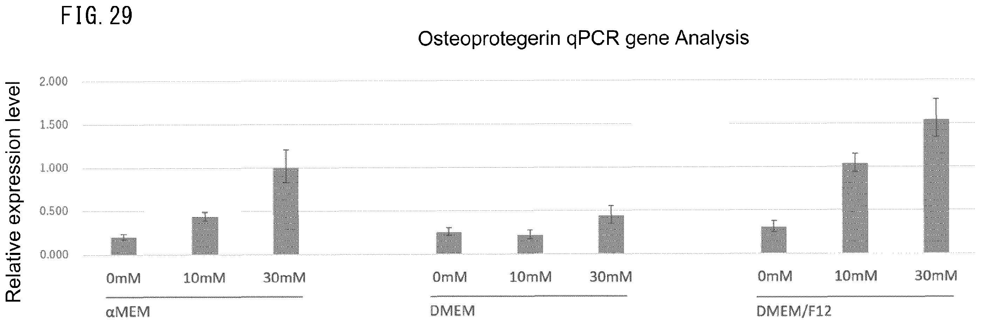

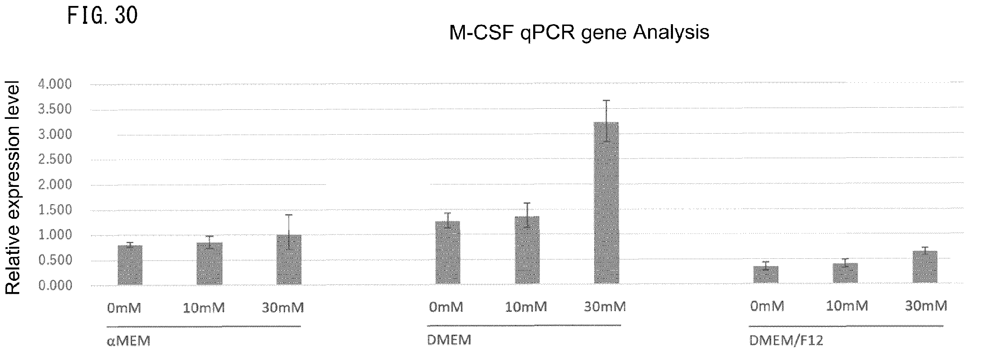

FIGS. 23 to 25 show the results of ELISA analysis of Osteoprotegerin, and FIGS. 26 to 28 show the results of ELISA analysis of M-CSF. The concentration (mM) in the figures refers to carnosine concentration. The supernatant recovery medium used was αMEM for FIGS. 23 and 26, DMEM for FIGS. 24 and 27, and DMEM/F12 for FIGS. 25 and 28. The addition of carnosine tended to increase Osteoprotegerin and M-CSF secretion in a concentration-dependent manner at the respective first, second, and third recovery times. FIG. 29 shows the results of qPCR analysis of Osteoprotegerin, and FIG. 30 shows the results of qPCR analysis of M-CSF. The concentration (mM) in the figures indicates carnosine concentration, and the name of the medium indicates the supernatant recovery medium used. Under each condition, the addition of carnosine tended to increase Osteoprotegerin and M-CSF gene expression in a concentration-dependent manner.

### Example 6: To analyze culture supernatant and MSCs

### 6.1 To culture MSCs using medium containing imidazole dipeptide and to recover culture supernatant

Human umbilical cord-derived mesenchymal stem cells (CET03 strain) established by the present inventors or human bone marrow-derived mesenchymal stem cells (ATCC) were used to recover the culture supernatant according to the experimental procedure up to the third production culture in Example 1 described above. In this case, MSC medium B or MSC medium C (see Example 3 for composition) was used as the growth medium (MSC medium B in Example 1). In addition, the supernatant recovery medium (MSC medium A in Example 1) used was DMEM/F12 (Sigma). In addition, the concentration of carnosine added was changed to 0, 10, 20, or 30 mM (500 mM carnosine solution in distilled water was used for the addition; in the case of 0 mM, the same volume of distilled water was used). In addition, instead of carnosine, anserine, an imidazole dipeptide, was added at a final concentration of 0, 5, 10, or 20 mM. The resulting first, second, and third production culture supernatants were obtained and used in ELISA tests. RNA was then extracted from the cells and used for quantitative PCR testing as described below. The procedures for ELISA analysis and quantitative PCR testing are the same as in Example 5.

### 6.2 Results

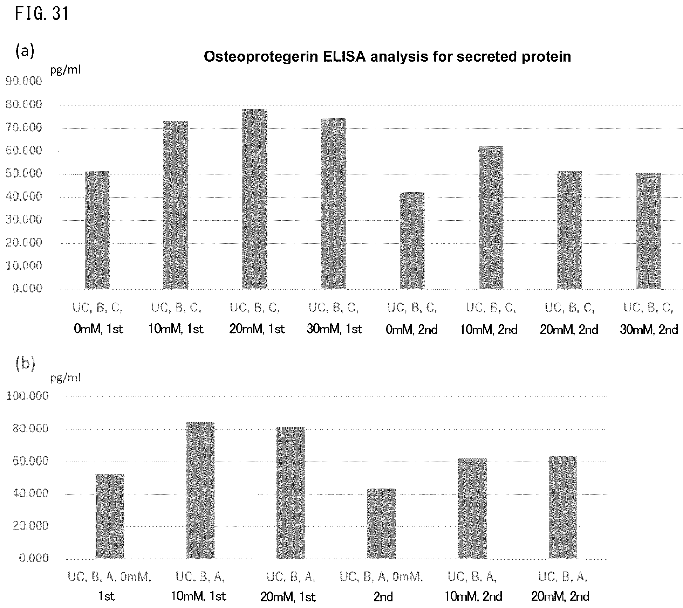

FIG. 31 shows the results of ELISA analysis of Osteoprotegerin. FIG. 31(a) shows the results under carnosine-added conditions and FIG. 31(b) shows the results under anserine-added conditions. In both cases, umbilical cord MSCs were used as the cells and MSC medium B was used as the growth medium. The addition of carnosine or anserine tended to increase Osteoprotegerin secretion at the respective first and second recovery times.

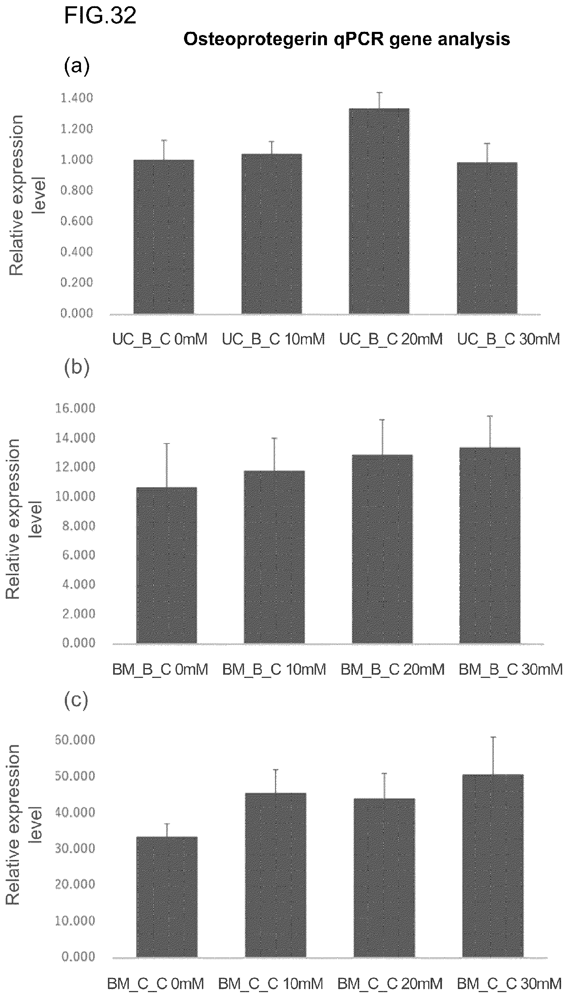

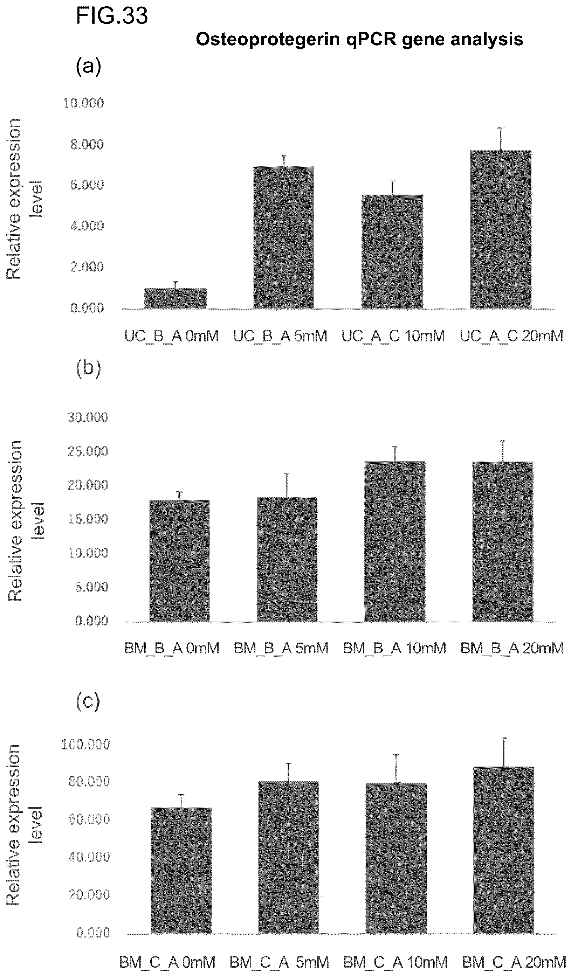

FIGS. 32 to 33 shows the results of qPCR analysis of Osteoprotegerin. FIGS. 32(a) to (c) show the results under carnosine-added conditions and FIGS. 33(a) to (c) show the results under anserine-added conditions. In each figure, UC_B_C means that umbilical cord MSCs were used as the cells, MSC medium B was used as the growth medium, and carnosine was used as the additive. In each figure, BM_B_C means that bone marrow MSCs were used as the cells, MSC medium B was used as the growth medium, and carnosine was used as the additive. In each figure, BM_C_C means that bone marrow MSCs were used as the cells, MSC medium C was used as the growth medium, and carnosine was used as the additive. In each figure, UC_B_A means that umbilical cord MSCs were used as the cells, MSC medium B was used as the growth medium, and anserine was used as the additive. In each figure, BM_B_A means that bone marrow MSCs were used as the cells, MSC medium B was used as the growth medium, and anserine was used as the additive. In each figure, BM_C_A means that bone marrow MSCs were used as the cells, MSC medium C was used as the growth medium, and anserine was used as the additive. Under each condition, the addition of carnosine or anserine tended to increase Osteoprotegerin gene expression levels.

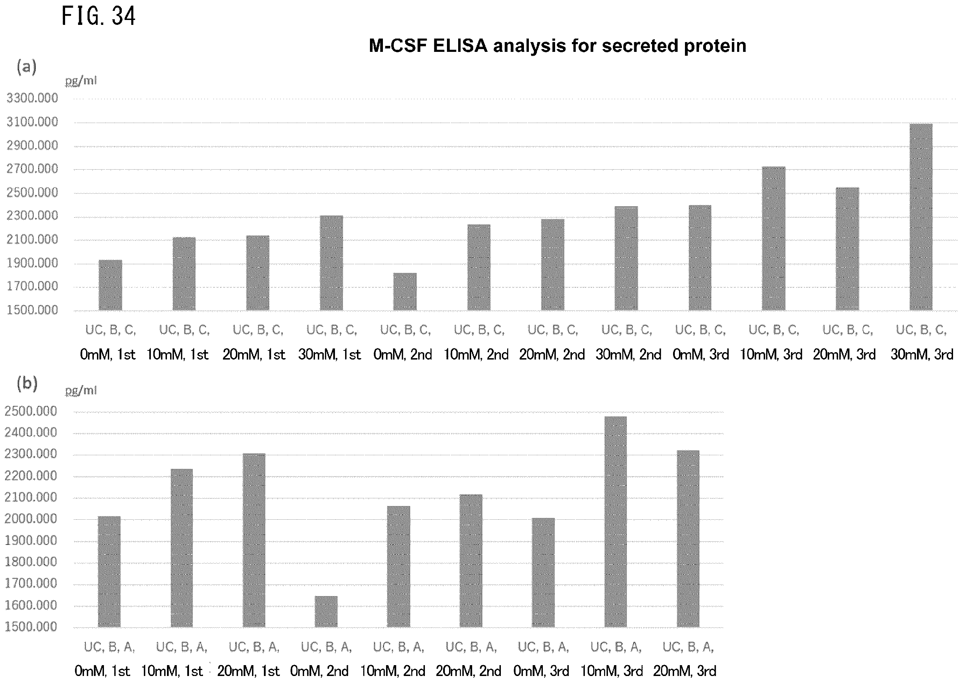

FIG. 34 shows the results of ELISA analysis of M-CSF. FIG. 34(a) shows the results under carnosine-added conditions and FIG. 34(b) shows the results under anserine-added conditions. In both cases, umbilical cord MSCs were used as the cells and MSC medium B was used as the growth medium. The addition of carnosine or anserine tended to increase M-CSF secretion at the respective first and second recovery times.

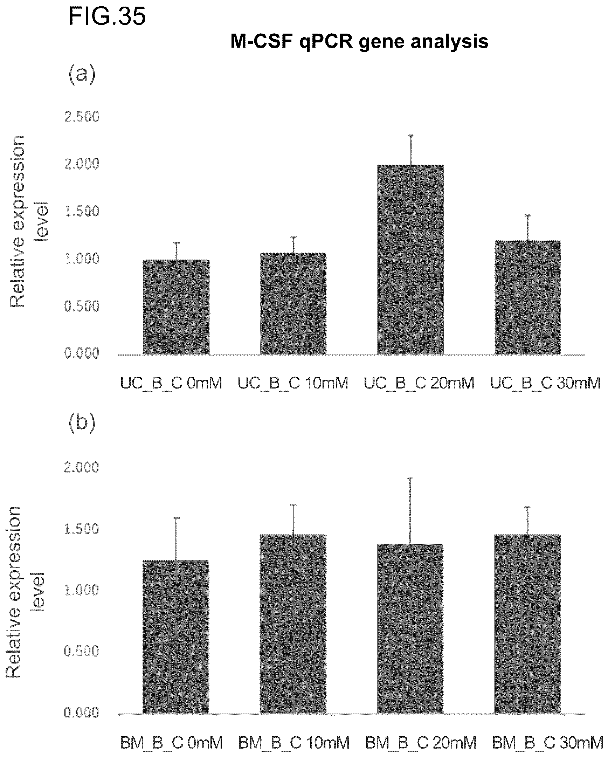

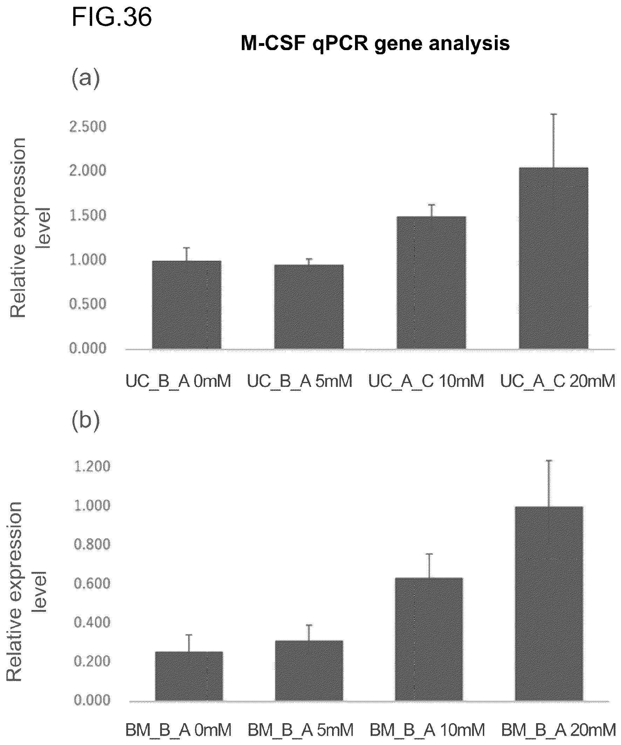

FIGS. 35 to 36 show the results of qPCR analysis of M-CSF. FIGS. 35(a) to (c) show the results under carnosine-added conditions and FIGS. 36(a) to (c) show the results under anserine-added conditions. In each figure, UC_B_C means that umbilical cord MSCs were used as the cells, MSC medium B was used as the growth medium, and carnosine was used as the additive. In each figure, BM_B_C means that bone marrow MSCs were used as the cells, MSC medium B was used as the growth medium, and carnosine was used as the additive. In each figure, UC_B_A means that umbilical cord MSCs were used as the cells, MSC medium B was used as the growth medium, and anserine was used as the additive. In each figure, BM_B_A means that bone marrow MSCs were used as the cells, MSC medium B was used as the growth medium, and anserine was used as the additive. Under each condition, the addition of carnosine or anserine tended to increase M-CSF gene expression levels.

### Example 7: Effect of MSCs on growth promotion

### 7.1 Experimental procedure

### 7.1.1 MSC expansion culture

Human umbilical cord-derived mesenchymal stem cells established by the present inventors were detached using TrypLE Select and seeded onto a 24-well plate at a cell density of 200 or 2000 cells/cm². The culture medium used was (1) MSC Expansion XSFM B (FUJIFILM Wako Pure Chemical Corporation) (hereinafter, also referred to as MSC medium B), (2) DMEM/F12 medium containing 10 ng/ml bFGF (PeproTech), 10 µg/ml insulin (NACALAI TESQUE), 1000 µg/ml albumin (Sigma), 10 ug/ml transferrin (NACALAI TESQUE), and 1 ng/ml LIF (PeproTech) (hereinafter, also referred to as MSC medium D), or (3) MSC medium D minus LIF (hereinafter, also referred to as MSC medium E). In addition, iMatrix-511 laminin fragment (Nippi, Inc.) was added to each medium at a final concentration of 0 or 0.1%, and carnosine or anserine at a final concentration of 0, 0.2, 0.5, 1, 2, or 5 mM was added, and the mixture was seeded. Each sample was cultured for 4 to 7 days while the medium was changed to the same medium as that used every 2 to 3 days. The cells were then stained with DAPI, phalloidin, and CD44 antibody. Each fluorescence image was captured using a confocal quantitative image cytometer CellVoyager CQ-1 (Yokogawa Electric Corporation). The cells were counted using DAPI, and the fluorescence area of phalloidin (actin filament marker) or CD44 (MSC marker) was then measured. A summary of each experimental condition is shown in Table 1.

**[Table 1]**

| Group | Medium | Imidazole dipeptide | Adhesion molecule | Seeding cell density |
|---|---|---|---|---|
| 1 | B | Carnosine: 0, 0.2, 0.5, 1, 2, or 5 mM | Laminin fragment | 200 cells/cm² |
| 2 | B | Carnosine: 0, 0.2, 0.5, 1, 2, or 5 mM | None | 2000 cells/cm² |
| 3 | D | Carnosine: 0, 0.2, 0.5, 1, 2, or 5 mM | Laminin fragment | 200 cells/cm² |
| 4 | D | Carnosine: 0, 0.2, 0.5, 1, 2, or 5 mM | None | 2000 cells/cm² |
| 5 | B | Anserine: 0, 0.2, 0.5, 1, 2, or 5 mM | Laminin fragment | 200 cells/cm² |
| 6 | B | Anserine: 0, 0.2, 0.5, 1, 2, or 5 mM | None | 2000 cells/cm² |
| 7 | D | Anserine: 0, 0.2, 0.5, 1, 2, or 5 mM | Laminin fragment | 200 cells/cm² |
| 8 | D | Anserine: 0, 0.2, 0.5, 1, 2, or 5 mM | None | 2000 cells/cm² |

In addition, umbilical cord MSCs established by the present inventors were detached using TrypLE Select and seeded onto a 24-well plate at a cell density of 200 cells/cm². The culture medium used was (4) MSC medium D, or (5) MSC medium D minus LIF (hereinafter, also referred to as MSC medium E). In addition, iMatrix-511 laminin fragment (Nippi, Inc.) was added to each medium at a final concentration of 0.1%, and carnosine at a final concentration of 0, 0.2, 1, or 5 mM was added, and the mixture was seeded. Each sample was cultured for 7 days while the medium was changed to the same medium as that used every 2 to 3 days. The cells were then stained with DAPI and phalloidin. Each fluorescence image was captured using a confocal quantitative image cytometer CellVoyager CQ-1 (Yokogawa Electric Corporation). The cells were counted using DAPI, the fluorescence area of phalloidin (actin filament marker) was then measured. A summary of each experimental condition is shown in Table 2.

**[Table 2]**

| Group | Medium | Imidazole dipeptide | Adhesion molecule | Seeding cell density |
|---|---|---|---|---|
| 9 | D | Carnosine: 0, 0.2, 1, or 5 mM | Laminin fragment | 200 cells/cm² |
| 10 | E | Carnosine: 0, 0.2, 1, or 5 mM | Laminin fragment | 200 cells/cm² |

### 7.1.2 To measure positive rate of MSC markers

Carnosine was added to MSC medium D, and umbilical cord MSCs were seeded at 200 cells/cm² and cultured in a T25 flask for 2 weeks (2 passages). The cells seeded in a T25 flask were detached with TrypLE Select and collected, and made into single cells. The collected cells were fixed with 4% formaldehyde solution, immunostained with CD31, 44, 45, 73, 90, and 105, HLA-ABC, and HLA-DR, and the percentage of cells positive for each marker was measured using a BD Accuri^{™} C6 Plus flow cytometer (BD Biosciences).

### 7.2 Results

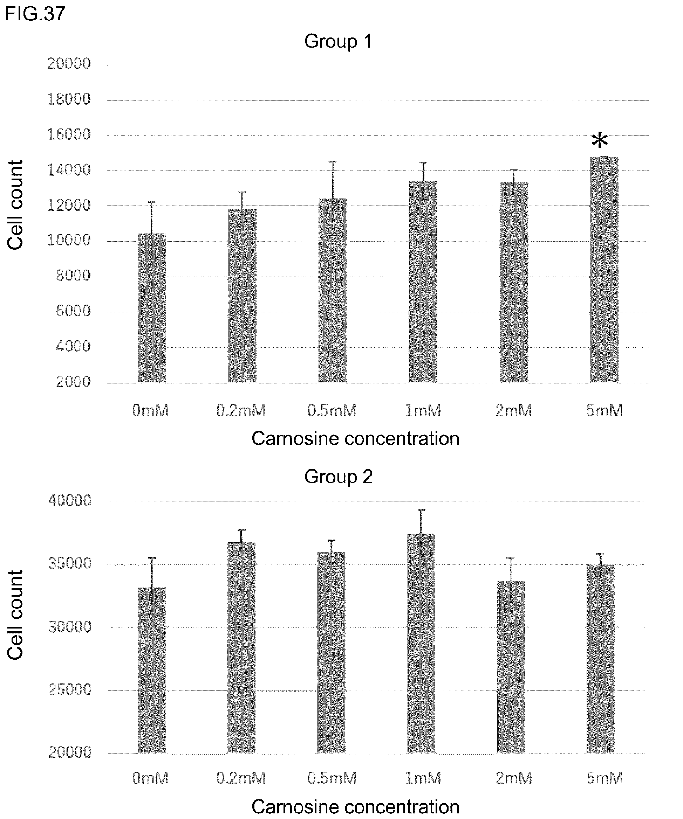

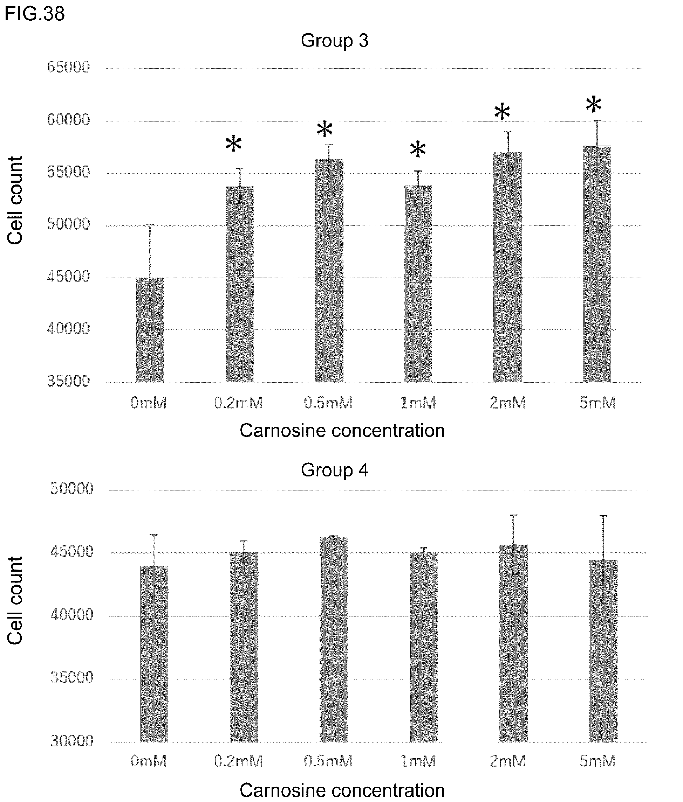

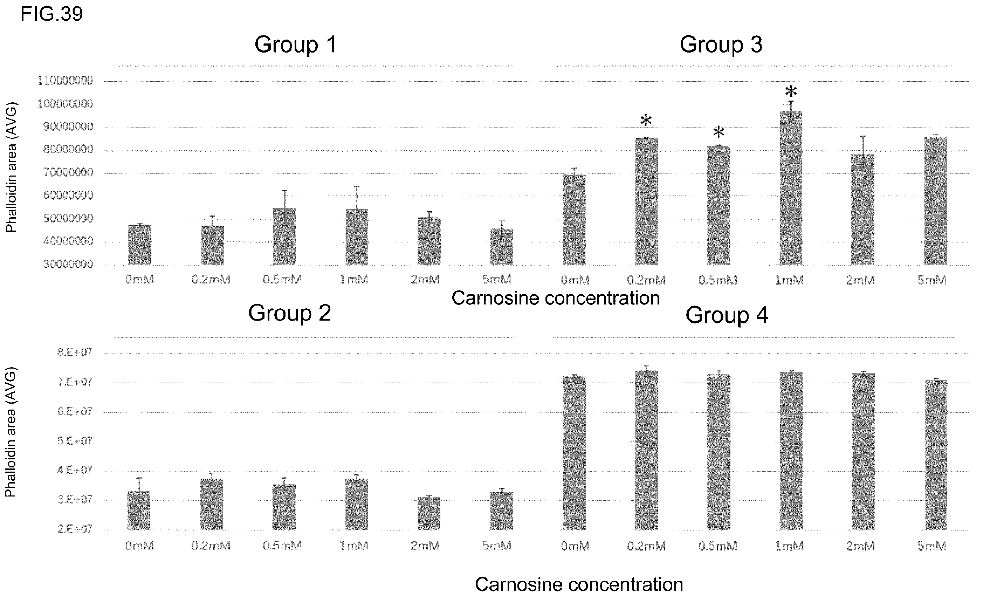

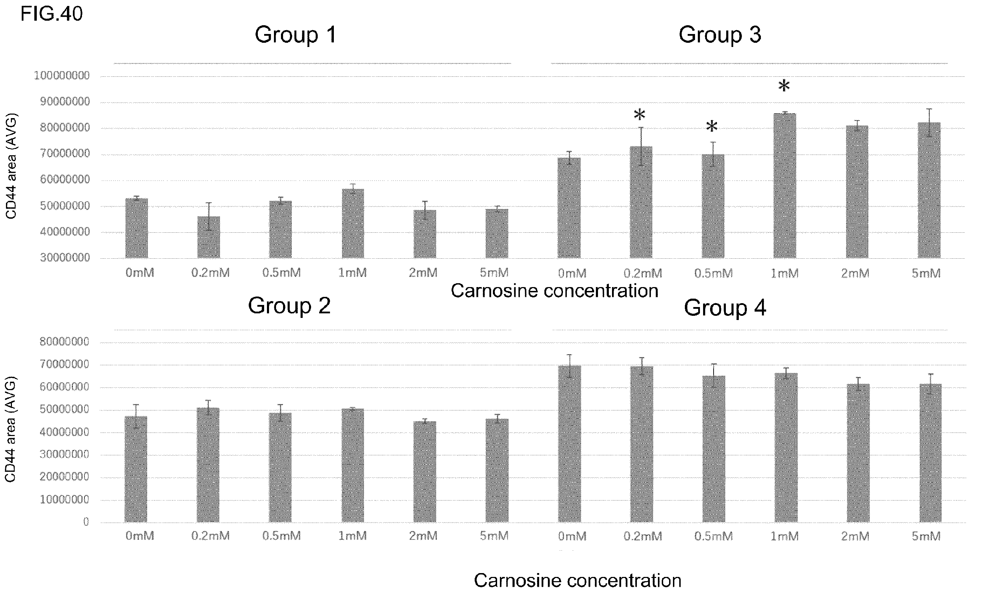

FIGS. 37 to 38 show the results of counting cells in groups 1 to 4. Especially in group 3, the addition of carnosine tended to increase the number of cells. FIG. 39 shows the results of measuring a phalloidin fluorescence area in groups 1 to 4. Especially in group 3, the addition of carnosine tended to increase the phalloidin area. FIG. 40 shows the results of measuring a CD44 fluorescence area in groups 1 to 4. Especially in group 3, the addition of carnosine tended to increase the CD44 area.

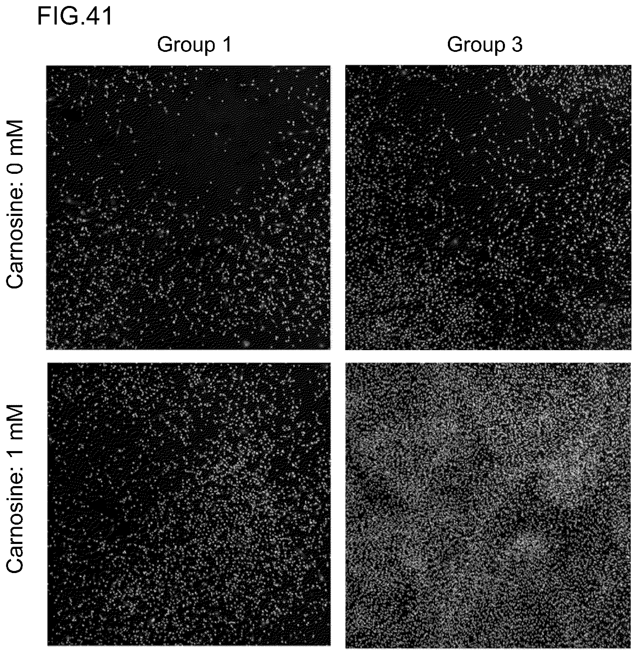

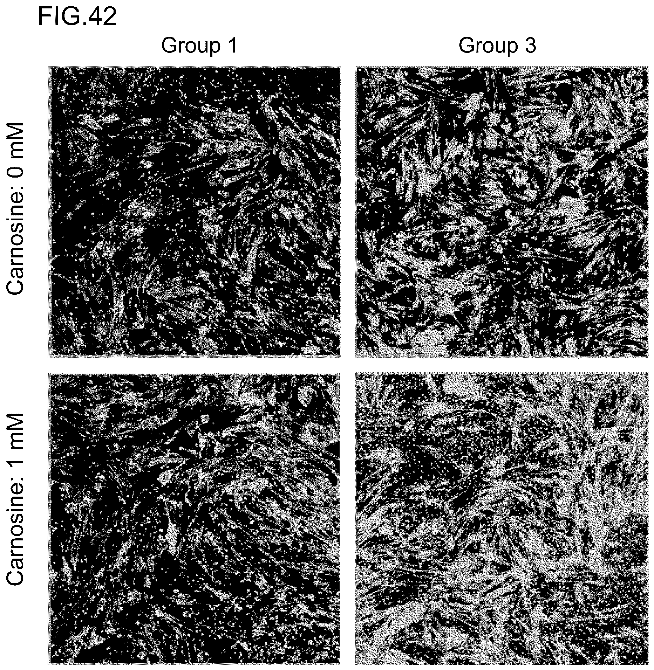

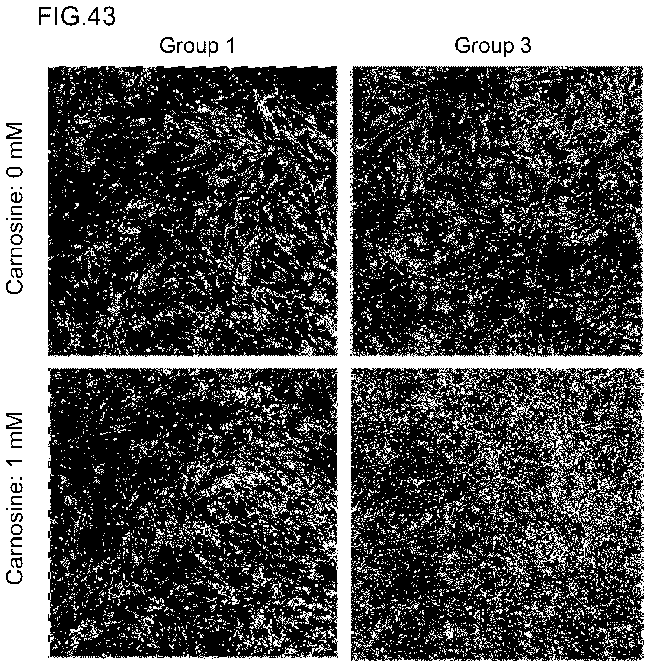

FIG. 41 shows the results of photographing fluorescent images (cell nucleus staining) of group 1 or 3. Especially in group 3, the number of cells tended to increase under the 1 mM carnosine conditions. FIG. 42 shows the results of photographing fluorescent images (phalloidin staining) of group 1 or 3. Especially in groups 1 and 3, the number of cells and the phalloidin area tended to increase under the 1 mM carnosine conditions. No significant differences in cell morphology were observed. FIG. 43 shows the results of photographing fluorescent images (CD44 staining) of group 1 or 3. Especially in group 3, the number of cells and the CD44 area tended to increase under the 1 mM carnosine conditions. No significant differences between their marker expression patterns were observed.

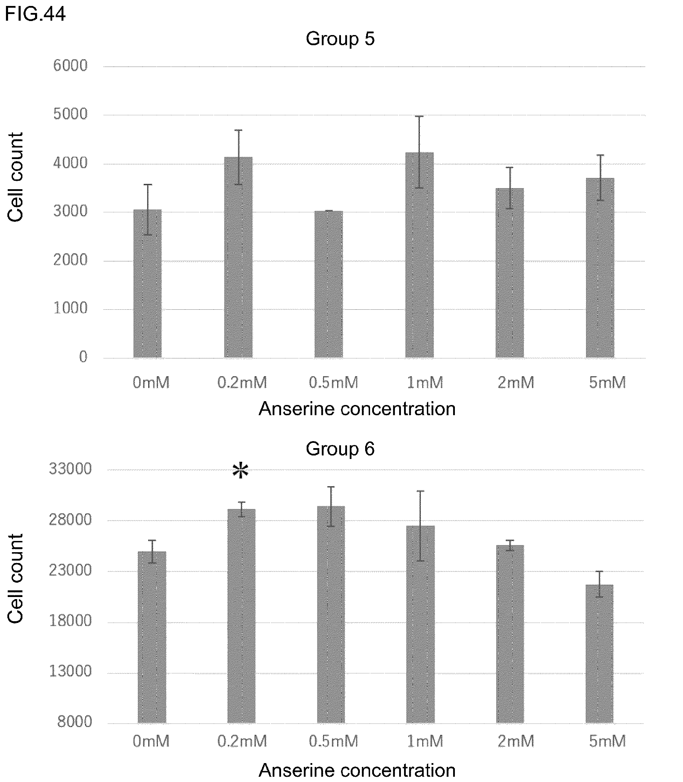

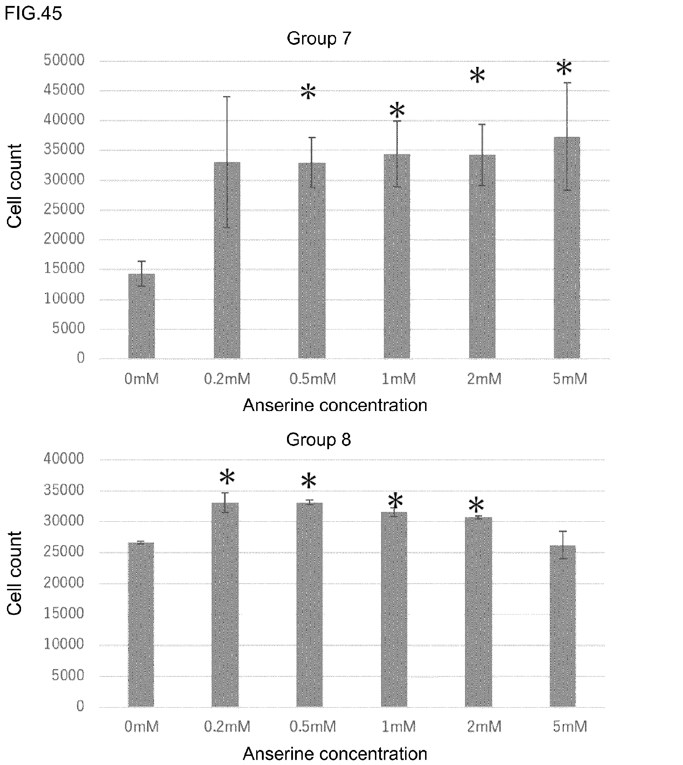

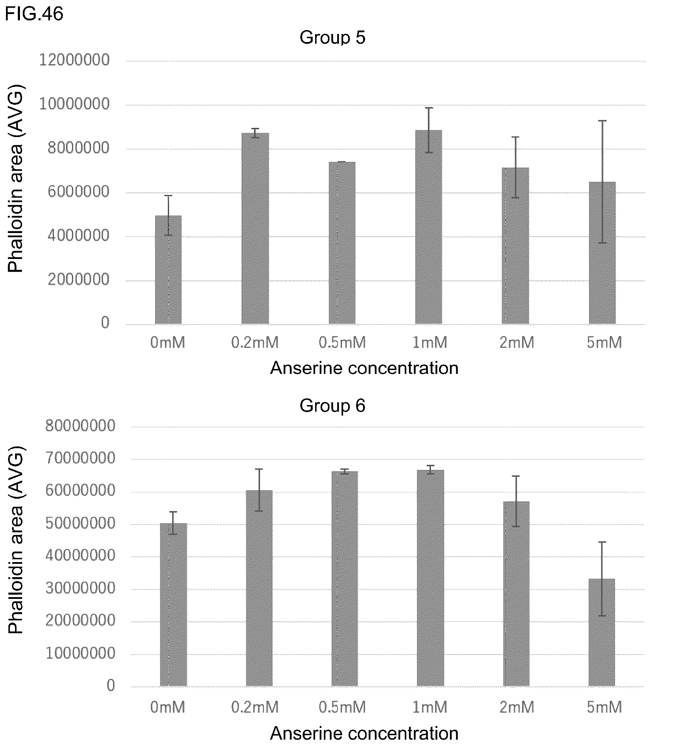

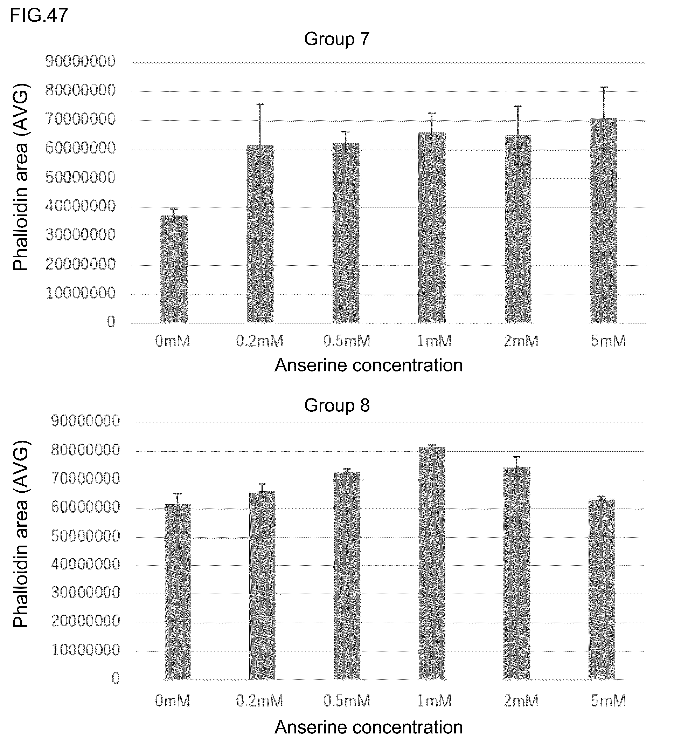

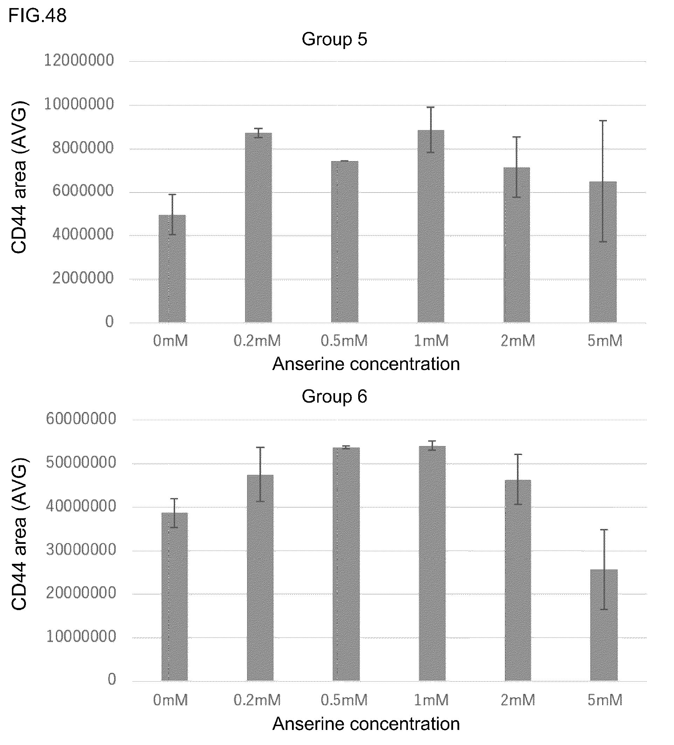

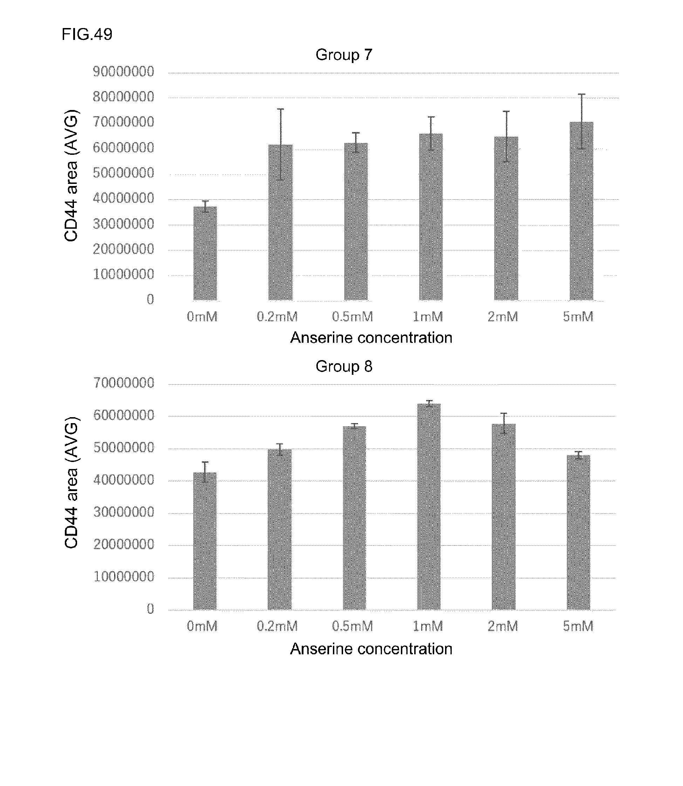

FIGS. 44 to 45 show the results of counting cells in groups 5 to 8. Especially in group 7, the addition of anserine tended to increase the number of cells. FIGS. 46 to 47 show the results of measuring a phalloidin fluorescence area in groups 5 to 8. Especially in group 7, the addition of anserine tended to increase the phalloidin area. FIGS. 48 to 49 show the results of measuring a CD44 fluorescence area in groups 5 to 8. Especially in group 7, the addition of anserine tended to increase the CD44 area.

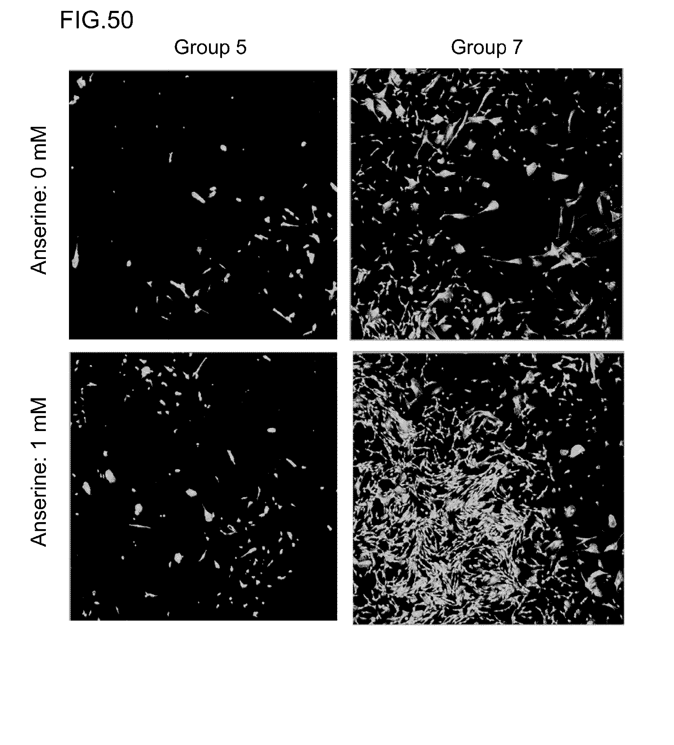

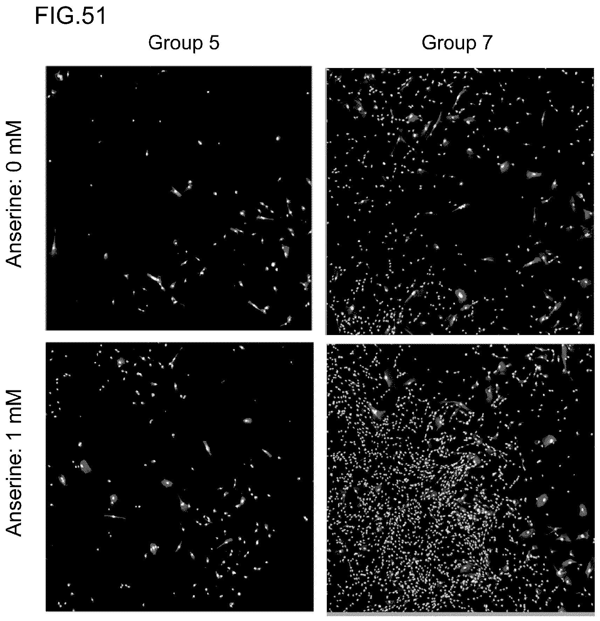

FIG. 50 shows the results of photographing fluorescent images (phalloidin staining) of group 5 or 7. Especially in group 7, the number of cells and the phalloidin area tended to increase under the 1 mM anserine conditions. No significant differences in cell morphology were observed. FIG. 51 shows the results of photographing fluorescent images (CD44 staining) of group 5 or 7. Especially in group 7, the number of cells and the CD44 area tended to increase under the 1 mM anserine conditions. No significant differences between their marker expression patterns were observed.

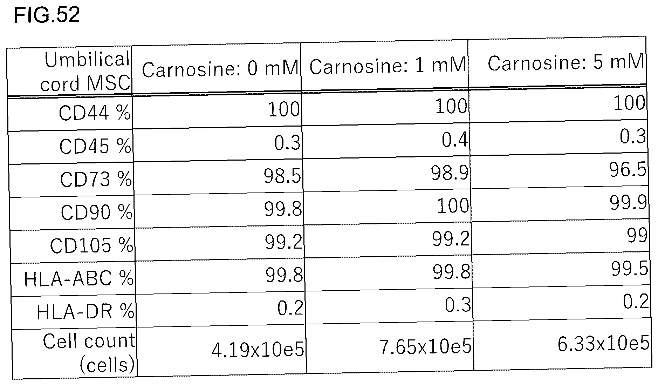

FIG. 52 shows the results of measuring the positive rate of each MSC marker after culturing umbilical cord MSCs in MSC medium D. The number of cells was increased by the addition of carnosine, while each MSC-positive or -negative marker was unchanged by the addition of carnosine.

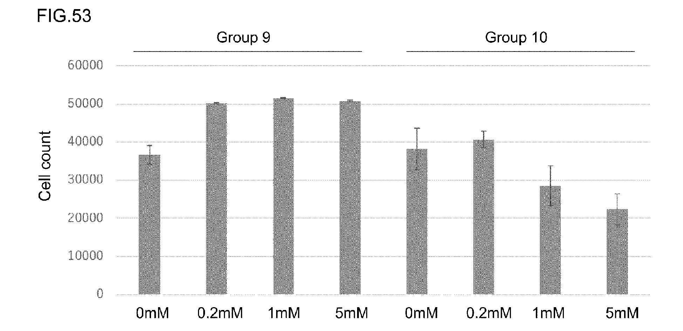

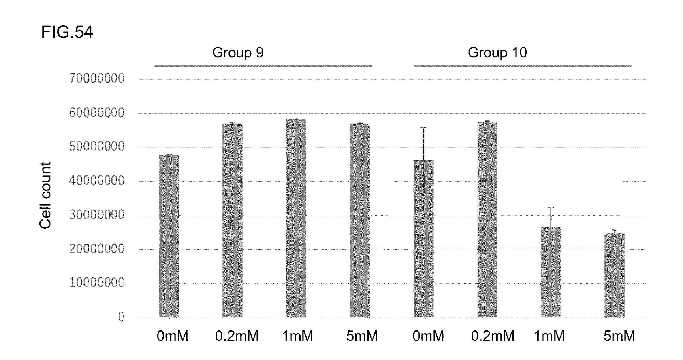

FIG. 53 shows the results of counting cells in group 9 or 10. In group 9, the addition of carnosine tended to increase the number of cells, while group 10 did not exhibit the increasing trend. FIG. 54 shows the results of measuring a phalloidin fluorescence area in group 9 or 10. In group 9, the addition of carnosine tended to increase the phalloidin area, while group 10 did not exhibit the increasing trend.

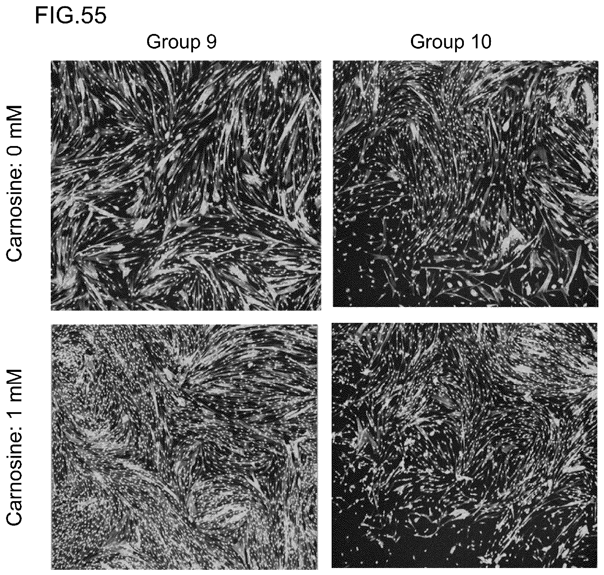

FIG. 55 shows the results of photographing fluorescent images (phalloidin staining) of group 9 or 10. In group 9 under 1 mM carnosine conditions, the addition of carnosine tended to increase the phalloidin area. By contrast, no increasing trend was observed under the 1 mM carnosine conditions in group 10.

The above results have demonstrated that the addition of carnosine to the LIF-containing medium promotes the proliferation of MSCs.

Hereinabove, the present invention has been described based on the Examples. The Examples are just examples. Those skilled in the art should understand that various modifications are allowed and such modified embodiments are within the scope of the present invention.

Sequence Listing

## Claims

1. A method for producing a culture supernatant, comprising the step of recovering a culture supernatant from a medium containing mesenchymal stem cells and an imidazole dipeptide.

2. The production method according to claim 1, wherein the mesenchymal stem cells are mesenchymal stem cells cultured in a medium containing an imidazole dipeptide.

3. The production method according to claim 1 or 2, comprising a step of culturing the mesenchymal stem cells in a medium containing an imidazole dipeptide to generate cultured mesenchymal stem cells.

4. The production method according to any one of claims 1 to 3, wherein the recovering step comprises a step of centrifuging or filtering the medium containing mesenchymal stem cells and an imidazole dipeptide.

5. The production method according to any one of claims 1 to 4, comprising a step of sterilizing the recovered culture supernatant to prepare a sterilized culture supernatant.

6. The production method according to any one of claims 1 to 5, wherein the culture supernatant is a culture supernatant obtained by adhesion culture.

7. The production method according to any one of claims 1 to 6, wherein the culture supernatant contains at least 500 pg/mL of G-CSF or at least 2700 pg/mL of IL-6.

8. The production method according to any one of claims 1 to 7, wherein the culture supernatant contains G-CSF, MCP-1, VEGF-C, TGF-β1, IL-6, IL-7, IL-8, and exosomes.

9. The production method according to any one of claims 1 to 8, wherein the imidazole dipeptide is a compound having a structure of the following formula (1) or (2): 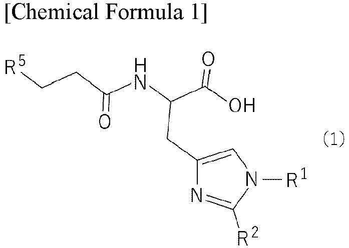 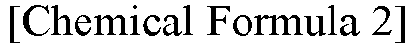 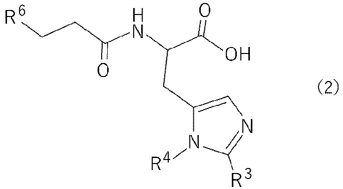 wherein R¹, R², R³ and R⁴ are each independently H or C₁₋₆ alkyl, R⁵ and R⁶ are each independently -NHR⁷ or -CH₂NHR⁷, R⁷ is H or -COR⁸, R⁸ is H, C₁₋₆ alkyl, optionally substituted phenyl, -OCH₂R⁹, or -CH=CHR⁹, and R⁹ is H, C₁₋₆ alkyl, or optionally substituted phenyl.

10. The production method according to any one of claims 1 to 9, wherein the imidazole dipeptide is carnosine, anserine, balenine, or homocarnosine.

11. The production method according to any one of claims 1 to 10, wherein the imidazole dipeptide is carnosine.

12. The production method according to any one of claims 1 to 10, wherein the imidazole dipeptide is anserine.

13. A culture supernatant obtained by the production method according to any one of claims 1 to 12.

14. A pharmaceutical composition comprising a culture supernatant obtained by the production method according to any one of claims 1 to 12.

15. The culture supernatant according to claim 13 or the pharmaceutical composition according to claim 14, for inhibiting osteogenic differentiation.

16. A syringe, vial, or medical bag comprising the culture supernatant according to claim 13 or the pharmaceutical composition according to claim 14 or 15.

17. A method of culturing cells, comprising the step of culturing mesenchymal stem cells in a serum-free medium containing an imidazole dipeptide to generate cultured cells.

18. The culture method according to claim 17, comprising a step of recovering the cultured cells from the medium.

19. The culture method according to claim 17 or 18, wherein the culturing comprises a step of culturing in the presence of an adhesion factor.

20. The culture method according to any one of claims 17 to 19, wherein the medium is an insulin-free, IGF-free, or FGF-free medium.

21. The culture method according to any one of claims 17 to 19, wherein the medium is a growth factor-free medium.

22. The culture method according to any one of claims 17 to 19, wherein the medium is a protein-free medium.

23. A method for producing cells, comprising the step of performing the culture method according to any one of claims 17 to 22.

24. A cell obtained by the production method according to claim 23.

25. A composition comprising a cell population of the cells according to claim 24.

26. A pharmaceutical composition comprising cells obtained by the production method according to claim 23.

27. The pharmaceutical composition according to claim 26, for inhibiting osteogenic differentiation.

28. A medium for culturing mesenchymal stem cells, wherein the medium comprises an imidazole dipeptide and is serum-free.

29. The medium according to claim 28, wherein the medium is insulin-free, IGF-free, or FGF-free.

30. The medium according to claim 28 or 29, wherein the medium is growth factor-free.

31. The medium according to any one of claims 28 to 30, wherein the medium is protein-free.

32. The medium according to any one of claims 28 to 31, comprising mesenchymal stem cells.

33. A method of inhibiting generation of osteoblasts, comprising the step of culturing mesenchymal stem cells in a medium containing an imidazole dipeptide.

34. A method of inhibiting generation of osteoblasts, comprising the step of bringing mesenchymal stem cells into contact with a culture supernatant obtained by culturing mesenchymal stem cells in a medium containing an imidazole dipeptide.

35. A composition for inhibiting generation of osteoblasts, comprising an imidazole dipeptide.

36. A composition for inhibiting generation of osteoblasts, comprising a culture supernatant obtained by culturing mesenchymal stem cells in a medium containing an imidazole dipeptide.

37. A method of promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker, the method comprising the step of culturing mesenchymal stem cells in a medium containing an imidazole dipeptide.

38. A composition for promoting expression of G-CSF, MCP-1, VEGF-C, TGF-β1, IL-6, IL-7, IL-8, M-CSF, Osteoprotegerin, Gremlin 1, SCF, FGF11, R-spondin 2, DAM8, IL-34, IGFBP2, DKK1, semaphorin 3B, or an exosome marker of mesenchymal stem cells, the composition comprising an imidazole dipeptide.

39. A method of promoting exosome secretion, comprising the step of culturing mesenchymal stem cells in a medium containing an imidazole dipeptide.

40. A composition for promoting secretion of exosomes from mesenchymal stem cells, comprising an imidazole dipeptide.

41. A culture supernatant of mesenchymal stem cells, comprising at least 500 pg/ml of G-CSF.

42. A culture supernatant of mesenchymal stem cells, comprising at least 2700 pg/ml of IL-6.

43. An umbilical cord-derived mesenchymal stem cell that expresses or secretes a high level of G-CSF or IL-6.

44. A composition comprising a cell population of the cells according to claim 43.

45. An IL-34-positive mesenchymal stem cell.

46. The mesenchymal stem cell according to claim 45, wherein the mesenchymal stem cell is purified.

47. An ADAM8-positive mesenchymal stem cell.

48. The mesenchymal stem cell according to claim 47, wherein the mesenchymal stem cell is purified.

49. The mesenchymal stem cell according to any one of claims 45 to 48, wherein the mesenchymal stem cell is positive for Osteoprotegerin, Gremlin 1, SCF, FGF11, R-spondin 2, ADAM8, IL-34, IGFBP2, DKK1, and semaphorin 3B.

50. The mesenchymal stem cell according to claim 49, wherein the mesenchymal stem cell is purified.

51. A pharmaceutical composition comprising the mesenchymal stem cells according to any one of claims 45 to 50.

52. The pharmaceutical composition according to claim 51, for inhibiting osteogenic differentiation.

53. A pharmaceutical composition for inhibiting osteogenic differentiation, comprising cells obtained by culturing mesenchymal stem cells in a medium containing an imidazole dipeptide.

54. The pharmaceutical composition according to claim 53, further comprising a pharmaceutically acceptable carrier.

55. The pharmaceutical composition according to claim 53 or 54, wherein the cells are positive for Osteoprotegerin, Gremlin 1, SCF, FGF11, R-spondin 2, ADAM8, IL-34, IGFBP2, DKK1, and semaphorin 3B.

56. The pharmaceutical composition according to any one of claims 53 to 55, wherein the cells are cells obtained by culturing in a medium containing an imidazole dipeptide and LIF.

57. The pharmaceutical composition according to any one of claims 53 to 56, wherein the culturing is adhesion culture.

58. A method for producing a pharmaceutical composition for inhibiting osteogenic differentiation, the method comprising the step of culturing mesenchymal stem cells in a medium containing an imidazole dipeptide to generate cultured cells.

59. The production method according to claim 58, comprising a step of recovering the cultured cells from the medium.

60. The production method according to claim 58 or 59, comprising a step of mixing the cultured cells with a pharmaceutically acceptable carrier.

61. A medium comprising an imidazole dipeptide and a leukemia inhibitory factor (LIF) component.

62. The medium according to claim 61, further comprising an adhesion factor.

63. The medium according to claim 61 or 62, for culturing mesenchymal stem cells.

64. The medium according to any one of claims 61 to 63, comprising mesenchymal stem cells.

65. A method for producing cells, comprising the step of culturing mesenchymal stem cells in the medium according to any one of claims 61 to 64 to generate cultured cells.

66. The production method according to claim 65, wherein the culturing is adhesion culture.

67. A cell obtained by the production method according to claim 65 or 66.

68. A composition comprising a cell population of the cells according to claim 67.

69. A pharmaceutical composition comprising cells obtained by the production method according to claim 65 or 66.

70. The pharmaceutical composition according to claim 69, for inhibiting osteogenic differentiation.

71. A kit comprising an imidazole dipeptide and a LIF component.

72. The kit according to claim 71, for use in preparation of a medium for culturing mesenchymal stem cells.
